# EUROPEAN PATENT SPECIFICATION

(11) **EP 2 118 128 B1**
(45) Date of publication and mention of the grant of the patent: **07.11.2012**
(21) Application number: 08727588.9
(22) Date of filing: 11.01.2008
(51) Int. Cl.: A61K 39/00, C07K 14/47

(54) **FUSION PROTEINS COMPRISING THE TUMOR REJECTION ANTIGENS NY-ESO-1 AND LAGE-1**
FUSIONSPROTEINE MIT DEN TUMOR-ANTIGENEN NY-ESO-1 UND LAGE-1
PROTÉINE HYBRIDE COMPRENANT LES ANTIGÈNES NY-ESO-1 ET LAGE-1

(30) Priority: 15.01.2007 GB 0700759; 30.04.2007 US 914848 P; 30.04.2007 US 914925 P; 21.05.2007 GB 0709707
(43) Date of publication of application: 18.11.2009
(73) Proprietor: GlaxoSmithKline Biologicals SA, 1330 Rixensart (BE)
(72) Inventor: BLAIS, Normand, Laval, Québec H7V 3S8 (CA); BOYER, Martine, Laval, Quebec H7V 3S8 (CA); BRICHARD, Vincent, B-1330 Rixensart (BE); LOUAHED, Jamila, B-1330 Rixensart (BE); MARTIN, Denis, Laval, Quebec H7V 3S8 (CA); PALMANTIER, Remi M., Laval, Quebec H7 V 3S8 (CA); RIOUX, Clement, Laval, Quebec H7V 3S8 (CA)
(74) Representative: Lovatt, Victoria Jayne
(86) International application number: PCT/US2008/050879
(87) International publication number: WO 2008/089074

(56) References cited:
- WO-A-01/74855
- WO-A-2005/071088
- RIMOLDI D ET AL: "Efficient simultaneous presentation of NY-ESO-1/LAGE-1 primary and nonprimary open reading frame-derived CTL epitopes in melanoma." JOURNAL OF IMMUNOLOGY (BALTIMORE, MD. : 1950) 15 DEC 2000, vol. 165, no. 12, 15 December 2000 (2000-12-15), pages 7253-7261, XP002500509 ISSN: 0022-1767
- ODUNSI KUNLE ET AL: "NY-ESO-1 and LAGE-1 cancer-testis antigens are potential targets for immunotherapy in epithelial ovarian cancer." CANCER RESEARCH, vol. 63, no. 18, 15 September 2003 (2003-09-15), pages 6076-6083, XP002500508 ISSN: 0008-5472

## Description

### FIELD OF THE INVENTION

The present invention relates generally to polypeptides and constructs comprising an antigen derived from one or both of the tumor rejection antigens NY-ESO-1 and LAGE-1.

### BACKGROUND TO THE INVENTION

Cancer testis (CT) antigens are a class of tumour-associated antigens with expression normally restricted to germ cells in the testis, ovaries or trophoblast cells. These antigens are not usually expressed in adult somatic tissues. See, Simpson, et al., Nat. Rev. Cancer, 5(8):615-625 (2005); Scanlan, et al., Immunol. Reviews, 188:22-32 (2002); Scanlan, et al., Canc. Immun., 4:1-15 (2004).

The gene regulation of CT antigens is disrupted in cancer patients, leading to the aberrant expression of these antigens in a wide variety of tumours. The first CT antigen to be identified, MAGE-1, was identified in the early 1990s by T-cell epitope cloning (van der Bruggen et al, 1991 Science 13;254(5038):1643-7; van der Bruggen et al, 1999 Science 254:1643-1647; Traversari, et al, 1992 Immunogenetics, 35(3):145-152; and U.S. Patent No. 5,342,774. Since then, serological expression cloning technique (SEREX) (Sahin, et al., Proc. Natl. Acad. Sci. USA, 92(25):11810-11813 (1995) and U.S. Patent No. 5,698,396), recombinant antigen expression on yeast surface (RAYS) (Mischo, et al., Canc. Immun., 3:5-16 (2003)) and differential mRNA expression analysis (Gure, et al., Int. J. Canc., 85(5):726-732 (2000)) have led to the identification of approximately 90 CT antigens, and their number is expected to grow in the coming years. The immunogenicity of some CT antigens in cancer patients makes them an ideal target for the development of tumour vaccines.

*NY-ESO-1.* A cancer testis antigen currently of interest for use in cancer immunotherapy is NY-ESO-1. This antigen was first identified by SEREX in an oesophageal squamous cell carcinoma in the late 90's at the New York Branch of the Ludwig Institute for Cancer Research (Chen, et al., PNAS USA, 94(5):1914-1918 (1997); and U.S. Patent No. 5,804,381.

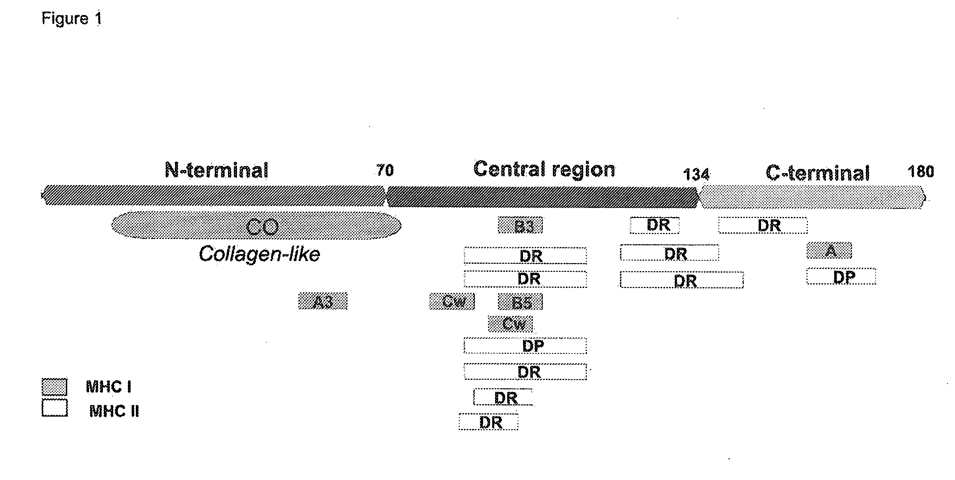

The protein NY-ESO-1 is 180 amino acids in length and can be described as being composed of three regions:
■ An N-terminal region about or approximately amino acids 1 to 70,
■ A central region about or approximately amino acids 71 to 134, and
■ A C-terminal region about or approximately amino acids 135 to 180.
A collagen-like region comprises about or approximately or about amino acids 15 to 73 of the N-terminal region (see Figure 1).

The protein NY-ESO-1 has been found in a wide variety of tumours, including but not limited to ovarian cancer, lung cancer, breast cancer, prostrate, oesophageal cancer, bladder cancer and in melanomas. (Nicholaou T, et al, Immunol Cell Biol. 2006 Jun;84(3):303-17 and Jungbluth, et al. 2001, Int. J. Canc., 92(6):856-860). Spontaneous humoral and cellular immune responses against this antigen have been described in patients with NY-ESO-1-positive tumours, and a number of HLA (Human Leukocyte Antigen) class I- and II-restricted peptides have been identified (Jager, et al., 1998 J. Exp. Med., 187(2):265-270; Yamaguchi, et al., 2004 Clin. Canc. Res., 10(3):890-961; and Davis, et al., 2004 Proc. Natl. Acad. Sci. USA, 101(29):10697-10702). Exemplary of the patent literature are U.S. Patent Nos. 6,140,050; 6,251,603; 6,242,052; 6,274,145; 6,338,947; 6,417,165; 6,525,177; 6,605,711; 6,689,742; 6,723,832; 6,756,044; and 6,800,730.

In a clinical trial, three partially overlapping NY-ESO-1-derived peptides with binding motifs to HLA-A2 (157-167, 157-165 and 155-163) have been used in a vaccine to treat twelve patients with metastatic NY-ESO-1 expressing tumours. This study demonstrated that synthetic NY-ESO-1 peptides can be administered safely and are capable of generating potentially beneficial T cell responses (Jager, et al., 2000 PNAS USA, 97(22):12198-12203).

A number of MHC (major histocompatibility complex) class I and II epitopes in the protein have been identified by different groups see, for example, Figure 1. These epitopes are merely representative of epitopes reported for the protein and the list in Figure 1 is not exhaustive. Furthermore, at least one or more of the epitopes reported and/or listed in Figure 1 have not been confirmed by experimentation. The collagen-like region in the N-terminal contains at least one MHC class I epitope referred to herein as A31. The central region comprises several MHC class 2 epitopes referred to herein as DR1, DR2, DR4, DR7 and DP4. This region also contains several MI-IC class I epitopes referred to herein as B35, B51, Cw3 and Cw6. The C-terminal is believed to contain at least two class II epitopes (DR4 and DP4) and one class I epitope (A2).

*LAGE-1.* A further cancer testis antigen, LAGE-1, has also been identified. Two LAGE-1 transcripts have been described, LAGE-Ia and LAGE1b. LAGE-1b is incompletely spliced and codes for a putative protein of approximately 210 amino acids residues, while the LAGE1a gene product contains 180 amino acid residues (Sun et al. Cancer Immunol Immunother 2006: 55: 644-652).

The N-terminal regions of the LAGE-1 and NY-ESO-1 proteins are highly conserved and are thought to have more than 97% identity. However, LAGE-1 differs from NY-ESO-1 in the central regions which are only 62% identical. The C-terminals of NY-ESO-1 and LAGE-1a are highly conserved (more than 97% identity). However, the C-terminal of LACE-1 b is longer and not conserved and is thought to have less than 50% identity with the same region in LAGE-1 a/NY-ESO-1.

General information relating to these proteins is available from the LICR web site (see www.cancerimmunity.org/Cldatabase).

### SUMMARY OF THE INVENTION

The present invention provides an immunogenic fusion protein comprising;
(i) NY-EWA or a fragment thereof, linked to
(II) LAGE-1 or a fragment thereof,
in which at least one of NY-ESO-1 and/or LAGE-1 is truncated or partially truncated, or is a fragment including one or more epitopes of NY-ESO-1 or LAGE-1. wherein the fusion protein comprises an amino acid sequence selected from the group consisting of SEQ ID NO:73, SEQ ID NO:75, SEQ ID NO:79, SEQ ID NO:81, SEQ ID NO:83, SEQ ID NO:85, SEQ ID NO:89, SEQ ID NO:93, and SEQ ID NO:97.

Compositions involving fusion proteins and polypeptides are also provided.

### BRIEF DESCRIPTION OF THE FIGURES

Figure 1 shows a number MHC (major histocompatibility complex) class I and II epitopes on the NY-ESO-1 protein that have been identified by different groups. These epitopes are merely representative of epitopes reported for the protein thus the list in Figure 1 is not exhaustive. Furthermore, at least one or more of the epitopes reported and/or listed in Figure 1 have not been confirmed by experimentation. The reported amino acid sequence for NY-ESO-1 is found herein in SEQ ID NO:49.

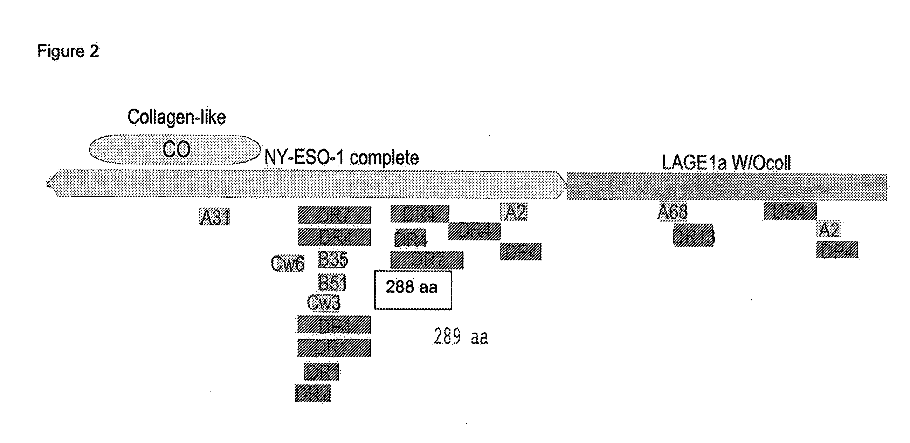

Figure 2 shows construct A, a fusion protein comprising full length NY-ESO-1 and truncated Large-1, such as LAGE-1a. In this embodiment, the C-terminus of the NY-ESO-1 is fused to the N-terminus of truncated Large-1, together with a Histidine affinity tag to provide a fusion protein of 288 amino acids in length. Further details of construct A are given in Table 1 (SEQ ID NO:1; SEQ ID NO:3).

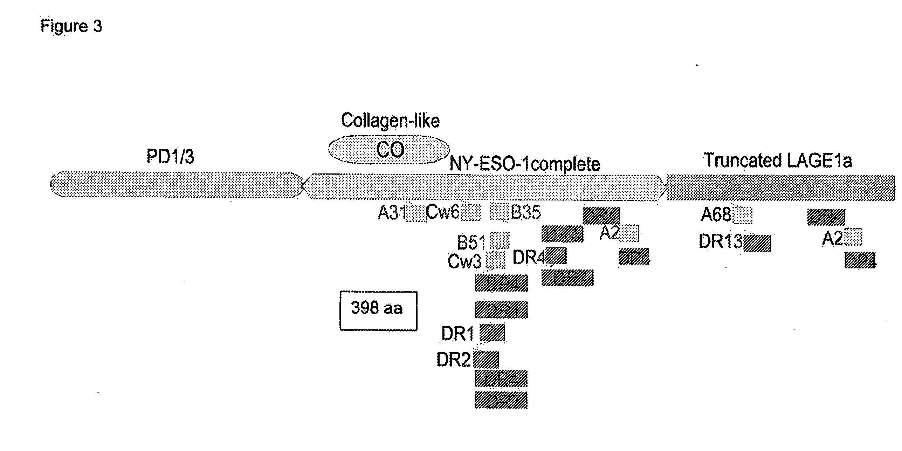

Figure 3 shows construct B, a fusion protein comprising the first third of protein D without its secretion signal (for example amino acids 20 to 127), full length NY-ESO-1 and truncated LAGE-1, such as LAGE-1a. In this embodiment, amino acid 127 of protein D is fused to the N-terminus of NY-ESO-1, the C-terminus thereof being fused to the N-terminus of truncated LAGE-1, to provide a fusion protein of 398 amino acids in length. Further details of construct B are given in Table 1 in section 1.6 (SEQ ID NO:2; SEQ ID NO:4).

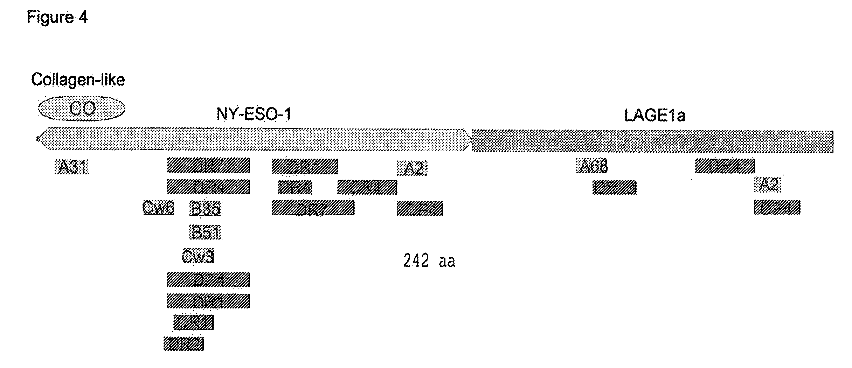

Figure 4 shows construct C, a fusion protein comprising partially truncated NY-ESO-1 and truncated LAGE-1, such as LAGE-1a. In this embodiment, the C-terminus of partially truncated NY-ESO-1 is fused to the N-terminus of truncated Large-1, to provide a fusion protein of 242 amino acids in length. Further details of construct C are given in Table 1 (SEQ ID NO:5; SEQ ID NO:7).

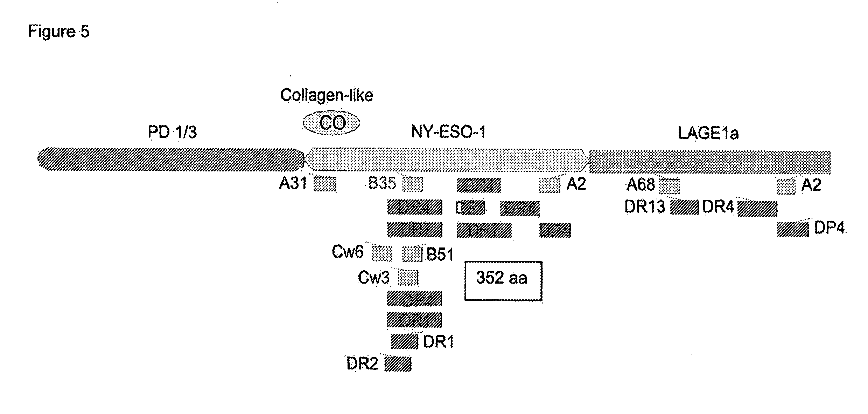

Figure 5 shows construct D, a fusion protein comprising the first third of protein D without its secretion signal (for example amino acids 20 to about or approximately 127), partially truncated NY-ESO-1 and truncated LAGE-1, such as LAGE-1a. In this embodiment, amino acid 127 of protein D is fused to the N-terminus of partially truncated NY-ESO-1, the C-terminus thereof being fused to the N-terminus of the truncated LAGE-1, to provide a fusion protein of 352 amino acids in length. Further details of this embodiment are given in Table 1 (SEQ ID NO:6; SEQ ID NO:8).

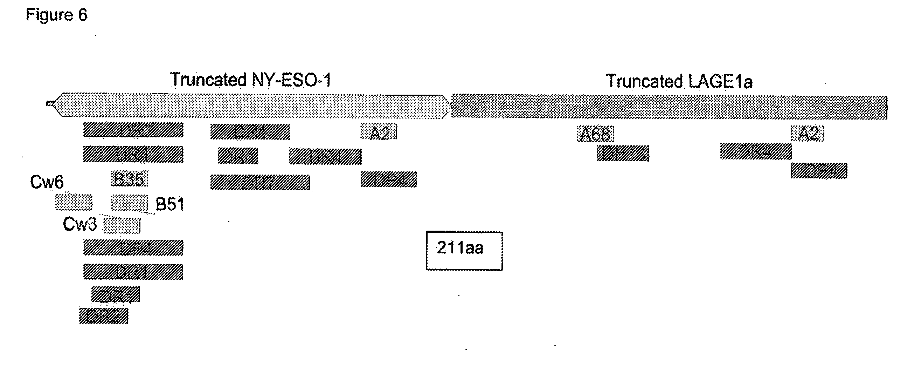

Figure 6 shows construct E, a fusion protein comprising truncated NY-ESO-1 and truncated LAGE-1, such as LAGE-1a. In this embodiment, the C-terminus of truncated NY-ESO-1 is fused to the N-terminus of truncated LAGE-1, to provide a fusion protein of 211 amino acids in length. Further details of construct E are given in Table 1 (SEQ ID NO:9; SEQ ID NO:11).

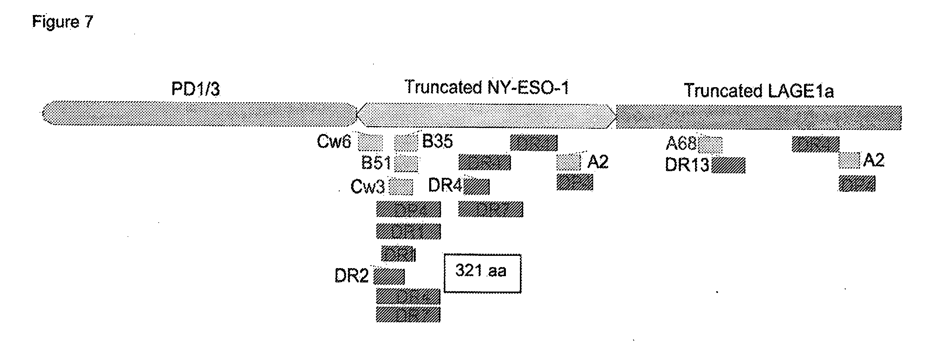

Figure 7 shows construct F, a fusion protein comprising the first third of protein D without its secretion signal (for example amino acids 20 to about or approximately 127), truncated NY-ESO-1 and truncated LAGE-1, such as LAGE-1a. In this embodiment, amino acid 127 of protein D is fused to the N-terminus of truncated NY-ESO-1, the C-terminus thereof being fused to the N-terminus of truncated LAGE-1, to provide a fusion protein of 321 amino acids in length. Further details of construct F are given in Table 1 (SEQ ID NO:10; SEQ ID NO:12).

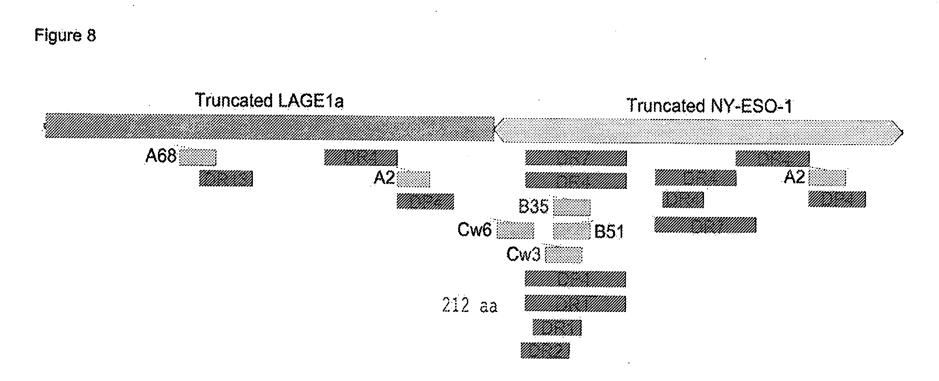

Figure 8 shows an alternative embodiment of construct E, namely E', in which the C-terminus of truncated LAGE-1 is fused to the N-terminus of truncated NY-ESO-1, to provide a fusion protein of 212 amino acids in length. Further details of this embodiment, construct E', are given in Table 1 (SEQ ID NO:21; SEQ ID NO:23).

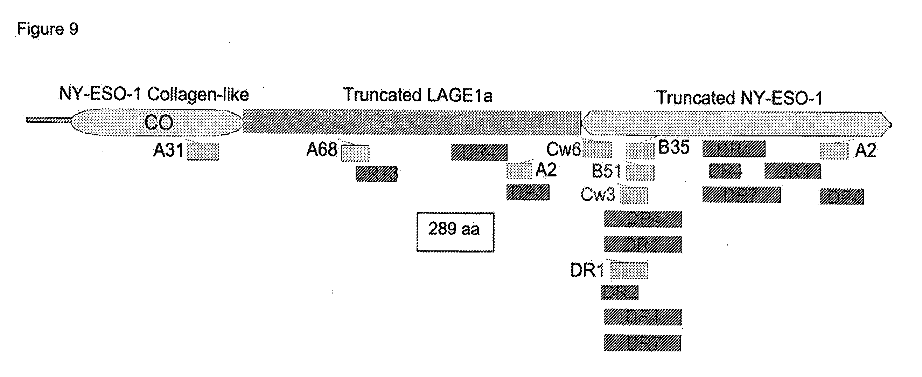

Figure 9 shows construct G, a fusion protein comprising truncated NY-ESO-1, truncated LAGE-1, such as LAGE-1a and the collagen-like region, such as the collagen region from NY-ESO-1. In this embodiment, the C-terminus of the collagen-like region is, for example, fused to the N-terminus of truncated LAGE-1. In turn the C-terminus of the truncated LAGE-1 is fused to the N-terminus of truncated NY-ESO-1, to provide a fusion protein of 289 amino acids in length. Further details of construct G are given in Table 1 (SEQ ID NO:13; SEQ ID NO:15).

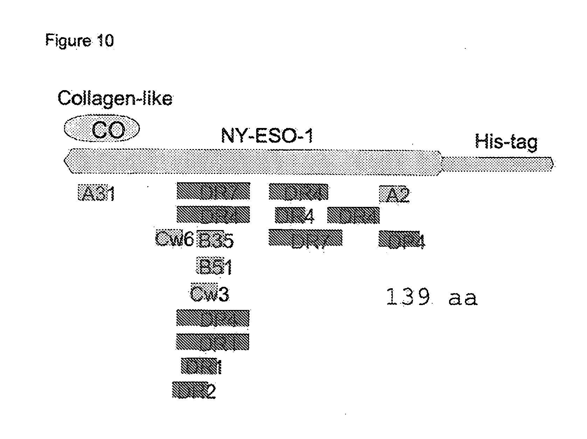

Figure 10 shows a schematic of an exemplary recombinant polypeptide comprising NY-ESO-1 with a partially truncated collagen like domain. The epitopes shown in Figures 10-13 are merely representative of epitopes reported for the protein and have not been confirmed by experimentation.

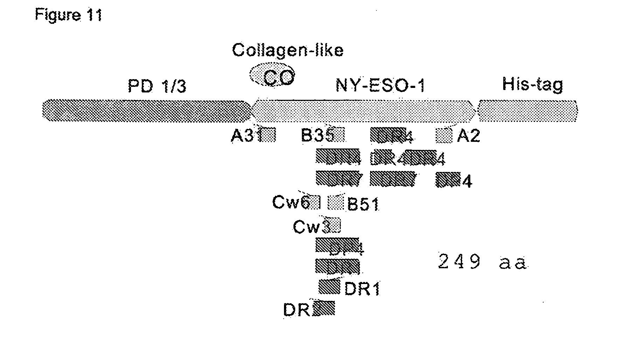

Figure 11 shows a schematic of an exemplary fusion protein comprising the first third of protein D without its secretion signal (for example amino acids 20 to about or approximately 127) and NY-ESO-1 with a partially truncated collagen-like domain.

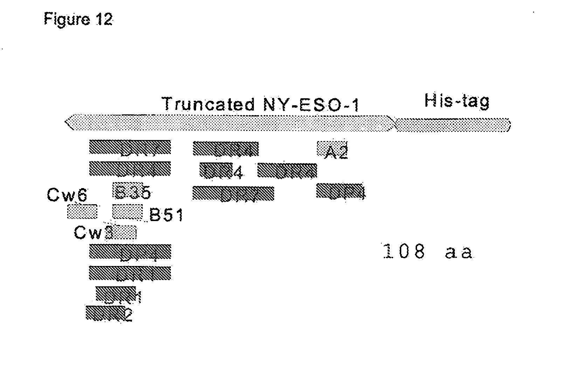

Figure 12 shows a schematic of an exemplary recombinant polypeptide comprising NY-ESO-1 with a partially truncated collagen-like domain.

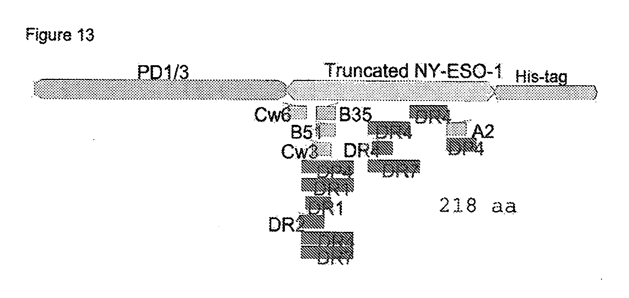

Figure 13 shows a schematic of an exemplary fusion protein comprising the first third of protein D without its secretion signal (for example amino acids 20 to about or approximately 127) and NY-ESO-1 with a truncated collagen-like domain.

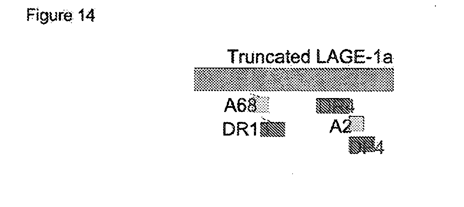

Figure 14 is a schematic that shows a number of epitopes identified within the truncated LAGE-1a protein. These epitopes are merely representative of epitopes reported for the protein thus the list is not exhaustive. For the avoidance of doubt, the epitopes reported and/or listed in the figures may or may not have not been confirmed by experimentation (i.e., they may have been predicted, etc.), unless otherwise stated herein. The complete LAGE-1a amino acid sequence is set forth in the sequence listing as SEQ ID NO:58. The complete LAGE-1b amino acid sequence (LAGE-1b not depicted in this Figure) is set forth in the sequence listing as SEQ ID NO:71.

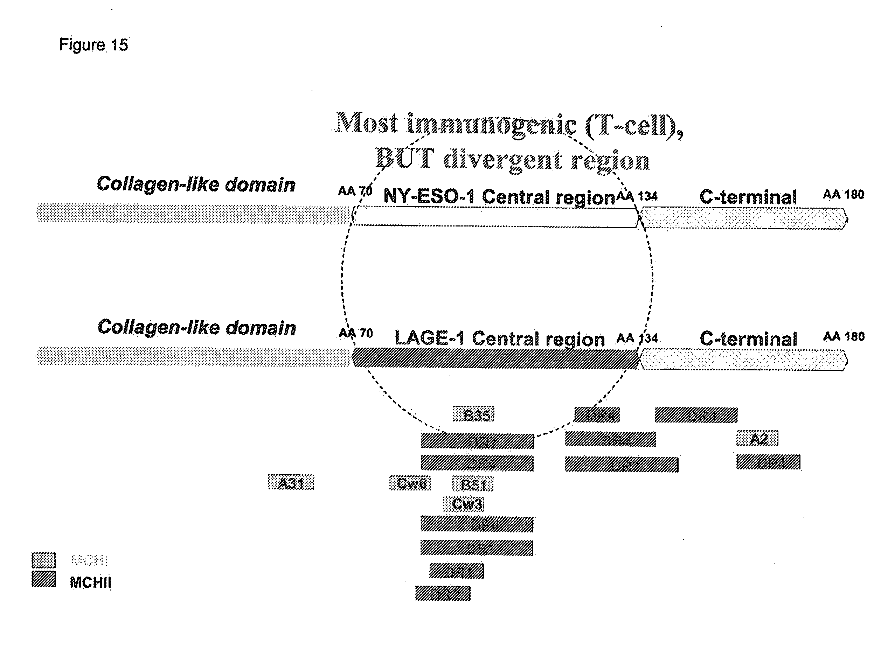

Figure 15 shows a schematic of both NY-ESO-1 and LAGE-1, as well as a number of MHC (major histocompatibility complex) class I and II epitopes. These epitopes are merely representative of epitopes reported for the protein thus the list is not exhaustive; one or more of the epitopes reported and/or listed have not been confirmed by experimentation.

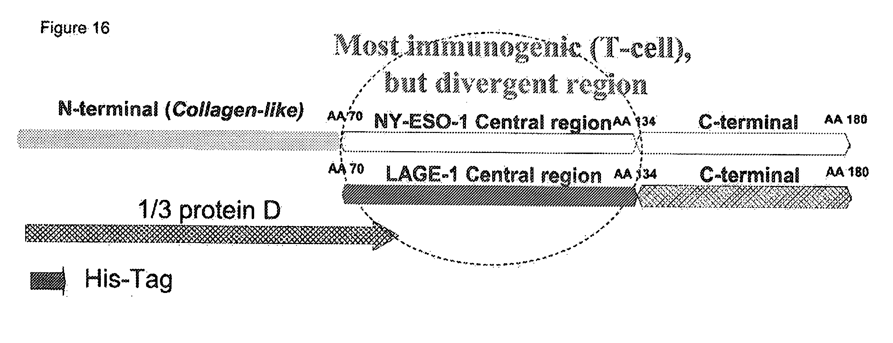

Figure 16 shows a schematic of the NY-ESO-1/LAGE-1 fusion design.

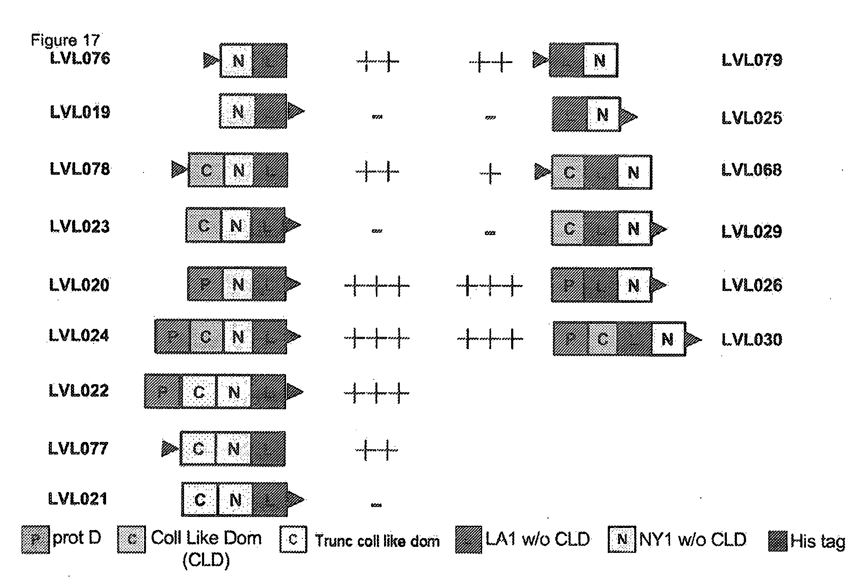

Figure 17 summarizes in schematic fashion fifteen constructs and their production levels. P = protein D; C (grey box) = NY-ESO-1 collagen-like domain; C (white box) = truncated collagen like domain; L = Lage 1 without the collagen like domain; N = NY-ESO-1 without the collagen like domain; black arrow = poly histidine tag; (-) = low production; (+) = some production; (++) = high production; (+++) = best production. The amino acid sequences for eight of the constructs and the nucleotide sequences encoding them are summarized in Table 4 and the sequence listing.

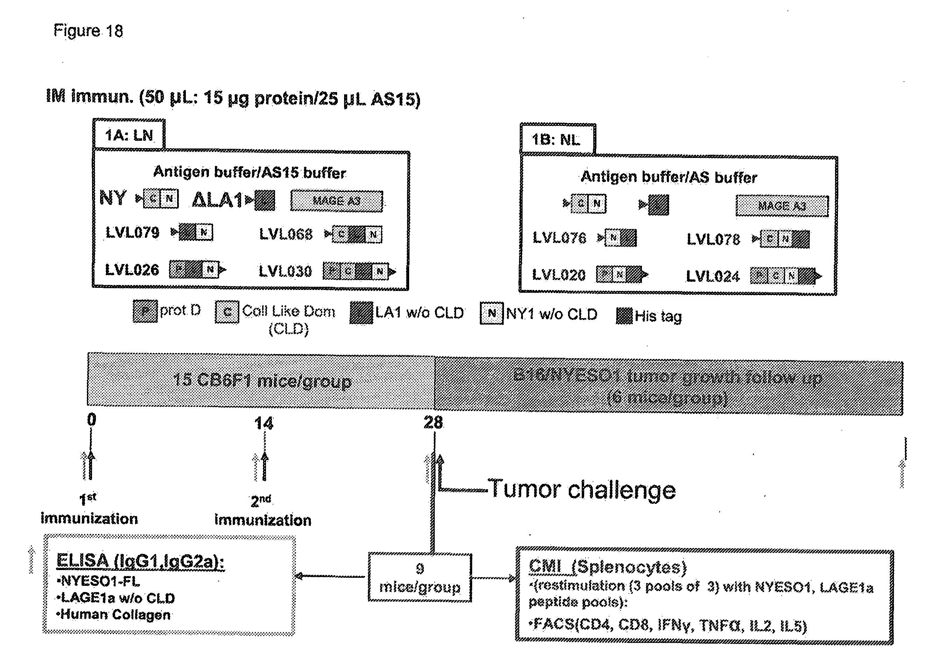

Figure 18 summarizes screening #1, a 76-day trial using CB6F1 mice to assess each of LVL076, LVL079, LVL78, LVL68, LVL020, LVL26, LVL024, LVL30 to determine whether intramuscular immunization with the fusion protein plus adjuvant conferred protection against subcutaneous challenge with transplanted tumors (B16/NYES01).

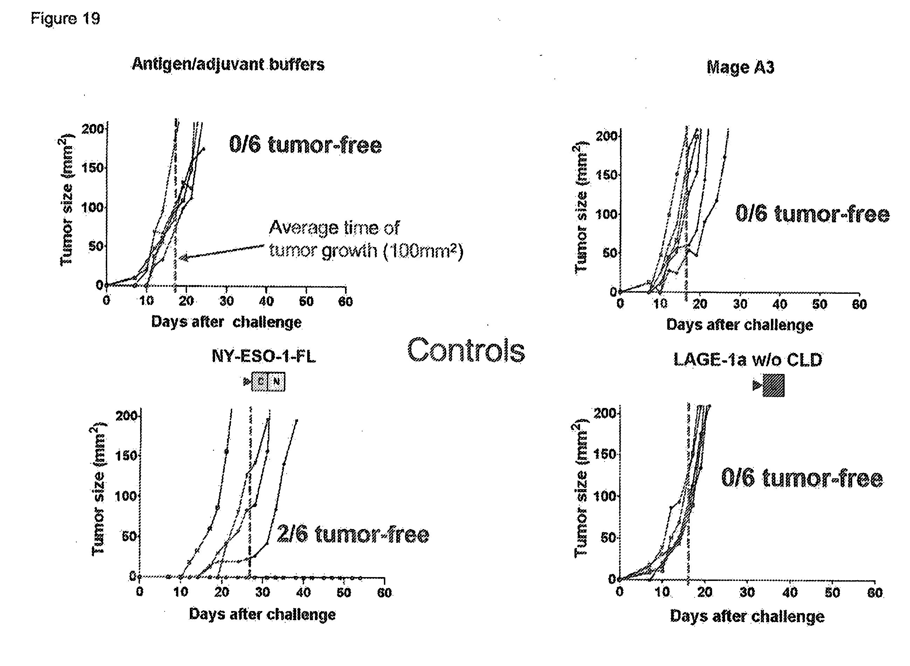

Figure 19 summarizes B-16-NY-ESO-1 tumor growth in the control mice used in the 76-day trial.

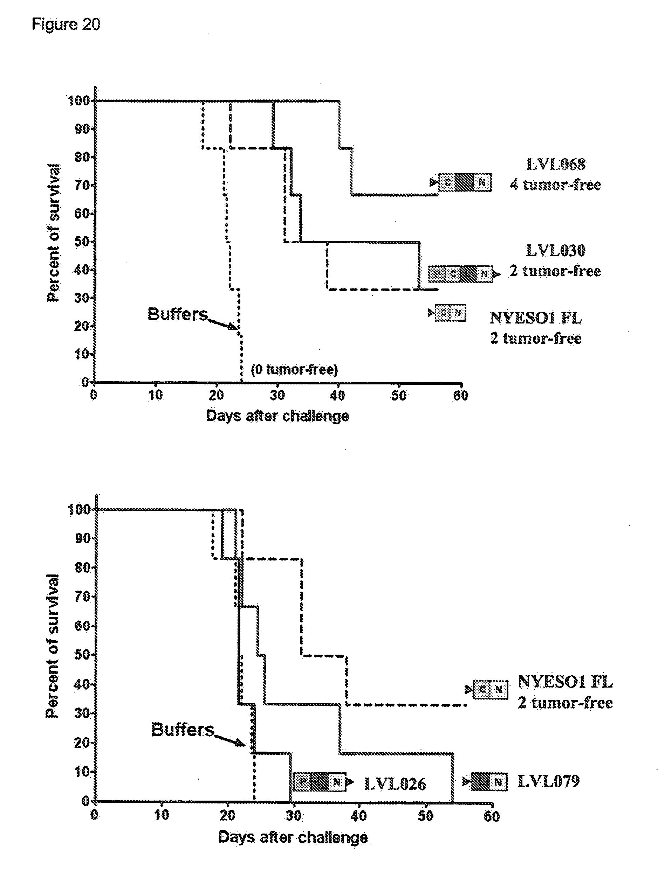

Figure 20 shows survival of mice immunized with full-length NY-ESO-1, LVL030, LVL068, LVL079, or LVL026.

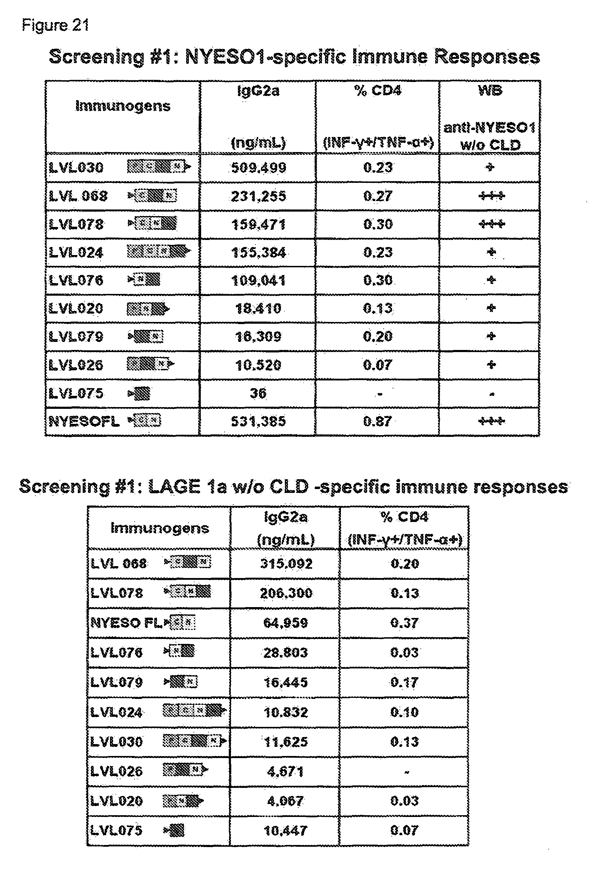

Figure 21 summarizes the NY-ESO-1-specific immune responses as assessed by ELISA, FACS, and Western Blot and LAGE-1 a(without the collagen like domain)-specific immune responses as assessed by ELISA and FACS.

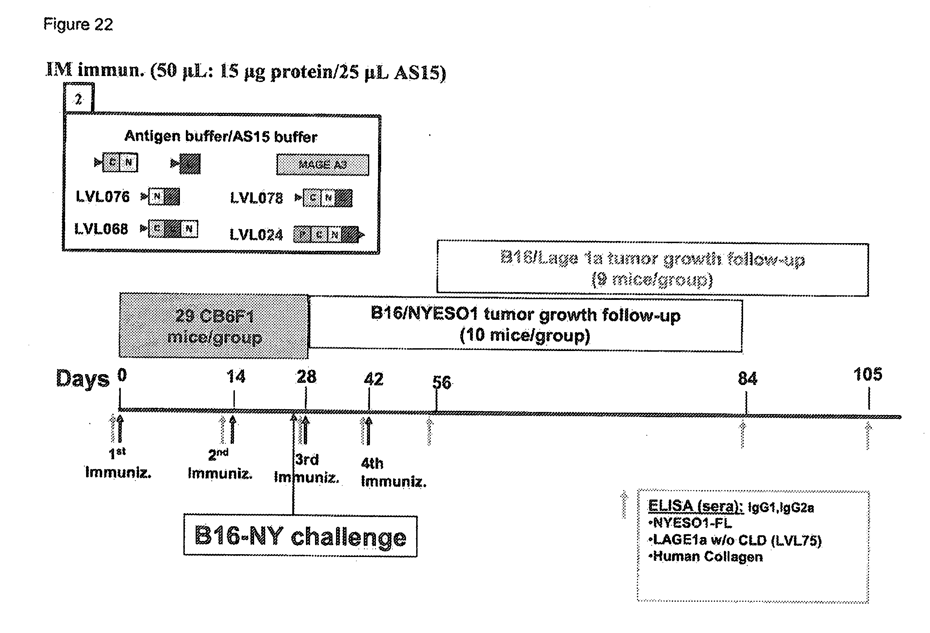

Figure 22 summarizes the experimental design of screening #2, a 105 day trial to determine whether intramuscular immunization with selected fusion proteins plus adjuvant confers protection against B16/NY-ESO-1 challenge and B16/LAGE-1a challenge. B16/NY-ESO-1 challenge is shown.

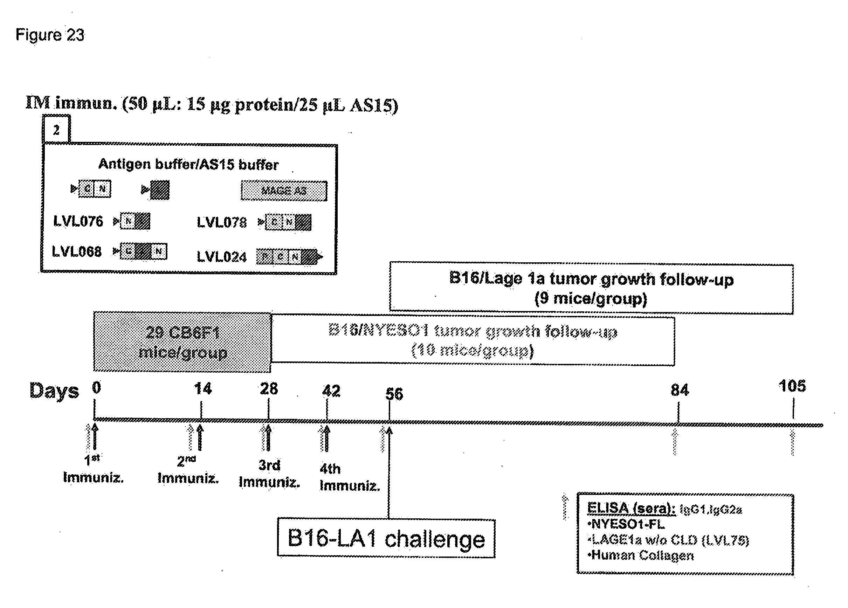

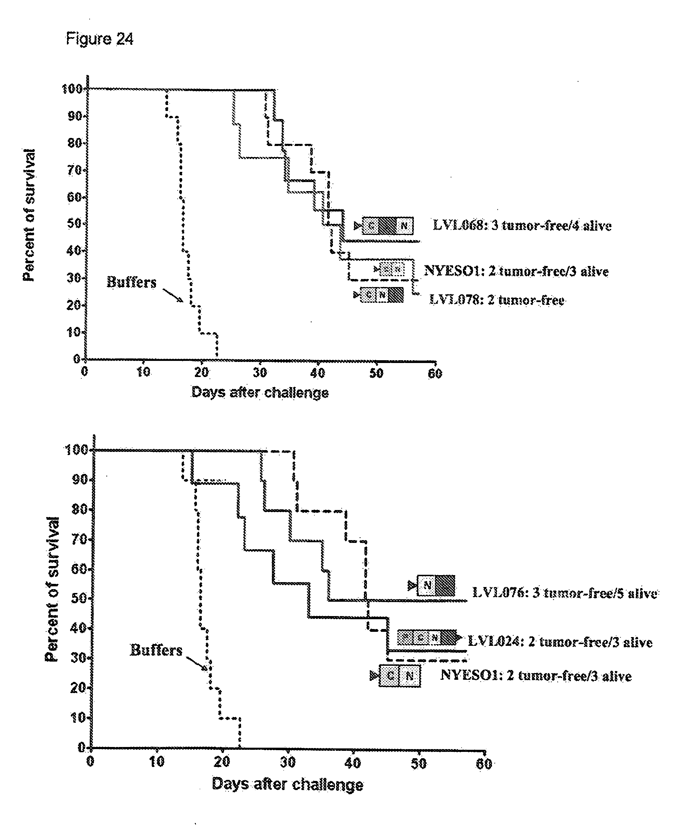

Figure 23 summarizes screening #2 and shows the B16/LAGE-1a challenge. Figure 24 shows survival of mice immunized with LVL078, LVL068, full-length NY-ESO-1, LVL024, and LVL076 post-B16/NY-ESO-1 challenge. See Figure 24.

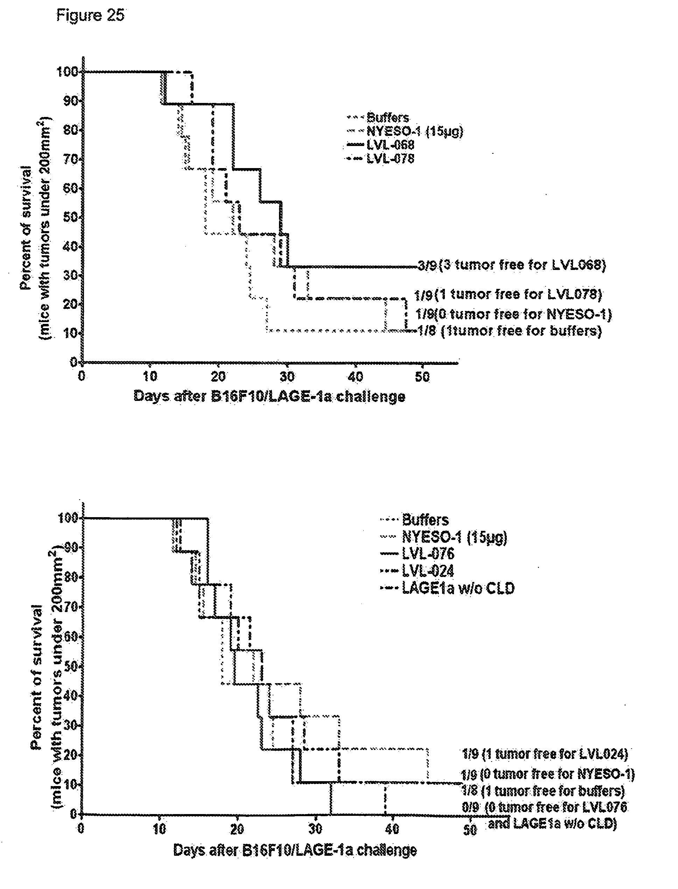

Figure 25 shows survival of mice immunized with LVL076, LAGE-1a without the collagen like region, LVL024, full-length NY-ESO-1, LVL078, or LVL068 post-B16/LAGE-1a challenge.

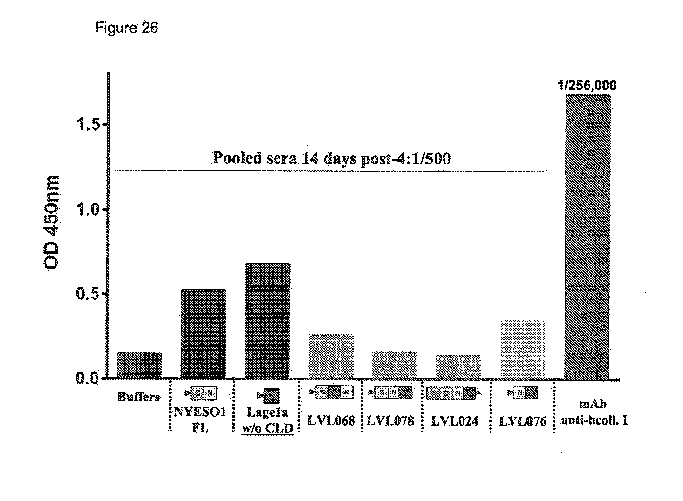

Figure 26. Columns 1-8, from left to right, show the results of ELISAs carried out to detect possible human collagen-specific immune responses in mice immunized with one of the following: (1) Buffers (control); (2) full-length NY-ESO-1; (3) LAGE-1a without the collagen like domain; (4) LVL068; (5) LVL078; (6) LVL024; (7) LVL076. Positive control (column 8) contains an anti-human collagen 1 monoclonal antibody (mAb anti-human collagen I).

### DETAILED DESCRIPTION OF THE INVENTION

*Fusion Proteins.* The fusion proteins of the invention are useful for the treatment of cancers, and more specifically for the treatment of: melanoma; breast cancer; prostate cancer; bladder cancer including transitional cell carcinoma; lung cancer including non-small cell lung carcinoma (NSCLC); head and neck cancer including oesophageal carcinoma; squamous cell carcinoma; carcinoma of the gastrointestinal tract; liver cancer; brain tumours; leukemia; and various sarcomas.

Based on the expression profiles of LAGE-1 and NY-ESO-1 the fusion protein according to the invention has the potential to be effective in an estimated 37% of breast cancers. The treatment according to the present invention may also be particularly suitable for the treatment of patients not eligible for Her2/neu targeted therapy. The fusion protein of the invention is also predicted to be effective in approximately 35% of prostate cancer patients, 35% of bladder cancer patients, 40% of melanoma patients and 35% of patients with NSCLC (non-small cell tung carcinoma). In one embodiment, the fusion protein of the invention may enable a broader population of patients to be treated because patients having tumours that express both NY-ESO-1. and/or LAGE-1 (including LAGE-1a and LAGE-1b) may be given a fusion protein of the present invention.

The fusion protein according to the invention may also be more immunogenic than its individual component proteins, for the following reasons:
■ removal of one or more of the collagen-like domains may reduce potential immunotolerance of the compound caused by its homology with natural endogenous collagen structure, or
■ the optional addition of a heterologous fusion partner may further stimulate CD4 T-cell responses. Thus, the fusion proteins are useful for inducing an immunogenic response to a cancer antigen such as NY-ESO-1 or LAGE-1, or both.

The NY-ESO-1 described herein may be full length, partially truncated or truncated NY-ESO-1 or any fragment thereof that includes one or more epitopes capable of raising an immune response to NY-ESO-1. Full length NY-ESO-1 protein in the context of this specification is intended to mean a protein of about or approximately 1 to 180 amino acids and at least 95, 96, 97, 98, 99% or 100% identical, to the naturally occurring protein (SEQ ID NO:49). As used herein, the term "LAGE-1" refers to one or more LAGE-1 family members such as LAGE-1 a and LAGE 1b, as described in the lines below. "Full length LAGE-1a" protein is intended to mean a protein 95, 96, 97, 98, 99% or 100% identical to SEQ ID NO:58. Similarly, "full length LAGE-1b" protein is intended to mean a protein 95, 96, 97, 98, 99% or 100% identical to the naturally occurring protein (SEQ ID NO:71).

In one example, the identity is over the full-length of the sequence. Thus, fusion proteins with conservative substitutions are also described. Conservative substitutions are well known and are generally set up as the default scoring matrices in sequence alignment computer programs. These programs include PAM250 (Dayhoft M.O. et al., (1978), "A model of evolutionary changes in proteins", "Atlas of Protein sequence and structure' 5(3) M.O. Dayhoft (ed.), 345-352), National Biomedical Research Foundation, Washington, and Blosum 62 (Steven Henikoft and Jorja G. Henikoft (1992), and "Amino acid substitution matricies from protein blocks"), Proc, Natl. Acad. Sci. USA 89 (Biochemistry): 10915-10919.

In general terms, substitution within the following groups are conservative substitutions, but substitutions between groups are considered non-conserved. The groups are:
i) Aspartate/asparagine/glutamate/glutamine,
ii) Serine/threonine,
iii) Lysine/arginine,
iv) Phenylalanine/tyrosine/tryptophane,
v) Leucine/isoleucine/valine/methionine,
vi) Glycine/alanine.

"Partially truncated" in the context of this specification is intended to mean NY ESO-1 or LAGE-1 protein (as appropriate) in which the majority of the collagen-like has been region removed but still comprising or consisting of the epitope A31 found in this region.

In one example, partially truncated NY-ESO-1 and/or LAGE-1 comprises or consists of a range of amino acids from amino acid 44, 45, 46, 47, 48, 49, 50, 51 or 52 to amino acid 175, 176, 177, 178, 179 or 180 or any combination of these amino acids, for example from amino acid 48 to amino acid 180 or from amino acid 46 to 178. In one embodiment partially truncated NY-ESO-1 or LAGE-1 comprises or consists of about or exactly amino acids 48 to 180 (or about or exactly amino acids 48-210 in the case of LAGE-1b). In one embodiment, the term "about" in this context may be taken to mean amino acids up to +/- 10% of the total number of amino acids of the sequence are optionally added or deleted from the sequence. In one embodiment, partially truncated NY-ESO-1 comprises or consists of amino acids 48 to 180 of NY-ESO-1.

In one example, partially truncated LAGE-1b comprises or consists of a range of amino acids from amino acid 44, 45, 46, 47, 48, 49, 50, 51 or 52 to amino acid 205, 206, 207, 208, 209 or 210 or any combination of these amino acids, for example from amino acid 48 to amino acid 210 or from amino acid 46 to 208. In one example partially truncated LAGE-1 b comprises or consists of about or exactly amino acids 48 to 210. The term "about in this context may be taken to mean amino acids up to +/- 10% of the total number of amino acids of the sequence are optionally added or deleted from the sequence. In one example, partially truncated LAGE-1b comprises or consists of amino acids 48 to 210 of LAGE-1b.

"Truncated" in the context of this specification is intended to mean NY-ESO-1 or LAGE-1 protein (as appropriate) in which the collagen-like region has been removed (including the removal of the A31 epitope). Truncated NY-ESO-1 and/or LAGE 1 comprises or consists of about or exactly amino acids 71-180 (or about or exactly amino aci 71-210 in the case of LAGE-1b).

In one example, truncated NY-ESO-1 or LAGE-1 comprises or consists of a range of amino acids from amino acid 67, 68, 69, 70, 71, 72, 73, 74 or 75 to amino acid 175, 176, 177, 178, 179 or 180 or any combination of these amino acids, for example from amino acid 71 to amino acid 180 or from amino acid 69 to 178. In one example truncated NY-ESO-1 or LAGE-1 comprises or consists of about or exactly amino acids 71 to 180 (or about or exactly amino acids 71-210 in the case of LAGE-1b).,

The term "about" in this context may be taken to mean amino 10 acids up to +/-10% of the total number of amino acids of the sequence are optionally added or deleted from the sequence. In one example, truncated NY-ESO-1 or LAGE-1 comprises or consists of amino acids 71 to 180 of NY-ESO-1 or LAGE-1.

In one example, truncated LAGE-1b comprises or consists of a range of amino acids from amino acid 67, 68, 69, 70, 71, 72, 73, 74 or 75 to amino acid 205, 206, 207, 208, 209 or 210 or any combination of these amino acids, for example from amino acid 71 to amino acid 210 or from amino acid 69 to 208. In one example truncated LAGE-1b comprises or consists of about or exactly amino acids 71 to 210. The term "about" in this context may be taken to mean amino acids up to +/-10% of the total number of amino acids the sequence are optionally added or deleted from the sequence. In one example, truncated LAGE-1b comprises or consists of amino acids 71 to 210 of LAGE-1 b.

By "other fragments" is intended those which, when incorporated into the fusion protein of the invention, result in a final protein with the desired properties and advantages of the fusion proteins of the invention.

*NY-ESO-1.* In accordance with the foregoing are provided modified antigens comprising an antigen derived from the tumor rejection antigen NY-ESO-1 wherein the collagen region is partially truncated or completely truncated. In some examples, more than the collagen region is removed. In some examples, the modified antigen is genetically modified. In some examples the modified antigen is recombinant. In some examples are provided polypeptides comprising an antigen as described in the preceding sentences. In some examples, exemplary polypeptides comprise a heterologous protein, such as protein D from Haemophilus influenzae type B or a fragment thereof. In some examples are provided constructs comprising a nucleotide sequence encoding the aforementioned polypeptides.

The proteins for inclusion in a fusion partner of the present invention may be chemically conjugated, or may be expressed as recombinant fusion proteins. In one embodiment, the fusion protein is expressed as a recombinant fusion protein.

The further heterologous fusion partner may assist in providing T helper epitopes (immunological fusion partner), or may assist in expressing the protein at higher yields (expression enhancer). In one embodiment, the further heterologous fusion partner may be both an immunological fusion partner and an expression enhancing partner.

In one embodiment, the protein D or derivative thereof comprises about or exactly the first 1/3 of the protein, for example about or exactly amino acids 1 to 109 of protein D. In this embodiment, amino acids 2-Lys and/or 3-Thr of the native protein D sequence may be substituted with the amino acids 2-Asp and/or 3-Pro. In a further embodiment, the protein D or derivative thereof comprises or consists of about or exactly amino acids 20 to 127 of protein D. In one embodiment, the protein D for use in the present invention does not include the secretion sequence of the protein. Generally, in fusion proteins of the present invention, the protein D derivative is not lipidated.

In one embodiment, the protein D further comprises the amino acids Met, Asp and Pro, for example fused to the N-terminus of the protein D fragment (ie the construct may comprise or consist of "MDP - 20-127 protein D"). It is thought these three additional amino acids may aid the stability of the protein and/or increase the level of the protein expression thereof.

In one aspect the invention provides a fusion protein in which the N-terminal fragment (i.e the first third) of protein D (as described above) is fused to the N-terminus of a fusion protein of the invention or an immunogenic fragment thereof. More specifically, a fusion of protein D and the N-terminus of the fusion protein of the invention may be effected such that the latter replaces the C-terminal-fragment of protein D that has been excised. Thus the N-terminus of protein D becomes the N-terminus of the fusion protein.

Other heterologous fusion partners or fragments thereof may be included in the fusion protein of the invention, instead of or in addition to protein D, for example:
■ the non-structural protein from influenzae virus, NS1 (haemagglutinin). Typically, the N-terminal 81 amino acids may be used, although different fragments may be used provided they include T-helper epitopes;
■ LytA derived from Streptococcus pneumoniae, which synthesize an N-acetyl-L-alanine amidase LytA coded by the LytA gene (Gene, 43 (1986) page 265-272) such as the repeat portion of the LytA molecule found in the C terminal end, for example starting at residue 178 such as residues 188 - 305. In one embodiment, the heterologous fusion partner is CLytA. In a further embodiment, the heterologous fusion partner is CPC, a fusion protein comprising CLytA-P2-CLytA, as described in W003/104272. Purification of hybrid proteins containing the C-LytA fragment at its amino terminus has been described in Biotechnology: 10, (1992) page 795-798.

Fusion proteins of the invention may further include an affinity tag, for example, a histidine tail (also known as a his-tag) comprising between 1 to 10, for example 6 or 10 histidine residues. These residues may, for example, be on the terminal portion, such as the N-terminal and/or the C-terminal portion of the protein. The affinity tag may be incorporated to further improve the purification of the protein.

Certain specific fusion proteins of the invention may, for example, be constructed as described in the Figures Each of the embodiments set forth in the Figures represent independent aspects of the invention. Further examples of constructs of fusion proteins according to the present invention are given in Tables 1-4 and in the Sequence Listing.

*Nucleic Acids.* The present invention also extends to the nucleic acids and polynucleic acids, such as DNA, encoding for fusion proteins of the invention. The processes of the invention may be performed by conventional recombinant techniques such as described in Maniatis et al., Molecular Cloning - A Laboratory Manual; Cold Spring Harbor, 1982-1989. In particular, a process may comprise the steps of:
i) preparing a replicable or integrating expression vector capable, in a host cell, of expressing a DNA polymer comprising a nucleotide sequence that encodes the fusion protein or an immunogenic derivative thereof;
ii) transforming a host cell with said vector;
iii) culturing said transformed host cell under conditions permitting expression of said DNA polymer to produce said protein; and
iv) recovering said protein.

The term 'transforming' is used herein to mean the introduction of foreign DNA into a host cell. This can be achieved, for example by transformation, transfection or infection with an appropriate plasmid or viral vector using e.g. conventional techniques as described in Genetic Engineering; Eds. S.M. Kingsman and A.J. Kingsman; Blackwell Scientific Publications; Oxford, England, 1988. The term 'transformed' or 'transformant' will hereafter apply to the resulting host cell containing and expressing the foreign gene of interest. Expression vectors comprising nucleotide sequences encoding fusion proteins of the present invention are novel and also form part of the invention.

The replicable expression vectors may be prepared in accordance with the invention, by cleaving a vector compatible with the host cell to provide a linear DNA segment having an intact replicon, and combining said linear segment with one or more DNA molecules, which, together with said linear segment encode the desired product, such as the DNA polymer encoding the protein of the invention, or derivative thereof, under ligating conditions. Thus, the DNA polymer may be preformed or formed during the construction of the vector, as desired.

The choice of vector will be determined in part by the host cell, which may be prokaryotic or eukaryotic but are generally E. coli or CHO cells. Suitable vectors include plasmids for example TMCP14 or pET21 or pET26, pcDNA3, bacteriophages, cosmids and recombinant viruses. In one embodiment in which expression is in baculovirus, yeast or CHO host cells, one of the following vectors could be used: pEE14, pPICZA, pPICZB, pPICZC, pDMT-DEST48 and pAcSG2. The preparation of the replicable expression vector may be carried out conventionally with appropriate enzymes for restriction, polymerisation and ligation of the DNA, by procedures described in, for example, Maniatis et.al. cited above.

The recombinant host cell is prepared, in accordance with the invention, by transforming a host cell with a replicable expression vector of the invention under transforming conditions. Suitable transforming conditions are conventional and are described in, for example, Maniatis et al. cited above, or "DNA Cloning" Vol. II, D.M. Glover ed., IRL Press Ltd, 1985. The choice of transforming conditions is determined by the host cell. Thus, a bacterial host such as E. coli may be treated with a solution of CaCl₂ (Cohen et al., Proc. Nat. Acad. Sci., 1973, 69, 2110) or with a solution comprising a mixture of RbCl, MnCl₂, potassium acetate and glycerol, and then with 3-[N-morpholino]-propane-sulphonic acid, RbCl and glycerol. Mammalian cells in culture may be transformed by calcium co-precipitation of the vector DNA onto the cells. The invention also extends to a host cell transformed with a replicable expression vector of the invention.

The DNA may be codon optimized by standard techniques to further facilitate expression of the relevant host.

Culturing the transformed host cell under conditions permitting expression of the DNA polymer is carried out conventionally, as described in, for example, Maniatis et al. and "DNA Cloning" cited above. Thus, preferably the cell is supplied with nutrient and cultured at a temperature below 50°C. The proteins of the present invention may be expressed in prokaryotes or eukaryotes such as yeast but are often expressed in E. coli. Particular strains of E. coli such as:
■ AR58: a cryptic λ lysogen derived from N99 that is gal E::Tn 10, Δ-8(chlD-pgl),Δ-H1 (cro-chIA),N⁺, and c1857 (ref: Proc.Natl.Acad.Sci.USA vol82,pp.88-92,January 1985 Biochemistry)
■ BLR (DE3) Novagen, WI, USA (catalogue number: 69053-4): BLR is a recA⁻ derivative of BL21, may be employed. Generally a selection marker of, for example kanamycine resistance or ampicillin resistance is incorporated to facilitate identification of the successful incorporation of the recombinant gene/construct into the expression system.

The product is recovered by conventional methods according to the host cell and according to the localisation of the expression product (intracellular or secreted into the culture medium or into the cell periplasm). Thus, where the host cell is bacterial, such as E. coli it may, for example, be lysed physically, chemically or enzymatically and the protein product isolated from the resulting lysate. Where the host cell is mammalian, the product may generally be isolated from the nutrient medium or from cell free extracts. Conventional protein isolation techniques include selective precipitation, adsorption chromatography, and affinity chromatography including a monoclonal antibody affinity column.

The proteins of the present invention are provided either soluble in a liquid form or in a lyophilised form. The present invention also provides pharmaceutical composition such as a vaccine comprising a fusion protein of the present invention and a pharmaceutically acceptable excipient.

When administered, the therapeutic compositions of the present invention can be administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents.

The amounts will depend, of course, on the particular condition being treated, the severity of the condition, the individual patient parameters including age, physical condition, size and weight, the duration of the treatment, the nature of concurrent therapy (if any), the specific route of administration and like factors, within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons. It is generally expected that each human dose will comprise 1 to 1000 µg of protein, and preferably 30 - 300 µg.

In one aspect the pharmaceutical compositions used to administer the fusion proteins of the invention will be a vaccine. The vaccine may optionally contain one or more other tumour-associated antigens, polypeptides and/or peptides. For example, members belonging to the MAGE, LAGE and GAGE families.

The fusion proteins of the present invention may additionally optionally comprise one or more amino acids as 'linkers" between the sequences of the antigen and the fusion partners or fusion partner proteins or between the antigen and a His tail, if present. The amino acids may be unrelated to the sequences of the antigen and/or fusion partner.

Fusion proteins of the present invention, as described herein, may additionally comprise amino acids Met-Asp-Pro at the N-terminal end of the fusion protein sequence. The Met amino acid may be from the original protein D sequence or may be from an unrelated sequence.

The present invention also extends to methods of preparing said vaccines/compositions and to fusion proteins and vaccines/compositions obtained by or obtainable by the methods described.

Vaccine preparation is generally described in Vaccine Design ("The subunit and adjuvant approach" (eds. Powell M.F. & Newman M.J). (1995) Plenum Press New York). Encapsulation within liposomes is described by Fullerton, US Patent 4,235,877.

The fusion proteins of the present invention may be adjuvanted in a vaccine formulation of the invention. Suitable adjuvants include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, or polyphosphazenes. Other known adjuvants include CpG containing oligonucleotides. The oligonucleotides are characterised in that the CpG dinucleotide is unmethylated. Such oligonucleotides are well known and are described in, for example WO 96/02555.

In the formulation of the inventions it may be desirable that the adjuvant composition induces an immune response preferentially of the TH1 type. In one embodiment there is provided an adjuvant system including, for example, a combination of monophosphoryl lipid A, preferably 3-de-O-acylated monophosphoryl lipid A (3D-MPL) together with an aluminium salt. CpG oligonucleotides may also induce a TH1 response and may also be included.

In one embodiment there is provided a composition comprising a fusion protein as described herein and an adjuvant composition comprising the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. One formulation that may be used comprises QS21, 3D-MPL & tocopherol in, for example, an oil in water emulsion is described in WO 95/17210. Another adjuvant formulation for use in the present invention may comprise QS21, 3D-MPL & CpG or equivalent thereof, for example, in an oil in water emulsion or as a liposomal formulation. Accordingly in one embodiment of the present invention there is provided a vaccine comprising a fusion protein of the invention and an adjuvant, for example as described above. The present invention also extends to methods of preparing vaccines and compositions comprising fusion proteins as described herein.

The present invention also contemplates delivery of nucleic acids, polypeptides or peptides as described herein for vaccination. Delivery of polypeptides and peptides can be accomplished according to standard vaccination protocols which are well known in the art. In another embodiment, the delivery of nucleic acid may be accomplished by ex vivo methods, i.e. by removing a cell from a subject, genetically engineering the cell to include a cancer associated antigen, and reintroducing the engineered cell into the subject. In general, this may involve introduction in vitro of a functional copy of a gene into a cell(s) of a subject, and returning the genetically engineered cell(s) to the subject. The functional copy of the gene is under operable control of regulatory elements, which permit expression of the gene in the genetically engineered cell(s). Numerous transfection and transduction techniques as well as appropriate expression vectors are well known to those of ordinary skill in the art, some of which are described in PCT application WO 95/00654. In vivo nucleic acid delivery using vectors such as viruses and targeted liposomes also is contemplated according to the invention.

### Abbreviations

- CO: collagen-like region
- W/Ocoll: without collagen-like region (collagen-like domain)
- PD1/3: protein D first third

Exemplary embodiments of fusion proteins and the nucleotide sequence encoding same are provided in Tables 1-3.

**Table 1. Exemplary embodiments of fusion proteins and the nucleotide sequence encoding same are provided. Each nucleotide sequence is described by subject matter, identified by unique nucleotide sequence identifier (SEQ ID NO:), and set forth in the sequence listing. Each fusion protein is described by subject matter, identified by unique amino acid sequence identifier (SEQ ID NO:), and set forth in the sequence listing.**

| **SEQ ID NO:** | **TABLE 1 CONSTRUCT DESCRIPTION** | **SEQUENCE COMPONENTS** |
|---|---|---|
| **Hybrid Collagen NY-ESO-1/LAGE1 a without collagen** | | |

| **1** | **Embodiment A - nucleotide sequence Hybrid Coll NY-ESO-1/LAGE1a WO coll (codon optimised)** | |
|---|---|---|
| | Collagen like domain | 1-210bp |
| | NY-ESO-1 | 1-537bp |
| | Linker | 538-543bp |
| | LAGE1a | 544-846bp |
| | His-tag | 847-864bp |
| | Stop | 865-867bp |

| **2** | **Embodiment B - nucleotide sequence 1/3 protein D/Hybrid Coll NY-ESO-1/LAGE1a WO coll (codon optimised)** | |
|---|---|---|
| | MDP initiation sequence | 1-9bp |
| | 1/3 protein D | 10-333bp |
| | Collagen like domain | 334-540bp |
| | NY-ESO-1 | 334-867bp |
| | Linker | 868-873bp |
| | LAGE1a | 874-1176bp |
| | His-tag | 1177-1194bp |
| | Stop | 1195-1197bp |

| **3** | **Embodiment A - amino acid sequence Hybrid Coll NY-ESO-1/LAGE1a WO coll with His-tag** | |
|---|---|---|
| | Collagen like domain | 1-70aa |
| | NY-ESO-1 | 1-179aa |
| | Linker | 180-181aa |

| **SEQ ID NO:** | **TABLE 1 CONSTRUCT DESCRIPTION** | **SEQUENCE COMPONENTS** |
|---|---|---|
| | LAGE1a | 182-282aa |
| | His-tag | 283-288aa |

| **4** | **Embodiment B - amino acid sequence 1/3 protein D/Hybrid Coll NY-ESO-1/LAGE1a WO coll with His-tag** | |
|---|---|---|
| | MDP initiation sequence | 1-3aa |
| | 1/3 protein D | 4-111aa |
| | Collagen like domain | 112-180aa |
| | NY-ESO-1 | 112-289aa |
| | Linker | 290-291 aa |
| | LAGE1a | 292-392aa |
| | His-tag | 393-398aa |

| **Hybrid Collagen truncated NY-ESO-1/LAGE1a without collagen** | | |
|---|---|---|
| **5** | **Embodiment C - Hybrid Coll trunc NY-ESO-1/LAGE1a WO coll (codon optimised)** | |
| | Collagen like domain | 1-72bp |
| | NY-ESO-1 | 1-399bp |
| | Linker | 400-405bp |
| | LAGE1a | 406-708bp |
| | His-tag | 709-726bp |
| | Stop | 727-729bp |

| **6** | **Embodiment D - nucleotide sequence-- 1/3 protein D/Hybrid Coll trunc NY-ESO-1/LAGE1a WO coll (codon optimised)** | |
|---|---|---|
| | MDP initiation sequence | 1-9bp |
| | 1/3 protein D | 10-333bp |
| | Collagen like domain | 334-402bp |
| | NY-ESO-1 | 334-729bp |
| | Linker | 730-735bp |
| | LAGE1a | 736-1038bp |
| | His-tag | 1039-1056bp |
| | Stop | 1057-1059bp |

| **SEQ ID NO:** | **TABLE 1 CONSTRUCT DESCRIPTION** | **SEQUENCE COMPONENTS** |
|---|---|---|
| **7** | **Embodiment C - Hybrid Coll trunc NY-ESO-1/LAGE1a WO coll with His-tag** | |
| | Collagen like domain | 1-24aa |
| | NY-ESO-1 | 1-133aa |
| | Linker | 134-135aa |
| | LAGE1a | 136-236aa |
| | His-tag | 237-242aa |

| **8** | **Embodiment D - amino acid sequence 1/3 protein D/Hybrid Coll trunc NY-ESO-1/LAGE1a WO coll with His-tag** | |
|---|---|---|
| | MDP initiation sequence | 1-3aa |
| | 1/3 protein D | 4-111 aa |
| | Collagen like domain | 112-134aa |
| | NY-ESO-1 | 112-243aa |
| | Linker | 244-245aa |
| | LAGE1a | 246-346aa |
| | His-tag | 347-352aa |

| **Hybrid NY-ESO-1/LAGE1a without collagen like domain and contiguous cystein rich region (8aa)** | | |
|---|---|---|
| **9** | **Embodiment E - nucleotide sequence Hybrid NY-ESO-1/LAGE1a WO coll (codon optimised)** | |
| | NY-ESO-1 | 1-306bp |
| | Linker | 307-312bp |
| | LAGE1a | 313-615bp |
| | His-tag | 616-633bp |
| | Stop | 634-636bp |

| **SEQ ID NO:** | **TABLE 1 CONSTRUCT DESCRIPTION** | **SEQUENCE COMPONENTS** |
|---|---|---|
| **10** | **Embodiment F - nucleotide sequence 1/3 protein D/Hybrid NY-ESO-1/LAGE1a WO coll (codon optimised)** | |
| | MDP initiation sequence | 1-9bp |
| | 1/3 protein D | 10-333bp |
| | NY-ESO-1 | 334-636bp |
| | Linker | 637-642bp |
| | LAGE1a | 643-945bp |
| | His-tag | 946-963bp |
| | Stop | 964-966bp |

| **11** | **Embodiment E - amino acid sequence Hybrid NY-ESO-1/LAGE1a WO coll with His-tag** | |
|---|---|---|
| | NY-ESO-1 | 1-102aa |
| | Linker | 103-104aa |
| | LAGE1 a | 105-205aa |
| | His-tag | 206-211 aa |

| **12** | **Embodiment F - amino acid sequence 1/3 protein D/Hybrid NY-ESO-1/LAGE1a WO coll with His-tag** | |
|---|---|---|
| | MDP initiation sequence | 1-3aa |
| | 1/3 protein D | 4-111 aa |
| | NY-ESO-1 | 112-212aa |
| | Linker | 213-214aa |
| | LAGE1a | 215-315aa |
| | His-tag | 316-321 aa |

| **Hybrid Collagen LAGEla/NY-ESO-1 without collagen** | | |
|---|---|---|
| **13** | **Embodiment G - nucleotide sequence Hybrid Coll LAGE1a/NY-ESO-1 WO coll (codon optimised)** | |
| | Collagen like domain of NY-ESO-1 | 1-210bp |
| | LAGE1a | 211-540bp |
| | Linker | 541-546bp |
| | NY-ESO-1 | 547-849bp |
| | His-tag | 850-867bp |
| | Stop | 868-870bp |

| **SEQ ID NO:** | **TABLE 1 CONSTRUCT DESCRIPTION** | **SEQUENCE COMPONENTS** |
|---|---|---|
| **14** | **1/3 protein D/Hybrid Coll LAGE1a/NY-ESO-1 WO coil (codon optimised)** | |
| | MDP initiation sequence | 1-9bp |
| | 1/3 protein D | 10-333bp |
| | Collagen like domain of NY-ESO-1 | 334-540bp |
| | LAGE1a | 541-870bp |
| | Linker | 871-876bp |
| | NY-ESO-1 | 877-1179bp |
| | His-tag | 1180-1197bp |
| | Stop | 1198-1200bp |

| **15** | **Embodiment G - amino acid sequence Hybrid Coll LAGE1a/NY-ESO-1 WO coll with His-tag** | |
|---|---|---|
| | Collagen like domain of NY-ESO-1 | 1-70aa |
| | LAGE1a | 71-180aa |
| | Linker | 181-182aa |
| | NY-ESO-1 | 183-283aa |
| | His-tag | 284-289aa |

| **16** | **1/3 protein D/Hybrid Coll LAGE1a/NY-ESO-1 WO coll with His-tag (encoded by SEQ ID NO:14)** | |
|---|---|---|
| | MDP initiation sequence | 1-3aa |
| | 1/3 protein D | 4-111aa |
| | Collagen like domain of NY-ESO-1 | 112-180aa |
| | LAGE1a | 181-290aa |
| | Linker | 291-292aa |
| | NY-ESO-1 | 293-393aa |
| | His-tag | 394-399aa |

| **SEQ ID NO:** | **TABLE 1 CONSTRUCT DESCRIPTION** | **SEQUENCE COMPONENTS** |
|---|---|---|
| **Hybrid Collagen truncated LAGE1a/NY-ESO-1 without collagen** | | |
| **17** | **Hybrid Coll trunc LAGE1a/NY-ESO-1 WO coll (codon optimised)** | |
| | Collagen like domain of NY-ESO-1 | 1-72bp |
| | LAGE1a | 73-402bp |
| | Linker | 403-408bp |
| | NY-ESO-1 | 409-711bp |
| | His-tag | 712-729bp |
| | Stop | 730-732bp |

| **18** | **1/3 protein D/Hybrid Coll trunc LAGE1a/NY-ESO-1 WO coil (codon optimised)** | |
|---|---|---|
| | MDP initiation sequence | 1-9bp |
| | 1/3 protein D | 10-333bp |
| | Collagen like domain of NY-ESO-1 | 334-402bp |
| | LAGE1a | 403-732bp |
| | Linker | 733-738bp |
| | NY-ESO-1 | 739-1041 bp |
| | His-tag | 1042-1059bp |
| | Stop | 1060-1062bp |

| **19** | **Hybrid Coll trunc LAGE1a/NY-ESO-1 WO coll with His-tag (encoded by SEQ ID NO:17)** | |
|---|---|---|
| | Collagen like domain of NY-ESO-1 | 1-24aa |
| | LAGE1a | 25-134aa |
| | Linker | 135-136aa |
| | NY-ESO-1 | 137-237aa |
| | His-tag | 238-243aa |

| **SEQ ID NO:** | **TABLE 1 CONSTRUCT DESCRIPTION** | **SEQUENCE COMPONENTS** |
|---|---|---|
| **20** | **1/3 protein D/Hybrid Coll trunc LAGE1a/NY-ESO-1 WO coll with His-tag (encoded by SEQ ID NO:18)** | |
| | MDP initiation sequence | 1-3aa |
| | 1/3 protein D | 4-111aa |
| | Collagen like domain of NY-ESO-1 | 112-134aa |
| | LAGE1a | 135-244aa |
| | Linker | 245-246aa |
| | NY-ESO-1 | 247-347aa |
| | His-tag | 348-353aa |

| **Hybrid LAGE1a/NY-ESO-1 without collagen like domain and contiguous cystein rich region (8aa)** | | |
|---|---|---|
| **21** | **Embodiment E' - nucleotide sequence Hybrid LAGE1a/NY-ESO-1 WO coll (codon optimised)** | |
| | LAGE1a | 1-309bp |
| | Linker | 310-315bp |
| | NY-ESO-1 | 316-618bp |
| | His-tag | 619-636bp |
| | Stop | 637-639bp |

| **22** | **1/3 protein D/Hybrid LAGE1a/NY-ESO1 WO coll (codon optimised)** | |
|---|---|---|
| | MDP initiation sequence | 1-9bp |
| | 1/3 protein D | 10-333bp |
| | LAGE1a | 334-639bp |
| | Linker | 640-645bp |
| | NY-ESO-1 | 646-948bp |
| | His-tag | 949-966bp |
| | Stop | 967-969bp |

| **23** | **Embodiment E' - amino acid sequence Hybrid LAGE1a/NY-ESO-1 WO coll with His-tag** | |
|---|---|---|
| | LAGE1a | 1-103aa |
| | Linker | 104-105aa |
| | NY-ESO-1 | 106-206aa |
| | His-tag | 207-212aa |

| **SEQ ID NO:** | **TABLE 1 CONSTRUCT DESCRIPTION** | **SEQUENCE COMPONENTS** |
|---|---|---|
| **24** | **1/3 protein D/Hybrid LAGE1a/NY-ESO-1 WO coll with His-tag (encoded by SEQ ID NO:22)** | |
| | MDP initiation sequence | 1-3aa |
| | 1/3 protein D | 4-111aa |
| | LAGE1a | 112-213aa |
| | Linker | 214-215aa |
| | NY-ESO-1 | 216-316aa |
| | His-tag | 317-322aa |

| **His N-terminal Hybrid NY-ESO-1/Lage1a without collagen and contiguous cystein rich region (8aa)** | | |
|---|---|---|
| **25** | **His-Enterokinase site-NY-ESO-1/LAGE1a (codon** | **optimised)** |
| | His-tag sequence | 1-36bp |
| | Enterokinase site | 37-72bp |
| | NY-ESO-1 | 73-375bp |
| | Linker | 376-381 bp |
| | LAGE1a | 382-684bp |
| | Stop | 685-687bp |

| **26** | **His-Enterokinase site-NY-ESO-1/LAGE1a (encoded by SEQ ID NO:25)** | |
|---|---|---|
| | His-tag (10 His) | 1-12aa |
| | Enterokinase site | 13-24aa |
| | NY-ESO-1 | 25-125aa |
| | Linker | 126-127aa |
| | LAGE1a | 128-228aa |

| **27** | **His-NY-ESO-1/LAGE1a (codon optimised)** | |
|---|---|---|
| | His-tag sequence | 1-21 bp |
| | NY-ESO-1 | 22-324bp |
| | Linker | 325-330bp |
| | LAGE1a | 331-633bp |
| | Stop | 634-636bp |

| **SEQ ID NO:** | **TABLE 1 CONSTRUCT DESCRIPTION** | **SEQUENCE COMPONENTS** |
|---|---|---|
| **28** | **His-NY-ESO-1/LAGE1a (encoded by SEQ ID NO:26)** | |
| | His-tag (6 His) | 1-7aa |
| | NY-ESO-1 | 8-108aa |
| | Linker | 109-110aa |
| | LAGE1a | 111-211aa |
| **His-N-terminal Hybrid Collagen truncated NY-ESO-1/ LAGE1a without collagen** | | |
| **29** | **His-Enterokinase site-Coll trunc-NY-ESO-1/LAGE1a optimised)** | **(codon** |
| | His-tag sequence | 1-36bp |
| | Enterokinase site | 37-72bp |
| | Collagen like domain | 73-141 bp |
| | NY-ESO-1 | 73-468bp |
| | Linker | 469-474bp |
| | LAGE1a | 475-777bp |
| | Stop | 778-780bp |
| **30** | **His-Enterokinase site-Coll trunc-NY-ESO-1/LAGE1a SEQ ID NO:29)** | **(encoded by** |
| | His-tag (10 His) | 1-12aa |
| | Enterokinase site | 13-24aa |
| | Collagen like domain | 25-47aa |
| | NY-ESO-1 | 25-156aa |
| | Linker | 157-158aa |
| | LAGE1a | 159-259aa |
| **31** | **His-Coll trunc-NY-ESO-1/LAGE1a (codon optimised)** | |
| | His-tag sequence | 1-21 bp |
| | Collagen like domain | 22-90bp |
| | NY-ESO-1 | 22-417bp |
| | Linker | 418-423bp |
| | LAGE1a | 424-726bp |
| | Stop | 727-729bp |

| **32** | **His-Coll trunc-NY-ESO-1/LAGE1a (encoded by SEQ ID NO:31)** | |
|---|---|---|
| | His-tag (6 His) | 1-7aa |
| | Collagen like domain | 8-30aa |
| | NY-ESO-1 | 31-139aa |
| | Linker | 140-141aa |
| | LAGE1a | 142-242aa |

| **His N-terminal Hybrid collagen NY-ESO-1/LAGE1a without collagen like domain** | | |
|---|---|---|
| **33** | **His-Enterokinase site-Coll-NY-ESO-1/LAGE1a (codon** | **optimised)** |
| | His-tag sequence | 1-36bp |
| | Enterokinase site | 37-72bp |
| | Collagen like domain | 73-279bp |
| | NY-ESO-1 | 73-606bp |
| | Linker | 607-612bp |
| | LAGE1a | 613-915bp |
| | Stop | 916-918bp |

| **34** | **His-Enterokinase site-Coll-NY-ESO-1/LAGE1a (encoded by SEQ ID NO:33)** | |
|---|---|---|
| | His-tag (10 His) | 1-12aa |
| | Enterokinase site | 13-24aa |
| | Collagen like domain | 25-93aa |
| | NY-ESO-1 | 25-202aa |
| | Linker | 203-204aa |
| | LAGE1a | 205-305aa |

| **35** | **His-Coll-NY-ESO-1/LAGE1a (codon optimised)** | |
|---|---|---|
| | His-tag sequence | 1-21 bp |
| | Collagen like domain | 22-228bp |
| | NY-ESO-1 | 22-555bp |
| | Linker | 556-561 bp |
| | LAGE1a | 562-864bp |
| | Stop | 865-867bp |

| **SEQ ID NO:** | **TABLE 1 CONSTRUCT DESCRIPTION** | **SEQUENCE COMPONENTS** |
|---|---|---|
| **36** | **His-Coll-NY-ESO-1/LAGE1a (encoded by SEQ ID NO:35)** | |
| | His-tag (6 His) | 1-7aa |
| | Collagen like domain | 8-76aa |
| | NY-ESO-1 | 77-185aa |
| | Linker | 186-187aa |
| | LAGE1a | 188-288aa |

| **His-N-terminal Hybrid Lage1a/NY-ESO-1 without collagen and contiguous cystein rich region (8aa)** | | |
|---|---|---|
| **37** | **His-Enterokinase site-LAGE1a/NY-ESO-1 (codon optimised)** | |
| | His-tag sequence | 1-36bp |
| | Enterokinase site | 37-72bp |
| | LAGE1a | 73-378bp |
| | Linker | 379-384bp |
| | NY-ESO-1 | 385-687bp |
| | Stop | 688-690bp |

| **38** | **His-Enterokinase site-LAGE1a/NY-ESO-1 (encoded by SEQ ID NO:37)** | |
|---|---|---|
| | His-tag (10 His) | 1-12aa |
| | Enterokinase site | 13-24aa |
| | LAGE1a | 25-126aa |
| | Linker | 127-128aa |
| | NY-ESO-1 | 129-229aa |

| **39** | **His-LAGE1a/NY-ESO-1 (codon optimised)** | |
|---|---|---|
| | His-tag sequence | 1-21 bp |
| | LAGE1a | 22-327bp |
| | Linker | 328-333bp |
| | NY-ESO-1 | 334-636bp |
| | Stop | 637-639bp |

| **SEQ ID NO:** | **TABLE 1 CONSTRUCT DESCRIPTION** | **SEQUENCE COMPONENTS** |
|---|---|---|
| **40** | **His-LAGE1a/NY-ESO-1 (encoded by SEQ ID NO:39)** | |
| | His-tag (6 His) | 1-7aa |
| | LAGE1a | 8-109aa |
| | Linker | 110-111aa |
| | NY-ESO-1 | 112-212aa |

| **His-N-terminal Hybrid Collagen truncated LAGE1a/NY-ESO-1 without collagen** | | |
|---|---|---|
| **41** | **His-Enterokinase site-Coll trunc-LAGE1a/NY-ESO-1 (codon optimised)** | |
| | His-tag sequence | 1-36bp |
| | Enterokinase site | 37-72bp |
| | Collagen like domain of NY-ESO-1 | 73-141 bp |
| | LAGE1a | 142-471bp |
| | Linker | 472-477bp |
| | NY-ESO-1 | 478-780bp |
| | Stop | 781-783bp |

| **42** | **His-Enterokinase site-Coll trunc-LAGE1a/NY-ESO-1 (encoded by SEQ ID NO:41)** | |
|---|---|---|
| | His-tag (10 His) | 1-12aa |
| | Enterokinase site | 13-24aa |
| | Collagen like domain of NY-ESO-1 | 25-47aa |
| | LAGE1a | 48-157aa |
| | Linker | 158-159aa |
| | NY-ESO-1 | 160-260aa |

| **43** | **His-Coll trunc-LAGE1a/NY-ESO-1 (codon optimised)** | |
|---|---|---|
| | His-tag sequence | 1-21bp |
| | Collagen like domain of NY-ESO-1 | 22-90bp |
| | LAGE1a | 91-420bp |
| | Linker | 421-426bp |
| | NY-ESO-1 | 427-729bp |
| | Stop | 730-732bp |

| **SEQ ID NO:** | **TABLE 1 CONSTRUCT DESCRIPTION** | **SEQUENCE COMPONENTS** |
|---|---|---|
| **44** | **His-Coll trunc-LAGE1a/NY-ESO-1 (encoded by SEQ ID NO:43)** | |
| | His-tag (6 His) | 1-7aa |
| | Collagen like domain of NY-ESO-1 | 8-30aa |
| | LAGE1a | 31-140aa |
| | Linker | 141-142aa |
| | NY-ESO-1 | 143-243aa |

| **His N-terminal Hybrid collagen LAGE1a/NY-ESO-1 without collagen like domain** | | |
|---|---|---|
| **45** | **His-Enterokinase site-Coll-LAGE1a/NY-ESO-1 (codon optimised)** | |
| | His-tag sequence | 1-36bp |
| | Enterokinase site | 37-72bp |
| | Collagen like domain of NY-ESO-1 | 73-279bp |
| | LAGE1a | 280-609bp |
| | Linker | 610-615bp |
| | NY-ESO-1 | 616-918bp |
| | Stop | 919-921 bp |

| **46** | **His-Enterokinase site-Coll-LAGE1a/NY-ESO-1 (encoded by SEQ ID NO:45)** | |
|---|---|---|
| | His-tag (10 His) | 1-12aa |
| | Enterokinase site | 13-24aa |
| | Collagen like domain of NY-ESO-1 | 25-93aa |
| | LAGE1a | 94-203aa |
| | Linker | 204-205aa |
| | NY-ESO-1 | 206-306aa |

| **47** | **His-Coll-LAGE1a/NY-ESO-1 (codon optimised)** | |
|---|---|---|
| | His-tag sequence | 1-21bp |
| | Collagen like domain of NY-ESO-1 | 22-228bp |
| | LAGE1a | 229-558bp |
| | Linker | 559-564bp |
| | NY-ESO-1 | 565-867bp |
| | Stop | 868-870bp |

| **SEQ ID NO:** | **TABLE 1 CONSTRUCT DESCRIPTION** | **SEQUENCE COMPONENTS** |
|---|---|---|
| **48** | **His-Coll-LAGE1a/NY-ESO-1 (encoded by SEQ ID NO:47)** | |
| | His-tag (6 His) | 1-7aa |
| | Collagen like domain of NY-ESO-1 | 8-76aa |
| | LAGE1a | 77-186aa |
| | Linker | 187-188aa |
| | NY-ESO-1 | 189-289aa |

**Table 2. Additional exemplary embodiments of fusion proteins and the nucleotide sequences encoding same are provided. Each nucleotide sequence is described by subject matter, identified by unique nucleotide sequence identifier (SEQ ID NO:), and set forth in the sequence listing. Each fusion protein is described by subject matter, identified by unique amino acid sequence identifier (SEQ ID NO:), and set forth in the sequence listing.**

| **SEQ ID NO:** | **TABLE 2. CONSTRUCT DESCRIPTION** | **SEQUENCE COMPONENTS** |
|---|---|---|
| **Partially truncated collagen NY-ESO-1** | | |
| **50** | **Coll trunc NY-ESO-1 (codon optimised)** | |
| | Collagen like domain | 1-72bp |
| | NY-ESO-1 | 1-399bp |
| | His-tag | 400-417bp |
| | Stop | 418-420bp |

| **51** | **Coll trunc NY-ESO-1 with His-tag (encoded by SEQ ID NO:50)** | |
|---|---|---|
| | Collagen like domain | 1-24aa |
| | NY-ESO-1 | 1-133aa |
| | His-tag | 134-139aa |

| **52** | **1/3 protein D/Coll trunc NY-ESO-1 (codon optimised)** | |
|---|---|---|
| | MDP initiation sequence | 1-9bp |
| | 1/3 protein D | 10-333bp |
| | Collagen like domain | 334-402bp |
| | NY-ESO-1 | 334-729bp |
| | His-tag | 730-747bp |
| | Stop | 748-750bp |

| **SEQ ID NO:** | **TABLE 2. CONSTRUCT DESCRIPTION** | **SEQUENCE COMPONENTS** |
|---|---|---|
| **53** | **1/3 protein D/Coll trunc NY-ESO-1 with His-tag (encoded by SEQ ID NO:52)** | |
| | MDP initiation sequence | 1-3aa |
| | 1/3 protein D | 4-111aa |
| | Collagen like domain | 112-134aa |
| | NY-ESO-1 | 112-243aa |
| | His-tag | 244-249aa |

| **NY-ESO-1 WO coll** | | |
|---|---|---|
| **54** | **NY-ESO-1 WO coll (codon optimised)** | |
| | NY-ESO-1 | 1-306bp |
| | His-tag | 307-324bp |
| | Stop | 325-327bp |

| **55** | **NY-ESO-1 WO coll with His-tag (encoded by SEQ ID NO:54)** | |
|---|---|---|
| NY-ESO-1 | | 1-102aa |
| His-tag | | 103-108aa |

| **56** | **1/3 protein D/NY-ESO-1 WO coll (codon optimised)** | |
|---|---|---|
| | MDP initiation sequence | 1-9bp |
| | 1/3 protein D | 10-333bp |
| | NY-ESO-1 | 334-636bp |
| | His-tag | 637-654bp |
| | Stop | 655-657bp |

| **57** | **1/3 protein D/NY-ESO-1 WO coll with His-tag (encoded by SEQ ID NO:56)** | |
|---|---|---|
| | MDP initiation sequence | 1-3aa |
| | 1/3 protein D | 4-111aa |
| | NY-ESO-1 | 112-212aa |
| | His-tag | 213-218aa |

**Table 3. Additional exemplary embodiments of fusion proteins and the nucleotide sequence encoding same are provided. Each nucleotide sequence is described by subject matter, identified by unique nucleotide sequence identifier (SEQ ID NO:), and set forth in the sequence listing. Each fusion protein is described by subject matter, identified by unique amino acid sequence identifier (SEQ ID NO:), and set forth in the sequence listing.**

| **SEQ ID NO:** | **TABLE 3 DESCRIPTION** | **SEQUENCE COMPONENTS** |
|---|---|---|
| **Hybrid Collagen LAGE-1a without LAGE-1a collagen-like domain** | | |
| **59** | **Hybrid Coll LAGE-1a WO coll (codon optimised)** | |
| | Collagen like domain of NY-ESO-1 | 1-210bp |
| | LAGE-1a | 211-540bp |
| | His-tag | 541-558bp |
| | Stop | 559-561bp |

| **60** | **Hybrid Coll LAGE-1a WO coll with His-tag (encoded by SEQ ID NO:59)** | |
|---|---|---|
| | Collagen like domain of NY-ESO-1 | 1-70aa |
| | LAGE-1a | 71-180aa |
| | His-tag | 181-186aa |

| **61** | **1/3 protein D/Hybrid Coll LAGE-1a WO coll with His-tag (codon optimised)** | |
|---|---|---|
| | MDP initiation sequence | 1-9bp |
| | 1/3 protein D | 10-333bp |
| | Collagen like domain of NY-ESO-1 | 334-540bp |
| | LAGE-1a | 541-870bp |
| | His-tag | 871-888bp |
| | Stop | 889-891 bp |

| **62** | **1/3 protein D/Hybrid Coll LAGE-1a WO coll with His-tag (encoded by SEQ ID NO:61)** | |
|---|---|---|
| | MDP initiation sequence | 1-3aa |
| | 1/3 protein D | 4-111aa |
| | Collagen like domain of NY-ESO-1 | 112-180aa |
| | LAGE-1a | 181-290aa |
| | His-tag | 291-296aa |

| **SEQ ID NO:** | **TABLE 3 DESCRIPTION** | **SEQUENCE COMPONENTS** |
|---|---|---|
| **Hybrid Collagen truncated LAGE-1a without collagen-like domain** | | |
| **63** | **Hybrid Collagen truncated LAGE-1a without collagen-like domain** | |
| | Collagen like domain of NY-ESO-1 | 1-72bp |
| | LAGE-1a | 73-402bp |
| | His-tag | 403-420bp |
| | Stop | 421-423bp |

| **64** | **Hybrid Coll trunc LAGE-1a WO coll with His-tag (encoded by SEQ ID NO:63)** | |
|---|---|---|
| | Collagen like domain of NY-ESO-1 | 1-24aa |
| | LAGE-1a | 25-134aa |
| | His-tag | 135-140aa |

| **65** | **1/3 protein D/Hybrid Coll trunc LAGE-1a WO coll with His-tag (codon optimised)** | |
|---|---|---|
| | MDP initiation sequence | 1-9bp |
| | 1/3 protein D | 10-333bp |
| | Collagen like domain of NY-ESO-1 | 334-402bp |
| | LAGE-1a | 403-732bp |
| | His-tag | 733-750bp |
| | Stop | 751-753bp |

| **66** | **1/3 protein D/Hybrid Coll trunc LAGE-1a WO coll with His-tag (encoded by SEQ ID NO:65)** | |
|---|---|---|
| | MDP initiation sequence | 1-3aa |
| | 1/3 protein D | 4-111aa |
| | Collagen like domain of NY-ESO-1 | 112-134aa |
| | LAGE-1a | 135-244aa |
| | His-tag | 245-250aa |

| **LAGE-1a without collagen like domain and contiguous cystein rich region (8aa)** | | |
|---|---|---|
| **67** | **LAGE-1a WO coll (codon optimised)** | |
| LAGE-1 a | | 1-309bp |
| His-tag | | 310-327bp |
| Stop | | 328-330bp |

| **SEQ ID NO:** | **TABLE 3 DESCRIPTION** | **SEQUENCE COMPONENTS** |
|---|---|---|
| **68** | **LAGE-1a WO coll with His-tag (encoded by SEQ ID NO:67)** | |
| LAGE-1a | | 1-103aa |
| His-tag | | 104-109aa |

| **69** | **1/3 protein D/LAGE-1a WO coll (codon optimised)** | |
|---|---|---|
| MDP initiation sequence | | 1-9bp |
| 1/3 protein D | | 10-333bp |
| LAGE-1a | | 334-639bp |
| His-tag | | 640-657bp |
| Stop | | 6578-660bp |

| **70** | **1/3 protein D/LAGE-1a WO coll with His-tag (encoded by SEQ ID NO: 69)** | |
|---|---|---|
| MDP initiation sequence | | 1-3aa |
| 1/3 protein D | | 4-111aa |
| LAGE-1a | | 112-213aa |
| His-tag | | 214-219aa |

**Table 4. The fusion proteins discussed in the Examples and the nucleotide sequences encoding same are provided. Each nucleotide sequence is described by subject matter, identified by unique nucleotide sequence identifier (SEQ ID NO:), and set forth in the sequence listing. Each fusion protein is described by subject matter, identified by unique amino acid sequence identifier (SEQ ID NO:), and set forth in the sequence listing.**

| **TABLE 4** | | |
|---|---|---|
| **CONSTRUCT NAME** | **NUCLEOTIDE SEQUENCE** | **AMINO ACID SEQUENCE** |
| LVL020 | SEQ ID NO:72 | SEQ ID NO:73 |
| LVL024 | SEQ ID NO:74 | SEQ ID NO:75 |
| LVL026 | SEQ ID NO:76 | SEQ ID NO:77 |
| LVL030 | SEQ ID NO:78 | SEQ ID NO:79 |
| LVL068 | SEQ ID NO:80 | SEQ ID NO:81 |
| LVL076 | SEQ ID NO:82 | SEQ ID NO:83 |
| LVL078 | SEQ ID NO:84 | SEQ ID NO:85 |
| LVL079 | SEQ ID NO:86 | SEQ ID NO:87 |
| LVL106 | SEQ ID NO:88 | SEQ ID NO:89 |
| LVL151 | SEQ ID NO:90 | SEQ ID NO:91 |
| LVL155 | SEQ ID NO:92 | SEQ ID NO:93 |
| LVL156 | SEQ ID NO:94 | SEQ ID NO:95 |
| LVL157 | SEQ ID NO:96 | SEQ ID NO:97 |

As will be evident from the sequence listing, many of the constructs of Table 4 have similar designs to one or more embodiment set forth in the preceding tables. For example, LVL068 shares the same design as the embodiment set forth as SEQ ID NO:45, Table 1. LVL076 shares the same design as the embodiment set forth as SEQ ID NO:25, Table 1. LVL078 shares the same design as the embodiment set forth as SEQ ID NO:33, Table 1. LVL079 shares the same design as the embodiment set forth as SEQ ID NO:37, Table 1.

In addition, several of the fusion protein constructs set forth in Table 4, namely LVL155, LVL106, LVL156, LVL157, LVL151, were generated by routine modifications of other fusion protein sequences set forth in Table 4, namely LVL068, LVL030, LVL076, LVL078, LVL024, respectively. Such modifications include the removal of the amino acid residues between protein D and the beginning of the chimers (i.e., the portion derived from either of NY-ESO-1 and LAGE-1) and the removal of the amino acids between the his-tag and the beginning of the chimer. Thus, certain of the fusion proteins of Table 4 correspond closely to other fusion proteins in Table 4. The correspondence between these fusion proteins is set forth in Table 5 and described in greater detail in Example 4.

**Table 5. Correspondence between LVL068, LVL030, LVL076, LVL078, LVL024 and the modified LVL155, LVL106, LVL156, LVL157, LVL151.**

| **TABLE 5** | | | |
|---|---|---|---|
| **FUSION PROTEIN CONSTRUCT** | **CORRESPONDS TO** | | **FUSION PROTEIN CONSTRUCT** |
| LVL068 | | LVL155 | |
| LVL030 | | LVL106 | |
| LVL076 | | LVL156 | |
| LVL078 | | LVL157 | |
| LVL024 | | LVL151 | |

### EXAMPLES

### Example 1. NY-LA 1 chimeric protein design and production

Several NY-ESO-1/LAGE-1 fusion proteins were designed with and without the collagen-Like domain, and with and without the end terminus of protein D as summarized in Figure 17. The designed constructs were codon optimized for expression in Escherichia coli. The synthetic gene was assembled from oligonucleotides and/or PCR products. The fragment was cloned into pGA4 backbone (AmpR) using Kpnl and SacI restriction sites with the addition of Ndel and Xhol sites in the 5' end and the 3' end of the optimized gene respectively.

The plasmid DNA was purified from transformed bacteria and concentration determined by UV spectrometry. The final construct was verified by sequencing. The optimized coding sequence for the different NY/LAGE chimeric constructs was subcloned directly into pET19 (AmpR) multiple cloning site using Ndel and Xhol restriction sites to get the NY/LAGE chimer expression plasmids. For cloning into pET26, PCR primers were designed in order to ad N-terminal Histidine-tail. This amplification resulted in the addition of the 6 Histidines tail in phase with the coding region of the different constructs. This amplified fragment was enzymatically digested with Ndel/Xhol restriction enzymes and the different NY/LAGE chimeric constructs were subsequently cloned into pET26 (KanR) multiple cloning site to get the expression plasmid. The final constructs were verified by sequencing.

### Shake-flask production. Growth and induction of bacterial host strain

### Culture

Bacteria were grown on 800 ml of Luria-Bertani (LB) broth (BD) + 1% (w/v) glucose (Laboratoire MAT, catalogue number: GR-0101) + antibiotic(Carbenicillin 100 µg/ml for pET19, kanamycin 40 µg/ml for pET26), in 2.5L shaking flask. Cultures were incubated at 37°C for BLR (DE3) cells until an O.D.₆₀₀ₙₘ around 0.8.

### Induction

At O.D.₆₀₀ₙₘ around 0.8, the cultures BLR (DE3) were induced at 1 mM isopropyl β-D-1-thiogalactopyranoside (IPTG; EMD Chemicals Inc., catalogue number: 5815) and incubated for 16-18 hours at 16°C. 5 to 15 mg of specific protein/800ml have been obtained with construct LVL106, 151, 155 and 157. The protein production for each construct is summarized in Figure 17.

### Example 2. Summary of preliminary purification and stability

### Extraction and Purification of the Protein

Cells are harvested by centrifugation then disrupted by physical or chemical means and the resulting crude extract retained to isolate the polypeptide of interest.

### Purification

The expressed recombinant proteins were solubilized with guanidine hydrochloride solution and loaded on an Immobilized Metal Affinity Chromatography (IMAC) resin. Proteins were then washed on column with 8M and 4M urea solutions before elution by increasing imidazole concentration. Proteins were then desalted in the final 4M urea buffer, pH 7.0 for further use. Purification was evaluated by SDS PAGE and Western Blot, to verify the purity and the identity of the proteins.

### Stability test of purified fusion protein

Stability assays were performed at 37°C and proteins were evaluated by SDS-PAGE. Preliminary stability assay did not reveal major issue.

### Preliminary solubilization assay

The solubility of the proteins was evaluated as summarized in the following Chart.

| **BUFFER** | **CONSTRUCT** | | | | | | | |
|---|---|---|---|---|---|---|---|---|
| | **LVL 076** | **LVL 079** | **LVL 78** | **LVL 68** | **LVL 020** | **LVL 26** | **LVL 024** | **LVL 30** |
| PBS 1 X; 1 mM TCEP; 1 mM EDTA, pH 7,03 | p | S | S | P | P | P | NT | P |
| 20mM Bicine;138mM NaCl; 1mM TCEP; 1mM EDTA, pH 8,68 | p | S | P | P | P | P | NT | P |
| 20mM imidazole; 138mM NaCl; 1mM TCEP; 1 mM EDTA, pH 5,99 | p | S | P | P | P | P | NT | P |
| 10mM Sodium Ac; 5mM NaCl; 1mM TCEP; 1 mM EDTA, pH 4.99 | S | S | S | S | S | S | NT | S |
| 10mM citrate acid; 5mM NaCl; 1mM TCEP; 1 mM EDTA, pH 3.7 | NT | NT | S | S | NT | NT | NT | S |

| | | | | | | | | |
|---|---|---|---|---|---|---|---|---|
| **Chart 1. Fusion Protein Solubility.** Key: P precipate; S no precipitate; NT not tested. | | | | | | | | |

### Example 3: IM immunization with fusion proteins

The fusion proteins were evaluated preclinically in a mouse model involving a series of intramuscular immunizations screening experiments, as described below. The mouse model chosen was CB6F1, a first generation resulting from the cross of C57BL6 mice and Balb/c mice. Such mice are commercially available from Charles River Laboratories, Inc., 251 Ballardvale Street, Wilmington, MA 01887-1000. The chosen tumor cell line was B16 (Mouse melanoma cell line), a transplantable murine melanoma commercially available for the study of cancer therapies.

### Screening #1

*Experimental design.* In a 76-day trial, CB6F1 mice were used to assess each of LVL076, LVL079, LVL078, LVL068, LVL020, LVL026, LVL024, LVL030 to determine whether intramuscular immunization with the fusion protein plus adjuvant conferred protection against subcutaneous challenge with transplanted tumor cells (B16/NYESO1). Specifically, mice were immunized intramuscularly with 50µL injections containing 15µg protein and an adjuvant. The adjuvant selected was AS15. AS15 is a liposomal adjuvant formulation comprising QS21, 3D-MPL and CpG.

Trials were carried out with fusion proteins set forth in 1A and 1B, below. Mice were divided into groups of 15 mice/group. Mice were immunized on day 0 and again on day 14 as follows:

### Trial 1A

■ LVL079
■ LVL026
■ LVL068
■ LVL030

### Trial 1B

■ LVL076
■ LVL020
■ LVL078
■ LVL024

### Controls

■ Antigen buffer/AS15 buffer
■ Full length NY-ESO-1
■ LAGE-1a without the collagen-like domain (CLD)
■ MAGE A3
Six mice/group were challenged with subcutaneous transplanted B16/NY-ESO-1 tumors on day 28. Antibody response to NY-ESO-1 full-length, LAGE-1a without collagen like domain, and human collagen was assessed at day 0, 14, 28, and 76 by ELISA (IgG1 and IgG2a). Cell-mediated response was assessed by FACS at day 28 using harvested splenocytes (restimulation--3 pools of 3--with NY-ESO-1 and LAGE-1a peptide pools). The experimental design of screening #1 is summarized in Figure 18.

*Results.* Of the four controls, only full-length NY-ESO-1 conferred some protection compared with buffer. See Figure 19. Of the groups of mice receiving either full-length NY-ESO-1 or LVL030, two from each group were tumor free at the end of the trial. Of the mice receiving LVL068, four were tumor free at the end of the study. LVL068 and LVL078 conferred longer survival compared with mice that received buffers. See Figure 20. NY-ESO-1-specific immune responses were assessed by ELISA, FACS, and Western blot. LAGE-1 a(without the collagen like domain)-specific immune responses were assessed by ELISA and FACS. See Figure 21. These results are summarized in the following Chart.

| **Immunogen** | **B16/NY-ESO-1 Protection** | **NY-ESO-1 Specific Immunity** | **LAGE1a Specific Immunity** |
|---|---|---|---|
| **LVL068** | ++ | ++ | ++ |
| **LVL078** | + | ++ | ++ |
| **LVL076** | + | ++ | + |
| **LVL024** | + | ++ | + |
| **LVL030** | + | ++ | + |
| **LVL020** | + | + | + |
| **LVL079** | - | + | + |
| **LVL026** | - | + | + |

| | | | |
|---|---|---|---|
| **Chart 2. Specific Immunity Summary.** Key: (-) - lowest response; (+) - medium response; (++) - highest response. *Screening #2* | | | |

*Experimental design.* In a 105-day trial, CB6F1 mice were used to assess each of LVL076, LVL078, LVL068, and LVL024 to determine whether intramuscular immunization with the fusion protein plus adjuvant conferred protection against subcutaneous challenge with B16/NYESO1 transplanted tumor cells (after two immunizations) or with B16/LAGE-1a tumor cells (after four immunizations). Specifically, mice were immunized intramuscularly with 50µL injections containing 15µg protein and 25µL of AS15 adjuvant.

Mice were divided into groups of 29 mice/group. Mice were immunized on day 0, 14, 28, and 42 as follows:

### Trial

■ LVL076
■ LVL068
■ LVL078
■ LVL024

### Controls

■ Antigen buffer/AS15 buffer
■ Full length NY-ESO-1
■ LAGE-1a without the collagen-like domain (CLD)
■ MAGE A3

Ten mice/group were challenged with subcutaneous transplanted B16/NY-ESO-1 tumor cells on day 28. Nine mice/group were challenged with subcutaneous transplanted B16/LAGE-1A tumor cells on day 56. Sera was taken and antibody response to (i) NY-ESO-1 full-length, (ii) LAGE-1a without collagen like domain, and (ii) human collagen was assessed at day 0, 14, 28, 42 56, 84 and 105 by ELISA (IgG1 and IgG2a). The experimental design of screening #2 is summarized in Figures 22 and 23.

### Results

### B16-NYESO1 Tumor Challenge.

Of the mice receiving LVL078, two were tumor free for over 50 days post B16-NY-ESO-1 challenge. Of the mice receiving either full-length NY-ESO-1 or LVL024, two from each group were tumor free for over 50 days, three were alive. Of the mice receiving LVL068, three were tumor free and four were alive. Of the mice receiving LVL076, 3 were tumor free and five were alive. See Figure 24.

### B16-LAGE1a Tumor Challenge.

All of the mice receiving LVL076 or LAGE-1a without the collagen like region were dead earlier than day 40 post challenge. Of the mice receiving buffer alone, one survived tumor free to the end of the study. Of the mice receiving LVL024, one was tumor free at the end of the study. Of the mice receiving full-length NY-ESO-1, none were tumor free, but one was still alive at the end of the study. Of the mice receiving LVL078, one was tumor free. Of the mice receiving LVL068, three were tumor free. Of the mice receiving LVL076, three were tumor free at the end of the study. See Figure 25. These results are summarized in the following Chart.

| | **NY-ESO-1** | | **LAGE-1a** | |
|---|---|---|---|---|
| **Immunogen** | **Protection** | **Specific Immunity** | **Protection** | **Specific Immunity** |
| **LVL068** | ++ | ++ | ++ | ++ |
| **LVL078** | ++ | ++ | ++ | ++ |
| **LVL024** | **±** | ++ | - | + |
| **LVL076** | + | + | - | + |

| | | | | |
|---|---|---|---|---|
| **Chart 3 Protection versus B16-LAGE1a Tumor Challenge**. Key: (-) - lowest; (±) - next lowest response; (+) - medium response; (++) - highest response. | | | | |

### Human collagen-specific Immune Responses

To study whether the collagen like domain of NY-ESO-1 stimulated human collagen-specific immune responses, sera was collected and pooled 14 days post-inoculation from mice immunized with one of the following: (1) Buffers (control); (2) full-length NY-ESO-1; (3) LAGE-1a without the collagen like domain; (4) LVL068; (5) LVL078; (6) LVL024; (7) LVL076. ELISAs were carried out for each of these seven sera pools, as well as for a positive control containing mAb anti-human collagen I. The collagen like domain did not stimulate mouse anti-human collagen I antibody production. See Figure 26. Similar studies (results not shown) were carried out for collagen III and collagen VI; neither mouse anti-human collagen III nor mouse anti-human collagen VI antibody production were detected.

### Example 4: Refined constructs

Modifications were carried out using routine cloning techniques on some of the constructs listed in Table 4. Specifically, LVL068, LVL030, LVL076, LVL078, LVL024 were modified to yield LVL155, LVL106, LVL156, LVL157, LVL151. There were two kinds of modifications, first the removal of 5 amino acid residues between protein D and the beginning of the chimers. For example, this modification was carried out with LVL024 (SEQ ID NO:74; SEQ ID NO:75) to yield LVL151 (SEQ ID NO:90; SEQ ID NO:91). Thus, LVL024 corresponds with LVL 151. The second type of modification was the removal of the amino acids between the his-tag and the beginning of the chimer. This modification was carried out with LVL068 (SEQ ID NO:80; SEQ ID NO:81) to yield LVL155 (SEQ ID NO:92; SEQ ID NO:93). Thus, LVL068 corresponds with LVL 151. Each fusion protein construct that was modified and the fusion protein construct to which it corresponds is set forth in Table 5 of the Description.

As is understood, the modifications described above are not expected to result in functional differences between a fusion protein and its corresponding modified fusion protein. Thus, it is expected that one may utilize each modified fusion protein listed on the right side of the Table 5 interchangeably with its corresponding fusion protein listed on the left hand side of the chart.

### Example 5.

*Experimental design.* In a 105-day trial, CB6F1 mice are used to assess each of LVL068, LVL030, LVL076, LVL078, LVL024 and the modified LVL155, LVL106, LVL156, LVL157, LVL151 to study intramuscular immunization with the fusion protein plus adjuvant against subcutaneous challenge with B16/NYES01 transplanted tumor cells (after two immunizations) or with B16/LAGE-1 a tumor cells (after four immunizations). Specifically, mice are immunized intramuscularly with 50µL injections containing 15µg protein and 25µL of AS15 adjuvant.

Mice are divided into groups of 29 mice/group. Mice are immunized on day 0, 14, 28, and 42 as follows:

### Trial

■ LVL068
■ LVL030
■ LVL076
■ LVL078
■ LVL024
■ LVL155
■ LVL106
■ LVL156
■ LVL157
■ LVL151

### Controls

■ Antigen buffer/AS15 buffer
■ Full length NY-ESO-1
■ LAGE-1a without the collagen domain
■ MAGE A3
Ten mice/group are challenged with subcutaneous transplanted B16/NY-ESO-1 tumor cells on day 28. Nine mice/group are challenged with subcutaneous transplanted B16/LAGE-1A tumor cells on day 56. To monitor specific immune response, sera can be taken and antibody response measured to (i) NY-ESO-1 full-length, (ii) LAGE-1a without collagen like domain, and (ii) human collagen at day 0, 14, 28, 42 56, 84 and 105 by ELISA (IgG1 and IgG2a).

The preceding examples are provided by way of illustration, not by way of limitation.

Within the present application, the article "a" and "an" are used herein to refer to one or more than one (i.e., to at least one) of the grammatical object of the article. By way of example, "an element" means one or more element. The terms approximately" and "about" as used herein are intended to be optionally deletable or replaceable with the term "exactly", if required by the applicant, in every instance.

Units, prefixes, and symbols may be denoted in their SI accepted form. Unless otherwise indicated, nucleic acids are written left to right in 5' to 3' orientation; amino acid sequences are written left to right in amino to carboxy orientation, respectively. Numeric ranges are inclusive of the numbers defining the range. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The above-defined terms are more fully defined by reference to the specification as a whole.

## Claims

1. A fusion protein comprising:
(a) NY-ESO-1 or a fragment thereof, linked to
(b) LAGE-1 or a fragment thereof,
wherein at least one of NY-ESO-1 and/or LAGE-1 is truncated or partially truncated, or is a fragment including one or more epitopes of NY-ESO-1 or LAGE-1, wherein the fusion protein comprises an amino acid sequence selected from the group consisting of: SEQ ID NO: 73, SEQ ID NO: 75, SEQ ID NO: 79, SEQ ID NO:81, SEQ ID NO: 83, SEQ ID NO:85, SEQ ID NO:89, SEQ ID NO:93, and SEQ ID NO:97.

2. A nucleic acid molecule encoding the fusion protein of claim 1.

3. A vector comprising the nucleic acid molecule of claim 2.

4. A host cell transformed with a vector of claim 3.

5. An immunogenic composition or vaccine comprising a fusion protein according to claim 1, a nucleic acid molecule as claimed in 2 or a vector as claimed in claim 3 additionally comprising an adjuvant, and/or immunostimulatory cytokine or chemokine.

6. An immunogenic composition or vaccine as claimed in claim 5 for use in medicine.

## Patentansprüche

1. Fusionsprotein, umfassend:
(a) NY-ESO-1 oder ein Fragment davon, verknüpft mit
(b) LAGE-1 oder einem Fragment davon,
wobei mindestens eines von NY-ESO-1 und/oder LAGE-1 trunkiert oder teilweise trunkiert ist oder ein Fragment ist, das ein oder mehrere Epitope von NY-ESO-1 oder LAGE-1 einschliesst, wobei das Fusionsprotein eine Aminosäuresequenz umfasst, die ausgewählt ist aus der Gruppe bestehend aus: SEQ ID NO: 73, SEQ ID NO: 75, SEQ ID NO: 79, SEQ ID NO: 81, SEQ ID NO: 83, SEQ ID NO: 85, SEQ ID NO: 89, SEQ ID NO: 93 und SEQ ID NO: 97.

2. Nukleinsäuremolekül, das das Fusionsprotein gemäss Anspruch 1 codiert.

3. Vektor, der das Nukleinsäuremolekül gemäss Anspruch 2 umfasst.

4. Wirtszelle, die mit einem Vektor gemäss Anspruch 3 transformiert ist.

5. Immunogene Zusammensetzung oder Impfstoff, umfassend ein Fusionsprotein gemäss Anspruch 1, ein Nukleinsäuremolekül wie in Anspruch 2 beansprucht oder einen Vektor wie in Anspruch 3 beansprucht, zusätzlich umfassend ein Adjuvans und/oder immunstimulatorisches Cytokin oder Chemokin.

6. Immunogene Zusammensetzung oder Impfstoff gemäss Anspruch 5 zur Verwendung in der Medizin.

## Revendications

1. Protéine de fusion comprenant :
(a) NY-ESO-1 ou l'un de ses fragments, liée à
(b) LAGE-1 ou l'un de ses fragments,
où au moins l'une de NY-ESO-1 et/ou de LAGE-1 est tronquée ou partiellement tronquée, ou est un fragment comprenant un ou plusieurs isotopes de NY-ESO ou de LAGE-1, où la protéine de fusion comprend une séquence d'acides aminés choisie dans le groupe constitué de : SEQ ID NO : 73, SEQ ID NO : 75, SEQ ID NO : 79, SEQ ID NO : 81, SEQ ID NO : 83, SEQ ID NO : 85, SEQ ID NO : 89, SEQ ID NO : 93 et SEQ ID NO : 97.

2. Molécule d'acide nucléique codant pour la protéine de fusion selon la revendication 1.

3. Vecteur comprenant la molécule d'acide nucléique selon la revendication 2.

4. Cellule hôte transformée avec un vecteur selon la revendication 3.

5. Composition immunogène ou vaccin comprenant une protéine de fusion selon la revendication 1, une molécule d'acide nucléique selon la revendication 2 ou un vecteur selon la revendication 3, comprenant en outre un adjuvant et/ou une cytokine ou une chimiokine immunostimulante.

6. Composition immunogène ou vaccin selon la revendication 5 pour une utilisation en médecine.


### SEQUENCE LISTING

<110> Denis Martin Remi Palmantier
<120> Novel Fusion Protein
<130> VB62288
<160> 97
<170> FastSEQ for Windows Version 4.0
<210> 1
   <211> 867
   <212> DNA
   <213> Artificial Sequence
<220>
   <223> NY-ESO-1/LAGE construct
<400> 1
   atgcaggcgg aaggccgtgg caccggtggt agcaccggcg atgcggatgg tccgggcggt 60
   ccgggtattc cggacgggcc tggtggtaat gcgggtgggc caggtgaagc gggtgcgacc 120
   ggtggtcgtg gtccgcgggg ggcaggcgca gcacgtgcat ctggtccggg tggtggtgca 180
   ccgcgcggtc cgcatggtgg tgcggcgagc ggcctgaatg gttgctgccg ttgcggtgcg 240
   cgtggtccgg aaagccgtct gctggaattt tatctggcca tgccgtttgc gaccccgatg 300
   gaagcggaac tggcccgtcg tagcctggct caagatgcac cgccgctgcc ggttccgggc 360
   gtgctgctga aagaatttac cgtgagcggc aacattctga ccattcgtct gacggcggca 420
   gaccatcgtc agctgcaact gagcattagc agctgcctgc aacagctgtc tctgctgatg 480
   tggattaccc agtgctttct gccggtgttt ctggcccagc cgccgtctgg tcaacgtggt 540
   ggcgcgcgtc gtccggattc tcgcctgctg gaactgcata ttaccatgcc gttcagctct 600
   ccaatggagg ccgaattagt gcgtcgcatt ctgagccgtg atgcggcacc gctgccgcgt 660
   ccaggtgcgg ttctgaaaga cttcaccgta tctggcaacc tgctgtttat ccgtctgacc 720
   gcagcggacc accgccaatt acaattatct atcagctctt gtttacaaca actgtcgctg 780
   ttaatgtgga tcactcaatg tttcctgcca gtattcctgg ctcaggcccc gagcggtcag 840
   cgtcgtcacc accaccacca ccactaa                                     867
<210> 2
   <211> 1197
   <212> DNA
   <213> Artificial Sequence
<220>
   <223> NY-ESO-1/LAGE constructs
<400> 2
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa acccaggcgg aaggccgtgg caccggtggt 360
   agcaccggcg atgcggatgg tccgggcggt ccgggtattc cggacgggcc tggtggtaat 420
   gcgggtgggc caggtgaagc gggtgcgacc ggtggtcgtg gtccgcgggg ggcaggcgca 480
   gcacgtgcat ctggtccggg tggtggtgca ccgcgcggtc cgcatggtgg tgcggcgagc 540
   ggcctgaatg gttgctgccg ttgcggtgcg cgtggtccgg aaagccgtct gctggaattt 600
   tatctggcca tgccgtttgc gaccccgatg gaagcggaac tggcccgtcg tagcctggct 660
   caagatgcac cgccgctgcc ggttccgggc gtgctgctga aagaatttac cgtgagcggc 720
   aacattctga ccattcgtct gacggcggca gaccatcgtc agctgcaact gagcattagc 780
   agctgcctgc aacagctgtc tctgctgatg tggattaccc agtgctttct gccggtgttt 840
   ctggcccagc cgccgtctgg tcaacgtggt ggcgcgcgtc gtccggattc tcgcctgctg 900
   gaactgcata ttaccatgcc gttcagctct ccaatggagg ccgaattagt gcgtcgcatt 960
   ctgagccgtg atgcggcacc gctgccgcgt ccaggtgcgg ttctgaaaga cttcaccgta 1020
   tctggcaacc tgctgtttat ccgtctgacc gcagcggacc accgccaatt acaattatct 1080
   atcagctctt gtttacaaca actgtcgctg ttaatgtgga tcactcaatg tttcctgcca 1140
   gtattcctgg ctcaggcccc gagcggtcag cgtcgtcacc accaccacca ccactaa    1197
<210> 3
   <211> 288
   <212> PRT
   <213> Artificial Sequence
<220>
   <223> NY-ESO-1/LAGE constructs
<400> 3
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Gly Gly Ala Arg Arg Pro Asp Ser Arg Leu Leu Glu Leu
               180                 185                 190
   His Ile Thr Met Pro Phe Ser Ser Pro Met Glu Ala Glu Leu Val Arg
           195                 200                 205
   Arg Ile Leu Ser Arg Asp Ala Ala Pro Leu Pro Arg Pro Gly Ala Val
       210                 215                 220
   Leu Lys Asp Phe Thr Val Ser Gly Asn Leu Leu Phe Ile Arg Leu Thr
   225                 230                 235                 240
   Ala Ala Asp His Arg Gln Leu Gln Leu Ser Ile Ser Ser Cys Leu Gln
                   245                 250                 255
   Gln Leu Ser Leu Leu Met Trp Ile Thr Gln Cys Phe Leu Pro Val Phe
               260                 265                 270
   Leu Ala Gln Ala Pro Ser Gly Gln Arg Arg His His His His His His
           275                 280                 285
<210> 4
   <211> 398
   <212> PRT
   <213> Artificial Sequence
<220>
   <223> NY-ESO-1/LAGE constructs
<400> 4
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gln
               100                 105                 110
   Ala Glu Gly Arg Gly Thr Gly Gly Ser Thr Gly Asp Ala Asp Gly Pro
           115                 120                 125
   Gly Gly Pro Gly Ile Pro Asp Gly Pro Gly Gly Asn Ala Gly Gly Pro
       130                 135                 140
   Gly Glu Ala Gly Ala Thr Gly Gly Arg Gly Pro Arg Gly Ala Gly Ala
   145                 150                 155                 160
   Ala Arg Ala Ser Gly Pro Gly Gly Gly Ala Pro Arg Gly Pro His Gly
                   165                 170                 175
   Gly Ala Ala Ser Gly Leu Asn Gly Cys Cys Arg Cys Gly Ala Arg Gly
               180                 185                 190
   Pro Glu Ser Arg Leu Leu Glu Phe Tyr Leu Ala Met Pro Phe Ala Thr
           195                 200                 205
   Pro Met Glu Ala Glu Leu Ala Arg Arg Ser Leu Ala Gln Asp Ala Pro
       210                 215                 220
   Pro Leu Pro Val Pro Gly Val Leu Leu Lys Glu Phe Thr Val Ser Gly
   225                 230                 235                 240
   Asn Ile Leu Thr Ile Arg Leu Thr Ala Ala Asp His Arg Gln Leu Gln
                   245                 250                 255
   Leu Ser Ile Ser Ser Cys Leu Gln Gln Leu Ser Leu Leu Met Trp Ile
               260                 265                 270
   Thr Gln Cys Phe Leu Pro Val Phe Leu Ala Gln Pro Pro Ser Gly Gln
           275                 280                 285
   Arg Gly Gly Ala Arg Arg Pro Asp Ser Arg Leu Leu Glu Leu His Ile
       290                 295                 300
   Thr Met Pro Phe Ser Ser Pro Met Glu Ala Glu Leu Val Arg Arg Ile
   305                 310                 315                 320
   Leu Ser Arg Asp Ala Ala Pro Leu Pro Arg Pro Gly Ala Val Leu Lys
                   325                 330                 335
   Asp Phe Thr Val Ser Gly Asn Leu Leu Phe Ile Arg Leu Thr Ala Ala
               340                 345                 350
   Asp His Arg Gln Leu Gln Leu Ser Ile Ser Ser Cys Leu Gln Gln Leu
           355                 360                 365
   Ser Leu Leu Met Trp Ile Thr Gln Cys Phe Leu Pro Val Phe Leu Ala
       370                 375                 380
   Gln Ala Pro Ser Gly Gln Arg Arg His His His His His His
   385                 390                 395
<210> 5
   <211> 729
   <212> DNA
   <213> Artificial Sequence
<220>
   <223> NY-ESO-1/LAGE constructs
<400> 5
   atggcaggcg cagcacgtgc atctggtccg ggtggtggtg caccgcgcgg tccgcatggt 60
   ggtgcggcga gcggcctgaa tggttgctgc cgttgcggtg cgcgtggtcc ggaaagccgt 120
   ctgctggaat tttatctggc catgccgttt gcgaccccga tggaagcgga actggcccgt 180
   cgtagcctgg ctcaagatgc accgccgctg ccggttccgg gcgtgctgct gaaagaattt 240
   accgtgagcg gcaacattct gaccattcgt ctgacggcgg cagaccatcg tcagctgcaa 300
   ctgagcatta gcagctgcct gcaacagctg tctctgctga tgtggattac ccagtgcttt 360
   ctgccggtgt ttctggccca gccgccgtct ggtcaacgtg gtggcgcgcg tcgtccggat 420
   tctcgcctgc tggaactgca tattaccatg ccgttcagct ctccaatgga ggccgaatta 480
   gtgcgtcgca ttctgagccg tgatgcggca ccgctgccgc gtccaggtgc ggttctgaaa 540
   gacttcaccg tatctggcaa cctgctgttt atccgtctga ccgcagcgga ccaccgccaa 600
   ttacaattat ctatcagctc ttgtttacaa caactgtcgc tgttaatgtg gatcactcaa 660
   tgtttcctgc cagtattcct ggctcaggcc ccgagcggtc agcgtcgtca ccaccaccac 720
   caccactaa                                                         729
<210> 6
   <211> 1059
   <212> DNA
   <213> Artificial Sequence
<220>
   <223> NY-ESO-1/LAGE constructs
<400> 6
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcaggcg cagcacgtgc atctggtccg 360
   ggtggtggtg caccgcgcgg tccgcatggt ggtgcggcga gcggcctgaa tggttgctgc 420
   cgttgcggtg cgcgtggtcc ggaaagccgt ctgctggaat tttatctggc catgccgttt 480
   gcgaccccga tggaagcgga actggcccgt cgtagcctgg ctcaagatgc accgccgctg 540
   ccggttccgg gcgtgctgct gaaagaattt accgtgagcg gcaacattct gaccattcgt 600
   ctgacggcgg cagaccatcg tcagctgcaa ctgagcatta gcagctgcct gcaacagctg 660
   tctctgctga tgtggattac ccagtgcttt ctgccggtgt ttctggccca gccgccgtct 720
   ggtcaacgtg gtggcgcgcg tcgtccggat tctcgcctgc tggaactgca tattaccatg 780
   ccgttcagct ctccaatgga ggccgaatta gtgcgtcgca ttctgagccg tgatgcggca 840
   ccgctgccgc gtccaggtgc ggttctgaaa gacttcaccg tatctggcaa cctgctgttt 900
   atccgtctga ccgcagcgga ccaccgccaa ttacaattat ctatcagctc ttgtttacaa 960
   caactgtcgc tgttaatgtg gatcactcaa tgtttcctgc cagtattcct ggctcaggcc 1020
   ccgagcggtc agcgtcgtca ccaccaccac caccactaa                        1059
<210> 7
   <211> 242
   <212> PRT
   <213> Artificial Sequence
<220>
   <223> NY-ESO-1/LAGE constructs
<400> 7
   Met Ala Gly Ala Ala Arg Ala Ser Gly Pro Gly Gly Gly Ala Pro Arg
    1               5                  10                  15
   Gly Pro His Gly Gly Ala Ala Ser Gly Leu Asn Gly Cys Cys Arg Cys
               20                  25                  30
   Gly Ala Arg Gly Pro Glu Ser Arg Leu Leu Glu Phe Tyr Leu Ala Met
           35                  40                  45
   Pro Phe Ala Thr Pro Met Glu Ala Glu Leu Ala Arg Arg Ser Leu Ala
       50                  55                  60
   Gln Asp Ala Pro Pro Leu Pro Val Pro Gly Val Leu Leu Lys Glu Phe
   65                  70                  75                  80
   Thr Val Ser Gly Asn Ile Leu Thr Ile Arg Leu Thr Ala Ala Asp His
                   85                  90                  95
   Arg Gln Leu Gln Leu Ser Ile Ser Ser Cys Leu Gln Gln Leu Ser Leu
               100                 105                 110
   Leu Met Trp Ile Thr Gln Cys Phe Leu Pro Val Phe Leu Ala Gln Pro
           115                 120                 125
   Pro Ser Gly Gln Arg Gly Gly Ala Arg Arg Pro Asp Ser Arg Leu Leu
       130                 135                 140
   Glu Leu His Ile Thr Met Pro Phe Ser Ser Pro Met Glu Ala Glu Leu
   145                 150                 155                 160
   Val Arg Arg Ile Leu Ser Arg Asp Ala Ala Pro Leu Pro Arg Pro Gly
                   165                 170                 175
   Ala Val Leu Lys Asp Phe Thr Val Ser Gly Asn Leu Leu Phe Ile Arg
               180                 185                 190
   Leu Thr Ala Ala Asp His Arg Gln Leu Gln Leu Ser Ile Ser Ser Cys
           195                 200                 205
   Leu Gln Gln Leu Ser Leu Leu Met Trp Ile Thr Gln Cys Phe Leu Pro
       210                 215                 220
   Val Phe Leu Ala Gln Ala Pro Ser Gly Gln Arg Arg His His His His
   225                 230                 235                 240
   His His
<210> 8
   <211> 352
   <212> PRT
   <213> Artificial Sequence
<220>
   <223> NY-ESO-1/LAGE constructs
<400> 8
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Gly Ala Ala Arg Ala Ser Gly Pro Gly Gly Gly Ala Pro Arg Gly Pro
           115                 120                 125
   His Gly Gly Ala Ala Ser Gly Leu Asn Gly Cys Cys Arg Cys Gly Ala
       130                 135                 140
   Arg Gly Pro Glu Ser Arg Leu Leu Glu Phe Tyr Leu Ala Met Pro Phe
   145                 150                 155                 160
   Ala Thr Pro Met Glu Ala Glu Leu Ala Arg Arg Ser Leu Ala Gln Asp
                   165                 170                 175
   Ala Pro Pro Leu Pro Val Pro Gly Val Leu Leu Lys Glu Phe Thr Val
               180                 185                 190
   Ser Gly Asn Ile Leu Thr Ile Arg Leu Thr Ala Ala Asp His Arg Gln
           195                 200                 205
   Leu Gln Leu Ser Ile Ser Ser Cys Leu Gln Gln Leu Ser Leu Leu Met
       210                 215                 220
   Trp Ile Thr Gln Cys Phe Leu Pro Val Phe Leu Ala Gln Pro Pro Ser
   225                 230                 235                 240
   Gly Gln Arg Gly Gly Ala Arg Arg Pro Asp Ser Arg Leu Leu Glu Leu
                   245                 250                 255
   His Ile Thr Met Pro Phe Ser Ser Pro Met Glu Ala Glu Leu Val Arg
               260                 265                 270
   Arg Ile Leu Ser Arg Asp Ala Ala Pro Leu Pro Arg Pro Gly Ala Val
           275                 280                 285
   Leu Lys Asp Phe Thr Val Ser Gly Asn Leu Leu Phe Ile Arg Leu Thr
       290                 295                 300
   Ala Ala Asp His Arg Gln Leu Gln Leu Ser Ile Ser Ser Cys Leu Gln
   305                 310                 315                 320
   Gln Leu Ser Leu Leu Met Trp Ile Thr Gln Cys Phe Leu Pro Val Phe
                   325                 330                 335
   Leu Ala Gln Ala Pro Ser Gly Gln Arg Arg His His His His His His
               340                 345                 350
<210> 9
   <211> 636
   <212> DNA
   <213> Artificial Sequence
<220>
   <223> NY-ESO-1/LAGE constructs
<400> 9
   atgggtgcgc gtggtccgga aagccgtctg ctggaatttt atctggccat gccgtttgcg 60
   accccgatgg aagcggaact ggcccgtcgt agcctggctc aagatgcacc gccgctgccg 120
   gttccgggcg tgctgctgaa agaatttacc gtgagcggca acattctgac cattcgtctg 180
   acggcggcag accatcgtca gctgcaactg agcattagca gctgcctgca acagctgtct 240
   ctgctgatgt ggattaccca gtgctttctg ccggtgtttc tggcccagcc gccgtctggt 300
   caacgtggtg gcgcgcgtcg tccggattct cgcctgctgg aactgcatat taccatgccg 360
   ttcagctctc caatggaggc cgaattagtg cgtcgcattc tgagccgtga tgcggcaccg 420
   ctgccgcgtc caggtgcggt tctgaaagac ttcaccgtat ctggcaacct gctgtttatc 480
   cgtctgaccg cagcggacca ccgccaatta caattatcta tcagctcttg tttacaacaa 540
   ctgtcgctgt taatgtggat cactcaatgt ttcctgccag tattcctggc tcaggccccg 600
   agcggtcagc gtcgtcacca ccaccaccac cactaa                            636
<210> 10
   <211> 966
   <212> DNA
   <213> Artificial Sequence
<220>
   <223> NY-ESO-1/LAGE constructs
<400> 10
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accggtgcgc gtggtccgga aagccgtctg 360
   ctggaatttt atctggccat gccgtttgcg accccgatgg aagcggaact ggcccgtcgt 420
   agcctggctc aagatgcacc gccgctgccg gttccgggcg tgctgctgaa agaatttacc 480
   gtgagcggca acattctgac cattcgtctg acggcggcag accatcgtca gctgcaactg 540
   agcattagca gctgcctgca acagctgtct ctgctgatgt ggattaccca gtgctttctg 600
   ccggtgtttc tggcccagcc gccgtctggt caacgtggtg gcgcgcgtcg tccggattct 660
   cgcctgctgg aactgcatat taccatgccg ttcagctctc caatggaggc cgaattagtg 720
   cgtcgcattc tgagccgtga tgcggcaccg ctgccgcgtc caggtgcggt tctgaaagac 780
   ttcaccgtat ctggcaacct gctgtttatc cgtctgaccg cagcggacca ccgccaatta 840
   caattatcta tcagctcttg tttacaacaa ctgtcgctgt taatgtggat cactcaatgt 900
   ttcctgccag tattcctggc tcaggccccg agcggtcagc gtcgtcacca ccaccaccac 960
   cactaa                                                             966
<210> 11
   <211> 211
   <212> PRT
   <213> Artificial Sequence
<220>
   <223> NY-ESO-1/LAGE constructs
<400> 11
   Met Gly Ala Arg Gly Pro Glu Ser Arg Leu Leu Glu Phe Tyr Leu Ala
    1               5                  10                  15
   Met Pro Phe Ala Thr Pro Met Glu Ala Glu Leu Ala Arg Arg Ser Leu
               20                  25                  30
   Ala Gln Asp Ala Pro Pro Leu Pro Val Pro Gly Val Leu Leu Lys Glu
           35                  40                  45
   Phe Thr Val Ser Gly Asn Ile Leu Thr Ile Arg Leu Thr Ala Ala Asp
       50                  55                  60
   His Arg Gln Leu Gln Leu Ser Ile Ser Ser Cys Leu Gln Gln Leu Ser
   65                  70                  75                  80
   Leu Leu Met Trp Ile Thr Gln Cys Phe Leu Pro Val Phe Leu Ala Gln
                   85                  90                  95
   Pro Pro Ser Gly Gln Arg Gly Gly Ala Arg Arg Pro Asp Ser Arg Leu
               100                 105                 110
   Leu Glu Leu His Ile Thr Met Pro Phe Ser Ser Pro Met Glu Ala Glu
           115                 120                 125
   Leu Val Arg Arg Ile Leu Ser Arg Asp Ala Ala Pro Leu Pro Arg Pro
       130                 135                 140
   Gly Ala Val Leu Lys Asp Phe Thr Val Ser Gly Asn Leu Leu Phe Ile
   145                 150                 155                 160
   Arg Leu Thr Ala Ala Asp His Arg Gln Leu Gln Leu Ser Ile Ser Ser
                   165                 170                 175
   Cys Leu Gln Gln Leu Ser Leu Leu Met Trp Ile Thr Gln Cys Phe Leu
               180                 185                 190
   Pro Val Phe Leu Ala Gln Ala Pro Ser Gly Gln Arg Arg His His His
           195                 200                 205
   His His His
       210
<210> 12
   <211> 321
   <212> PRT
   <213> Artificial Sequence
<220>
   <223> NY-ESO-1/LAGE constructs
<400> 12
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gly
               100                 105                 110
   Ala Arg Gly Pro Glu Ser Arg Leu Leu Glu Phe Tyr Leu Ala Met Pro
           115                 120                 125
   Phe Ala Thr Pro Met Glu Ala Glu Leu Ala Arg Arg Ser Leu Ala Gln
       130                 135                 140
   Asp Ala Pro Pro Leu Pro Val Pro Gly Val Leu Leu Lys Glu Phe Thr
   145                 150                 155                 160
   Val Ser Gly Asn Ile Leu Thr Ile Arg Leu Thr Ala Ala Asp His Arg
                   165                 170                 175
   Gln Leu Gln Leu Ser Ile Ser Ser Cys Leu Gln Gln Leu Ser Leu Leu
               180                 185                 190
   Met Trp Ile Thr Gln Cys Phe Leu Pro Val Phe Leu Ala Gln Pro Pro
           195                 200                 205
   Ser Gly Gln Arg Gly Gly Ala Arg Arg Pro Asp Ser Arg Leu Leu Glu
       210                 215                 220
   Leu His Ile Thr Met Pro Phe Ser Ser Pro Met Glu Ala Glu Leu Val
   225                 230                 235                 240
   Arg Arg Ile Leu Ser Arg Asp Ala Ala Pro Leu Pro Arg Pro Gly Ala
                   245                 250                 255
   Val Leu Lys Asp Phe Thr Val Ser Gly Asn Leu Leu Phe Ile Arg Leu
               260                 265                 270
   Thr Ala Ala Asp His Arg Gln Leu Gln Leu Ser Ile Ser Ser Cys Leu
           275                 280                 285
   Gln Gln Leu Ser Leu Leu Met Trp Ile Thr Gln Cys Phe Leu Pro Val
       290                 295                 300
   Phe Leu Ala Gln Ala Pro Ser Gly Gln Arg Arg His His His His His
   305                 310                 315                 320
   His
<210> 13
   <211> 870
   <212> DNA
   <213> Artificial Sequence
<220>
   <223> LAGE/NY-ESO-1 constructs
<400> 13
   atgcaggcgg aaggccgtgg tactggcggt agcaccggcg atgcagatgg tccgggcggt 60
   ccgggtattc cggatggtcc gggtggtaat gcaggtggtc caggtgaagc aggtgcgact 120
   ggcggtcgtg gtccacgcgg tgcaggtgca gcgcgtgcat ctggtccagg tggcggtgcg 180
   ccgcgtggcc cgcatggtgg tgcagctagt gcgcaagatg gtcgttgccc gtgtggtgcg 240
   cgtcgtccgg atagccgtct gctggagctg catattacca tgccgtttag cagcccaatg 300
   gaagctgagc tggtgcgtcg tattctgtct cgtgacgcag caccgctgcc acgtccgggt 360
   gcggttctga aagattttac cgtgagcggc aacctgctgt ttattcgtct gaccgcggca 420
   gatcatcgtc agctgcaact gagcattagc agctgcctgc aacagctgtc tctgctgatg 480
   tggattaccc agtgctttct gccggtgttt ctggctcagg cgccgtctgg tcagcgtcgt 540
   ggtggtgccc gtggcccgga atctcgtctg ctggaatttt atctggccat gccgttcgcg 600
   acgccgatgg aagcagagct ggcccgtcgc agcctggctc aggatgcacc gccgctgccg 660
   gttccgggcg tgctgctgaa agaatttacg gttagcggta acattctgac catccgtctg 720
   accgcagcgg accaccgcca actgcaactg tctatcagct cttgcctgca acaactgtcg 780
   ttattaatgt ggatcactca atgtttttta ccagtattcc tggcccaacc gccgagcggc 840
   caacgtcgtc accaccacca ccaccactaa                                  870
<210> 14
   <211> 1200
   <212> DNA
   <213> Artificial Sequence
<220>
   <223> LAGE/NY-ESO-1 constructs
<400> 14
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa acccaggcgg aaggccgtgg tactggcggt 360
   agcaccggcg atgcagatgg tccgggcggt ccgggtattc cggatggtcc gggtggtaat 420
   gcaggtggtc caggtgaagc aggtgcgact ggcggtcgtg gtccacgcgg tgcaggtgca 480
   gcgcgtgcat ctggtccagg tggcggtgcg ccgcgtggcc cgcatggtgg tgcagctagt 540
   gcgcaagatg gtcgttgccc gtgtggtgcg cgtcgtccgg atagccgtct gctggagctg 600
   catattacca tgccgtttag cagcccaatg gaagctgagc tggtgcgtcg tattctgtct 660
   cgtgacgcag caccgctgcc acgtccgggt gcggttctga aagattttac cgtgagcggc 720
   aacctgctgt ttattcgtct gaccgcggca gatcatcgtc agctgcaact gagcattagc 780
   agctgcctgc aacagctgtc tctgctgatg tggattaccc agtgctttct gccggtgttt 840
   ctggctcagg cgccgtctgg tcagcgtcgt ggtggtgccc gtggcccgga atctcgtctg 900
   ctggaatttt atctggccat gccgttcgcg acgccgatgg aagcagagct ggcccgtcgc 960
   agcctggctc aggatgcacc gccgctgccg gttccgggcg tgctgctgaa agaatttacg 1020
   gttagcggta acattctgac catccgtctg accgcagcgg accaccgcca actgcaactg 1080
   tctatcagct cttgcctgca acaactgtcg ttattaatgt ggatcactca atgtttttta 1140
   ccagtattcc tggcccaacc gccgagcggc caacgtcgtc accaccacca ccaccactaa 1200
<210> 15
   <211> 289
   <212> PRT
   <213> Artificial Sequence
<220>
   <223> LAGE/NY-ESO-1 constructs
<400> 15
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Ala Gln Asp Gly Arg Cys Pro Cys Gly Ala
   65                  70                  75                  80
   Arg Arg Pro Asp Ser Arg Leu Leu Glu Leu His Ile Thr Met Pro Phe
                   85                  90                  95
   Ser Ser Pro Met Glu Ala Glu Leu Val Arg Arg Ile Leu Ser Arg Asp
               100                 105                 110
   Ala Ala Pro Leu Pro Arg Pro Gly Ala Val Leu Lys Asp Phe Thr Val
           115                 120                 125
   Ser Gly Asn Leu Leu Phe Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Ala Pro Ser
                   165                 170                 175
   Gly Gln Arg Arg Gly Gly Ala Arg Gly Pro Glu Ser Arg Leu Leu Glu
               180                 185                 190
   Phe Tyr Leu Ala Met Pro Phe Ala Thr Pro Met Glu Ala Glu Leu Ala
           195                 200                 205
   Arg Arg Ser Leu Ala Gln Asp Ala Pro Pro Leu Pro Val Pro Gly Val
       210                 215                 220
   Leu Leu Lys Glu Phe Thr Val Ser Gly Asn Ile Leu Thr Ile Arg Leu
   225                 230                 235                 240
   Thr Ala Ala Asp His Arg Gln Leu Gln Leu Ser Ile Ser Ser Cys Leu
                   245                 250                 255
   Gln Gln Leu Ser Leu Leu Met Trp Ile Thr Gln Cys Phe Leu Pro Val
               260                 265                 270
   Phe Leu Ala Gln Pro Pro Ser Gly Gln Arg Arg His His His His His
           275                 280                 285
   His
<210> 16
   <211> 399
   <212> PRT
   <213> Artificial Sequence
<220>
   <223> LAGE/NY-ESO-1 constructs
<400> 16
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gln
               100                 105                 110
   Ala Glu Gly Arg Gly Thr Gly Gly Ser Thr Gly Asp Ala Asp Gly Pro
           115                 120                 125
   Gly Gly Pro Gly Ile Pro Asp Gly Pro Gly Gly Asn Ala Gly Gly Pro
       130                 135                 140
   Gly Glu Ala Gly Ala Thr Gly Gly Arg Gly Pro Arg Gly Ala Gly Ala
   145                 150                 155                 160
   Ala Arg Ala Ser Gly Pro Gly Gly Gly Ala Pro Arg Gly Pro His Gly
                   165                 170                 175
   Gly Ala Ala Ser Ala Gln Asp Gly Arg Cys Pro Cys Gly Ala Arg Arg
               180                 185                 190
   Pro Asp Ser Arg Leu Leu Glu Leu His Ile Thr Met Pro Phe Ser Ser
           195                 200                 205
   Pro Met Glu Ala Glu Leu Val Arg Arg Ile Leu Ser Arg Asp Ala Ala
       210                 215                 220
   Pro Leu Pro Arg Pro Gly Ala Val Leu Lys Asp Phe Thr Val Ser Gly
   225                 230                 235                 240
   Asn Leu Leu Phe Ile Arg Leu Thr Ala Ala Asp His Arg Gln Leu Gln
                   245                 250                 255
   Leu Ser Ile Ser Ser Cys Leu Gln Gln Leu Ser Leu Leu Met Trp Ile
               260                 265                 270
   Thr Gln Cys Phe Leu Pro Val Phe Leu Ala Gln Ala Pro Ser Gly Gln
           275                 280                 285
   Arg Arg Gly Gly Ala Arg Gly Pro Glu Ser Arg Leu Leu Glu Phe Tyr
       290                 295                 300
   Leu Ala Met Pro Phe Ala Thr Pro Met Glu Ala Glu Leu Ala Arg Arg
   305                 310                 315                 320
   Ser Leu Ala Gln Asp Ala Pro Pro Leu Pro Val Pro Gly Val Leu Leu
                   325                 330                 335
   Lys Glu Phe Thr Val Ser Gly Asn Ile Leu Thr Ile Arg Leu Thr Ala
               340                 345                 350
   Ala Asp His Arg Gln Leu Gln Leu Ser Ile Ser Ser Cys Leu Gln Gln
           355                 360                 365
   Leu Ser Leu Leu Met Trp Ile Thr Gln Cys Phe Leu Pro Val Phe Leu
       370                 375                 380
   Ala Gln Pro Pro Ser Gly Gln Arg Arg His His His His His His
   385                 390                 395
<210> 17
   <211> 732
   <212> DNA
   <213> Artificial Sequence
<220>
   <223> LAGE/NY-ESO-1 constructs
<400> 17
   atggcaggtg cagcgcgtgc atctggtcca ggtggcggtg cgccgcgtgg cccgcatggt 60
   ggtgcagcta gtgcgcaaga tggtcgttgc ccgtgtggtg cgcgtcgtcc ggatagccgt 120
   ctgctggagc tgcatattac catgccgttt agcagcccaa tggaagctga gctggtgcgt 180
   cgtattctgt ctcgtgacgc agcaccgctg ccacgtccgg gtgcggttct gaaagatttt 240
   accgtgagcg gcaacctgct gtttattcgt ctgaccgcgg cagatcatcg tcagctgcaa 300
   ctgagcatta gcagctgcct gcaacagctg tctctgctga tgtggattac ccagtgcttt 360
   ctgccggtgt ttctggctca ggcgccgtct ggtcagcgtc gtggtggtgc ccgtggcccg 420
   gaatctcgtc tgctggaatt ttatctggcc atgccgttcg cgacgccgat ggaagcagag 480
   ctggcccgtc gcagcctggc tcaggatgca ccgccgctgc cggttccggg cgtgctgctg 540
   aaagaattta cggttagcgg taacattctg accatccgtc tgaccgcagc ggaccaccgc 600
   caactgcaac tgtctatcag ctcttgcctg caacaactgt cgttattaat gtggatcact 660
   caatgttttt taccagtatt cctggcccaa ccgccgagcg gccaacgtcg tcaccaccac 720
   caccaccact aa                                                     732
<210> 18
   <211> 1062
   <212> DNA
   <213> Artificial Sequence
<220>
   <223> LAGE/NY-ESO-1 constructs
<400> 18
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcaggtg cagcgcgtgc atctggtcca 360
   ggtggcggtg cgccgcgtgg cccgcatggt ggtgcagcta gtgcgcaaga tggtcgttgc 420
   ccgtgtggtg cgcgtcgtcc ggatagccgt ctgctggagc tgcatattac catgccgttt 480
   agcagcccaa tggaagctga gctggtgcgt cgtattctgt ctcgtgacgc agcaccgctg 540
   ccacgtccgg gtgcggttct gaaagatttt accgtgagcg gcaacctgct gtttattcgt 600
   ctgaccgcgg cagatcatcg tcagctgcaa ctgagcatta gcagctgcct gcaacagctg 660
   tctctgctga tgtggattac ccagtgcttt ctgccggtgt ttctggctca ggcgccgtct 720
   ggtcagcgtc gtggtggtgc ccgtggcccg gaatctcgtc tgctggaatt ttatctggcc 780
   atgccgttcg cgacgccgat ggaagcagag ctggcccgtc gcagcctggc tcaggatgca 840
   ccgccgctgc cggttccggg cgtgctgctg aaagaattta cggttagcgg taacattctg 900
   accatccgtc tgaccgcagc ggaccaccgc caactgcaac tgtctatcag ctcttgcctg 960
   caacaactgt cgttattaat gtggatcact caatgttttt taccagtatt cctggcccaa 1020
   ccgccgagcg gccaacgtcg tcaccaccac caccaccact aa                    1062
<210> 19
   <211> 243
   <212> PRT
   <213> Artificial Sequence
<220>
   <223> LAGE/NY-ESO-1 constructs
<400> 19
   Met Ala Gly Ala Ala Arg Ala Ser Gly Pro Gly Gly Gly Ala Pro Arg
    1               5                  10                  15
   Gly Pro His Gly Gly Ala Ala Ser Ala Gln Asp Gly Arg Cys Pro Cys
               20                  25                  30
   Gly Ala Arg Arg Pro Asp Ser Arg Leu Leu Glu Leu His Ile Thr Met
           35                  40                  45
   Pro Phe Ser Ser Pro Met Glu Ala Glu Leu Val Arg Arg Ile Leu Ser
       50                  55                  60
   Arg Asp Ala Ala Pro Leu Pro Arg Pro Gly Ala Val Leu Lys Asp Phe
   65                  70                  75                  80
   Thr Val Ser Gly Asn Leu Leu Phe Ile Arg Leu Thr Ala Ala Asp His
                   85                  90                  95
   Arg Gln Leu Gln Leu Ser Ile Ser Ser Cys Leu Gln Gln Leu Ser Leu
               100                 105                 110
   Leu Met Trp Ile Thr Gln Cys Phe Leu Pro Val Phe Leu Ala Gln Ala
           115                 120                 125
   Pro Ser Gly Gln Arg Arg Gly Gly Ala Arg Gly Pro Glu Ser Arg Leu
       130                 135                 140
   Leu Glu Phe Tyr Leu Ala Met Pro Phe Ala Thr Pro Met Glu Ala Glu
   145                 150                 155                 160
   Leu Ala Arg Arg Ser Leu Ala Gln Asp Ala Pro Pro Leu Pro Val Pro
                   165                 170                 175
   Gly Val Leu Leu Lys Glu Phe Thr Val Ser Gly Asn Ile Leu Thr Ile
               180                 185                 190
   Arg Leu Thr Ala Ala Asp His Arg Gln Leu Gln Leu Ser Ile Ser Ser
           195                 200                 205
   Cys Leu Gln Gln Leu Ser Leu Leu Met Trp Ile Thr Gln Cys Phe Leu
       210                 215                 220
   Pro Val Phe Leu Ala Gln Pro Pro Ser Gly Gln Arg Arg His His His
   225                 230                 235                 240
   His His His
<210> 20
   <211> 353
   <212> PRT
   <213> Artificial Sequence
<220>
   <223> LAGE/NY-ESO-1 constructs
<400> 20
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Gly Ala Ala Arg Ala Ser Gly Pro Gly Gly Gly Ala Pro Arg Gly Pro
           115                 120                 125
   His Gly Gly Ala Ala Ser Ala Gln Asp Gly Arg Cys Pro Cys Gly Ala
       130                 135                 140
   Arg Arg Pro Asp Ser Arg Leu Leu Glu Leu His Ile Thr Met Pro Phe
   145                 150                 155                 160
   Ser Ser Pro Met Glu Ala Glu Leu Val Arg Arg Ile Leu Ser Arg Asp
                   165                 170                 175
   Ala Ala Pro Leu Pro Arg Pro Gly Ala Val Leu Lys Asp Phe Thr Val
               180                 185                 190
   Ser Gly Asn Leu Leu Phe Ile Arg Leu Thr Ala Ala Asp His Arg Gln
           195                 200                 205
   Leu Gln Leu Ser Ile Ser Ser Cys Leu Gln Gln Leu Ser Leu Leu Met
       210                 215                 220
   Trp Ile Thr Gln Cys Phe Leu Pro Val Phe Leu Ala Gln Ala Pro Ser
   225                 230                 235                 240
   Gly Gln Arg Arg Gly Gly Ala Arg Gly Pro Glu Ser Arg Leu Leu Glu
                   245                 250                 255
   Phe Tyr Leu Ala Met Pro Phe Ala Thr Pro Met Glu Ala Glu Leu Ala
               260                 265                 270
   Arg Arg Ser Leu Ala Gln Asp Ala Pro Pro Leu Pro Val Pro Gly Val
           275                 280                 285
   Leu Leu Lys Glu Phe Thr Val Ser Gly Asn Ile Leu Thr Ile Arg Leu
       290                 295                 300
   Thr Ala Ala Asp His Arg Gln Leu Gln Leu Ser Ile Ser Ser Cys Leu
   305                 310                 315                 320
   Gln Gln Leu Ser Leu Leu Met Trp Ile Thr Gln Cys Phe Leu Pro Val
                   325                 330                 335
   Phe Leu Ala Gln Pro Pro Ser Gly Gln Arg Arg His His His His His
               340                 345                 350
   His
<210> 21
   <211> 639
   <212> DNA
   <213> Artificial Sequence
<220>
   <223> LAGE/NY-ESO-1 constructs
<400> 21
   atgggtgcgc gtcgtccgga tagccgtctg ctggagctgc atattaccat gccgtttagc 60
   agcccaatgg aagctgagct ggtgcgtcgt attctgtctc gtgacgcagc accgctgcca 120
   cgtccgggtg cggttctgaa agatttaccc gtgagcggca acctgctgtt tattcgtctg 180
   accgcggcag atcatcgtca gctgcaactg agcattagca gctgcctgca acagctgtct 240
   ctgctgatgt ggattaccca gtgctttctg ccggtgtttc tggctcaggc gccgtctggt 300
   cagcgtcgtg gtggtgcccg tggcccggaa tctcgtctgc tggaatttta tctggccatg 360
   ccgttcgcga cgccgatgga agcagagctg gcccgtcgca gcctggctca ggatgcaccg 420
   ccgctgccgg ttccgggcgt gctgctgaaa gaatttacgg ttagcggtaa cattctgacc 480
   atccgtctga ccgcagcgga ccaccgccaa ctgcaactgt ctatcagctc ttgcctgcaa 540
   caactgtcgt tattaatgtg gatcactcaa tgttttttac cagtattcct ggcccaaccg 600
   ccgagcggcc aacgtcgtca ccaccaccac caccactaa                        639
<210> 22
   <211> 969
   <212> DNA
   <213> Artificial Sequence
<220>
   <223> LAGE/NY-ESO-1 constructs
<400> 22
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accggtgcgc gtcgtccgga tagccgtctg 360
   ctggagctgc atattaccat gccgtttagc agcccaatgg aagctgagct ggtgcgtcgt 420
   attctgtctc gtgacgcagc accgctgcca cgtccgggtg cggttctgaa agattttacc 480
   gtgagcggca acctgctgtt tattcgtctg accgcggcag atcatcgtca gctgcaactg 540
   agcattagca gctgcctgca acagctgtct ctgctgatgt ggattaccca gtgctttctg 600
   ccggtgtttc tggctcaggc gccgtctggt cagcgtcgtg gtggtgcccg tggcccggaa 660
   tctcgtctgc tggaatttta tctggccatg ccgttcgcga cgccgatgga agcagagctg 720
   gcccgtcgca gcctggctca ggatgcaccg ccgctgccgg ttccgggcgt gctgctgaaa 780
   gaatttacgg ttagcggtaa cattctgacc atccgtctga ccgcagcgga ccaccgccaa 840
   ctgcaactgt ctatcagctc ttgcctgcaa caactgtcgt tattaatgtg gatcactcaa 900
   tgttttttac cagtattcct ggcccaaccg ccgagcggcc aacgtcgtca ccaccaccac 960
   caccactaa                                                         969
<210> 23
   <211> 212
   <212> PRT
   <213> Artificial Sequence
<220>
   <223> LAGE/NY-ESO-1 constructs
<400> 23
   Met Gly Ala Arg Arg Pro Asp Ser Arg Leu Leu Glu Leu His Ile Thr
    1               5                  10                  15
   Met Pro Phe Ser Ser Pro Met Glu Ala Glu Leu Val Arg Arg Ile Leu
               20                  25                  30
   Ser Arg Asp Ala Ala Pro Leu Pro Arg Pro Gly Ala Val Leu Lys Asp
           35                  40                  45
   Phe Thr Val Ser Gly Asn Leu Leu Phe Ile Arg Leu Thr Ala Ala Asp
       50                  55                  60
   His Arg Gln Leu Gln Leu Ser Ile Ser Ser Cys Leu Gln Gln Leu Ser
   65                  70                  75                  80
   Leu Leu Met Trp Ile Thr Gln Cys Phe Leu Pro Val Phe Leu Ala Gln
                   85                  90                  95
   Ala Pro Ser Gly Gln Arg Arg Gly Gly Ala Arg Gly Pro Glu Ser Arg
               100                 105                 110
   Leu Leu Glu Phe Tyr Leu Ala Met Pro Phe Ala Thr Pro Met Glu Ala
           115                 120                 125
   Glu Leu Ala Arg Arg Ser Leu Ala Gln Asp Ala Pro Pro Leu Pro Val
       130                 135                 140
   Pro Gly Val Leu Leu Lys Glu Phe Thr Val Ser Gly Asn Ile Leu Thr
   145                 150                 155                 160
   Ile Arg Leu Thr Ala Ala Asp His Arg Gln Leu Gln Leu Ser Ile Ser
                   165                 170                 175
   Ser Cys Leu Gln Gln Leu Ser Leu Leu Met Trp Ile Thr Gln Cys Phe
               180                 185                 190
   Leu Pro Val Phe Leu Ala Gln Pro Pro Ser Gly Gln Arg Arg His His
           195                 200                 205
   His His His His
       210
<210> 24
   <211> 322
   <212> PRT
   <213> Artificial Sequence
<220>
   <223> LAGE/NY-ESO-1 constructs
<400> 24
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gly
               100                 105                 110
   Ala Arg Arg Pro Asp Ser Arg Leu Leu Glu Leu His Ile Thr Met Pro
           115                 120                 125
   Phe Ser Ser Pro Met Glu Ala Glu Leu Val Arg Arg Ile Leu Ser Arg
       130                 135                 140
   Asp Ala Ala Pro Leu Pro Arg Pro Gly Ala Val Leu Lys Asp Phe Thr
   145                 150                 155                 160
   Val Ser Gly Asn Leu Leu Phe Ile Arg Leu Thr Ala Ala Asp His Arg
                   165                 170                 175
   Gln Leu Gln Leu Ser Ile Ser Ser Cys Leu Gln Gln Leu Ser Leu Leu
               180                 185                 190
   Met Trp Ile Thr Gln Cys Phe Leu Pro Val Phe Leu Ala Gln Ala Pro
           195                 200                 205
   Ser Gly Gln Arg Arg Gly Gly Ala Arg Gly Pro Glu Ser Arg Leu Leu
       210                 215                 220
   Glu Phe Tyr Leu Ala Met Pro Phe Ala Thr Pro Met Glu Ala Glu Leu
   225                 230                 235                 240
   Ala Arg Arg Ser Leu Ala Gln Asp Ala Pro Pro Leu Pro Val Pro Gly
                   245                 250                 255
   Val Leu Leu Lys Glu Phe Thr Val Ser Gly Asn Ile Leu Thr Ile Arg
               260                 265                 270
   Leu Thr Ala Ala Asp His Arg Gln Leu Gln Leu Ser Ile Ser Ser Cys
           275                 280                 285
   Leu Gln Gln Leu Ser Leu Leu Met Trp Ile Thr Gln Cys Phe Leu Pro
       290                 295                 300
   Val Phe Leu Ala Gln Pro Pro Ser Gly Gln Arg Arg His His His His
   305                 310                 315                 320
   His His
<210> 25
   <211> 687
   <212> DNA
   <213> Artificial Sequence
<220>
   <223> NY-ESO-1/LAGE construct
<400> 25
   atgggccatc atcatcatca tcatcatcat catcacagca gcggccatat cgacgacgac 60
   gacaagcata tgggtgcgcg tggtccggaa agccgtctgc tggaatttta tctggccatg 120
   ccgtttgcga ccccgatgga agcggaactg gcccgtcgta gcctggctca agatgcaccg 180
   ccgctgccgg ttccgggcgt gctgctgaaa gaatttaccg tgagcggcaa cattctgacc 240
   attcgtctga cggcggcaga ccatcgtcag ctgcaactga gcattagcag ctgcctgcaa 300
   cagctgtctc tgctgatgtg gattacccag tgctttctgc cggtgtttct ggcccagccg 360
   ccgtctggtc aacgtggtgg cgcgcgtcgt ccggattctc gcctgctgga actgcatatt 420
   accatgccgt tcagctctcc aatggaggcc gaattagtgc gtcgcattct gagccgtgat 480
   gcggcaccgc tgccgcgtcc aggtgcggtt ctgaaagact tcaccgtatc tggcaacctg 540
   ctgtttatcc gtctgaccgc agcggaccac cgccaattac aattatctat cagctcttgt 600
   ttacaacaac tgtcgctgtt aatgtggatc actcaatgtt tcctgccagt attcctggct 660
   caggccccga gcggtcagcg tcgttaa                                     687
<210> 26
   <211> 228
   <212> PRT
   <213> Artificial Sequence
<220>
   <223> NY-ESO-1/LAGE construct
<400> 26
   Met Gly His His His His His His His His His His Ser Ser Gly His
    1               5                  10                  15
   Ile Asp Asp Asp Asp Lys His Met Gly Ala Arg Gly Pro Glu Ser Arg
               20                  25                  30
   Leu Leu Glu Phe Tyr Leu Ala Met Pro Phe Ala Thr Pro Met Glu Ala
           35                  40                  45
   Glu Leu Ala Arg Arg Ser Leu Ala Gln Asp Ala Pro Pro Leu Pro Val
       50                  55                  60
   Pro Gly Val Leu Leu Lys Glu Phe Thr Val Ser Gly Asn Ile Leu Thr
   65                  70                  75                  80
   Ile Arg Leu Thr Ala Ala Asp His Arg Gln Leu Gln Leu Ser Ile Ser
                   85                  90                  95
   Ser Cys Leu Gln Gln Leu Ser Leu Leu Met Trp Ile Thr Gln Cys Phe
               100                 105                 110
   Leu Pro Val Phe Leu Ala Gln Pro Pro Ser Gly Gln Arg Gly Gly Ala
           115                 120                 125
   Arg Arg Pro Asp Ser Arg Leu Leu Glu Leu His Ile Thr Met Pro Phe
       130                 135                 140
   Ser Ser Pro Met Glu Ala Glu Leu Val Arg Arg Ile Leu Ser Arg Asp
   145                 150                 155                 160
   Ala Ala Pro Leu Pro Arg Pro Gly Ala Val Leu Lys Asp Phe Thr Val
                   165                 170                 175
   Ser Gly Asn Leu Leu Phe Ile Arg Leu Thr Ala Ala Asp His Arg Gln
               180                 185                 190
   Leu Gln Leu Ser Ile Ser Ser Cys Leu Gln Gln Leu Ser Leu Leu Met
           195                 200                 205
   Trp Ile Thr Gln Cys Phe Leu Pro Val Phe Leu Ala Gln Ala Pro Ser
       210                 215                 220
   Gly Gln Arg Arg
   225
<210> 27
   <211> 636
   <212> DNA
   <213> Artificial Sequence
<220>
   <223> NY-ESO-1/LAGE construct
<400> 27
   atgcatcatc atcatcatca cggtgcgcgt ggtccggaaa gccgtctgct ggaattttat 60
   ctggccatgc cgtttgcgac cccgatggaa gcggaactgg cccgtcgtag cctggctcaa 120
   gatgcaccgc cgctgccggt tccgggcgtg ctgctgaaag aatttaccgt gagcggcaac 180
   attctgacca ttcgtctgac ggcggcagac catcgtcagc tgcaactgag cattagcagc 240
   tgcctgcaac agctgtctct gctgatgtgg attacccagt gctttctgcc ggtgtttctg 300
   gcccagccgc cgtctggtca acgtggtggc gcgcgtcgtc cggattctcg cctgctggaa 360
   ctgcatatta ccatgccgtt cagctctcca atggaggccg aattagtgcg tcgcattctg 420
   agccgtgatg cggcaccgct gccgcgtcca ggtgcggttc tgaaagactt caccgtatct 480
   ggcaacctgc tgtttatccg tctgaccgca gcggaccacc gccaattaca attatctatc 540
   agctcttgtt tacaacaact gtcgctgtta atgtggatca ctcaatgttt cctgccagta 600
   ttcctggctc aggccccgag cggtcagcgt cgttaa                           636
<210> 28
   <211> 211
   <212> PRT
   <213> Artificial Sequence
<220>
   <223> NY-ESO-1/LAGE construct
<400> 28
   Met His His His His His His Gly Ala Arg Gly Pro Glu Ser Arg Leu
    1               5                  10                  15
   Leu Glu Phe Tyr Leu Ala Met Pro Phe Ala Thr Pro Met Glu Ala Glu
               20                  25                  30
   Leu Ala Arg Arg Ser Leu Ala Gln Asp Ala Pro Pro Leu Pro Val Pro
           35                  40                  45
   Gly Val Leu Leu Lys Glu Phe Thr Val Ser Gly Asn Ile Leu Thr Ile
       50                  55                  60
   Arg Leu Thr Ala Ala Asp His Arg Gln Leu Gln Leu Ser Ile Ser Ser
   65                  70                  75                  80
   Cys Leu Gln Gln Leu Ser Leu Leu Met Trp Ile Thr Gln Cys Phe Leu
                   85                  90                  95
   Pro Val Phe Leu Ala Gln Pro Pro Ser Gly Gln Arg Gly Gly Ala Arg
               100                 105                 110
   Arg Pro Asp Ser Arg Leu Leu Glu Leu His Ile Thr Met Pro Phe Ser
           115                 120                 125
   Ser Pro Met Glu Ala Glu Leu Val Arg Arg Ile Leu Ser Arg Asp Ala
       130                 135                 140
   Ala Pro Leu Pro Arg Pro Gly Ala Val Leu Lys Asp Phe Thr Val Ser
   145                 150                 155                 160
   Gly Asn Leu Leu Phe Ile Arg Leu Thr Ala Ala Asp His Arg Gln Leu
                   165                 170                 175
   Gln Leu Ser Ile Ser Ser Cys Leu Gln Gln Leu Ser Leu Leu Met Trp
               180                 185                 190
   Ile Thr Gln Cys Phe Leu Pro Val Phe Leu Ala Gln Ala Pro Ser Gly
           195                 200                 205
   Gln Arg Arg
       210
<210> 29
   <211> 780
   <212> DNA
   <213> Artificial Sequence
<220>
   <223> NY-ESO-1/LAGE construct
<400> 29
   atgggccatc atcatcatca tcatcatcat catcacagca gcggccatat cgacgacgac 60
   gacaagcata tggcaggcgc agcacgtgca tctggtccgg gtggtggtgc accgcgcggt 120
   ccgcatggtg gtgcggcgag cggcctgaat ggttgctgcc gttgcggtgc gcgtggtccg 180
   gaaagccgtc tgctggaatt ttatctggcc atgccgtttg cgaccccgat ggaagcggaa 240
   ctggcccgtc gtagcctggc tcaagatgca ccgccgctgc cggttccggg cgtgctgctg 300
   aaagaattta ccgtgagcgg caacattctg accattcgtc tgacggcggc agaccatcgt 360
   cagctgcaac tgagcattag cagctgcctg caacagctgt ctctgctgat gtggattacc 420
   cagtgctttc tgccggtgtt tctggcccag ccgccgtctg gtcaacgtgg tggcgcgcgt 480
   cgtccggatt ctcgcctgct ggaactgcat attaccatgc cgttcagctc tccaatggag 540
   gccgaattag tgcgtcgcat tctgagccgt gatgcggcac cgctgccgcg tccaggtgcg 600
   gttctgaaag acttcaccgt atctggcaac ctgctgttta tccgtctgac cgcagcggac 660
   caccgccaat tacaattatc tatcagctct tgtttacaac aactgtcgct gttaatgtgg 720
   atcactcaat gtttcctgcc agtattcctg gctcaggccc cgagcggtca gcgtcgttaa 780
<210> 30
   <211> 259
   <212> PRT
   <213> Artificial Sequence
<220>
   <223> NY-ESO-1/LAGE construct
<400> 30
   Met Gly His His His His His His His His His His Ser Ser Gly His
    1               5                  10                  15
   Ile Asp Asp Asp Asp Lys His Met Ala Gly Ala Ala Arg Ala Ser Gly
               20                  25                  30
   Pro Gly Gly Gly Ala Pro Arg Gly Pro His Gly Gly Ala Ala Ser Gly
           35                  40                  45
   Leu Asn Gly Cys Cys Arg Cys Gly Ala Arg Gly Pro Glu Ser Arg Leu
       50                  55                  60
   Leu Glu Phe Tyr Leu Ala Met Pro Phe Ala Thr Pro Met Glu Ala Glu
   65                  70                  75                  80
   Leu Ala Arg Arg Ser Leu Ala Gln Asp Ala Pro Pro Leu Pro Val Pro
                   85                  90                  95
   Gly Val Leu Leu Lys Glu Phe Thr Val Ser Gly Asn Ile Leu Thr Ile
               100                 105                 110
   Arg Leu Thr Ala Ala Asp His Arg Gln Leu Gln Leu Ser Ile Ser Ser
           115                 120                 125
   Cys Leu Gln Gln Leu Ser Leu Leu Met Trp Ile Thr Gln Cys Phe Leu
       130                 135                 140
   Pro Val Phe Leu Ala Gln Pro Pro Ser Gly Gln Arg Gly Gly Ala Arg
   145                 150                 155                 160
   Arg Pro Asp Ser Arg Leu Leu Glu Leu His Ile Thr Met Pro Phe Ser
                   165                 170                 175
   Ser Pro Met Glu Ala Glu Leu Val Arg Arg Ile Leu Ser Arg Asp Ala
               180                 185                 190
   Ala Pro Leu Pro Arg Pro Gly Ala Val Leu Lys Asp Phe Thr Val Ser
           195                 200                 205
   Gly Asn Leu Leu Phe Ile Arg Leu Thr Ala Ala Asp His Arg Gln Leu
       210                 215                 220
   Gln Leu Ser Ile Ser Ser Cys Leu Gln Gln Leu Ser Leu Leu Met Trp
   225                 230                 235                 240
   Ile Thr Gln Cys Phe Leu Pro Val Phe Leu Ala Gln Ala Pro Ser Gly
                   245                 250                 255
   Gln Arg Arg
<210> 31
   <211> 729
   <212> DNA
   <213> Artificial Sequence
<220>
   <223> NY-ESO-1/LAGE construct
<400> 31
   atgcatcatc atcatcatca cgcaggcgca gcacgtgcat ctggtccggg tggtggtgca 60
   ccgcgcggtc cgcatggtgg tgcggcgagc ggcctgaatg gttgctgccg ttgcggtgcg 120
   cgtggtccgg aaagccgtct gctggaattt tatctggcca tgccgtttgc gaccccgatg 180
   gaagcggaac tggcccgtcg tagcctggct caagatgcac cgccgctgcc ggttccgggc 240
   gtgctgctga aagaatttac cgtgagcggc aacattctga ccattcgtct gacggcggca 300
   gaccatcgtc agctgcaact gagcattagc agctgcctgc aacagctgtc tctgctgatg 360
   tggattaccc agtgctttct gccggtgttt ctggcccagc cgccgtctgg tcaacgtggt 420
   ggcgcgcgtc gtccggattc tcgcctgctg gaactgcata ttaccatgcc gttcagctct 480
   ccaatggagg ccgaattagt gcgtcgcatt ctgagccgtg atgcggcacc gctgccgcgt 540
   ccaggtgcgg ttctgaaaga cttcaccgta tctggcaacc tgctgtttat ccgtctgacc 600
   gcagcggacc accgccaatt acaattatct atcagctctt gtttacaaca actgtcgctg 660
   ttaatgtgga tcactcaatg tttcctgcca gtattcctgg ctcaggcccc gagcggtcag 720
   cgtcgttaa                                                         729
<210> 32
   <211> 242
   <212> PRT
   <213> Artificial Sequence
<220>
   <223> NY-ESO-1/LAGE construct
<400> 32
   Met His His His His His His Ala Gly Ala Ala Arg Ala Ser Gly Pro
    1               5                  10                  15
   Gly Gly Gly Ala Pro Arg Gly Pro His Gly Gly Ala Ala Ser Gly Leu
               20                  25                  30
   Asn Gly Cys Cys Arg Cys Gly Ala Arg Gly Pro Glu Ser Arg Leu Leu
           35                  40                  45
   Glu Phe Tyr Leu Ala Met Pro Phe Ala Thr Pro Met Glu Ala Glu Leu
       50                  55                  60
   Ala Arg Arg Ser Leu Ala Gln Asp Ala Pro Pro Leu Pro Val Pro Gly
   65                  70                  75                  80
   Val Leu Leu Lys Glu Phe Thr Val Ser Gly Asn Ile Leu Thr Ile Arg
                   85                  90                  95
   Leu Thr Ala Ala Asp His Arg Gln Leu Gln Leu Ser Ile Ser Ser Cys
               100                 105                 110
   Leu Gln Gln Leu Ser Leu Leu Met Trp Ile Thr Gln Cys Phe Leu Pro
           115                 120                 125
   Val Phe Leu Ala Gln Pro Pro Ser Gly Gln Arg Gly Gly Ala Arg Arg
       130                 135                 140
   Pro Asp Ser Arg Leu Leu Glu Leu His Ile Thr Met Pro Phe Ser Ser
   145                 150                 155                 160
   Pro Met Glu Ala Glu Leu Val Arg Arg Ile Leu Ser Arg Asp Ala Ala
                   165                 170                 175
   Pro Leu Pro Arg Pro Gly Ala Val Leu Lys Asp Phe Thr Val Ser Gly
               180                 185                 190
   Asn Leu Leu Phe Ile Arg Leu Thr Ala Ala Asp His Arg Gln Leu Gln
           195                 200                 205
   Leu Ser Ile Ser Ser Cys Leu Gln Gln Leu Ser Leu Leu Met Trp Ile
       210                 215                 220
   Thr Gln Cys Phe Leu Pro Val Phe Leu Ala Gln Ala Pro Ser Gly Gln
   225                 230                 235                 240
   Arg Arg
<210> 33
   <211> 918
   <212> DNA
   <213> Artificial Sequence
<220>
   <223> NY-ESO-1/LAGE construct
<400> 33
   atgggccatc atcatcatca tcatcatcat catcacagca gcggccatat cgacgacgac 60
   gacaagcata tgcaggcgga aggccgtggc accggtggta gcaccggcga tgcggatggt 120
   ccgggcggtc cgggtattcc ggacgggcct ggtggtaatg cgggtgggcc aggtgaagcg 180
   ggtgcgaccg gtggtcgtgg tccgcggggg gcaggcgcag cacgtgcatc tggtccgggt 240
   ggtggtgcac cgcgcggtcc gcatggtggt gcggcgagcg gcctgaatgg ttgctgccgt 300
   tgcggtgcgc gtggtccgga aagccgtctg ctggaatttt atctggccat gccgtttgcg 360
   accccgatgg aagcggaact ggcccgtcgt agcctggctc aagatgcacc gccgctgccg 420
   gttccgggcg tgctgctgaa agaatttacc gtgagcggca acattctgac cattcgtctg 480
   acggcggcag accatcgtca gctgcaactg agcattagca gctgcctgca acagctgtct 540
   ctgctgatgt ggattaccca gtgctttctg ccggtgtttc tggcccagcc gccgtctggt 600
   caacgtggtg gcgcgcgtcg tccggattct cgcctgctgg aactgcatat taccatgccg 660
   ttcagctctc caatggaggc cgaattagtg cgtcgcattc tgagccgtga tgcggcaccg 720
   ctgccgcgtc caggtgcggt tctgaaagac ttcaccgtat ctggcaacct gctgtttatc 780
   cgtctgaccg cagcggacca ccgccaatta caattatcta tcagctcttg tttacaacaa 840
   ctgtcgctgt taatgtggat cactcaatgt ttcctgccag tattcctggc tcaggccccg 900
   agcggtcagc gtcgttaa                                                918
<210> 34
   <211> 305
   <212> PRT
   <213> Artificial Sequence
<220>
   <223> NY-ESO-1/LAGE construct
<400> 34
   Met Gly His His His His His His His His His His Ser Ser Gly His
    1               5                  10                  15
   Ile Asp Asp Asp Asp Lys His Met Gln Ala Glu Gly Arg Gly Thr Gly
               20                  25                  30
   Gly Ser Thr Gly Asp Ala Asp Gly Pro Gly Gly Pro Gly Ile Pro Asp
           35                  40                  45
   Gly Pro Gly Gly Asn Ala Gly Gly Pro Gly Glu Ala Gly Ala Thr Gly
       50                  55                  60
   Gly Arg Gly Pro Arg Gly Ala Gly Ala Ala Arg Ala Ser Gly Pro Gly
   65                  70                  75                  80
   Gly Gly Ala Pro Arg Gly Pro His Gly Gly Ala Ala Ser Gly Leu Asn
                   85                  90                  95
   Gly Cys Cys Arg Cys Gly Ala Arg Gly Pro Glu Ser Arg Leu Leu Glu
               100                 105                 110
   Phe Tyr Leu Ala Met Pro Phe Ala Thr Pro Met Glu Ala Glu Leu Ala
           115                 120                 125
   Arg Arg Ser Leu Ala Gln Asp Ala Pro Pro Leu Pro Val Pro Gly Val
       130                 135                 140
   Leu Leu Lys Glu Phe Thr Val Ser Gly Asn Ile Leu Thr Ile Arg Leu
   145                 150                 155                 160
   Thr Ala Ala Asp His Arg Gln Leu Gln Leu Ser Ile Ser Ser Cys Leu
                   165                 170                 175
   Gln Gln Leu Ser Leu Leu Met Trp Ile Thr Gln Cys Phe Leu Pro Val
               180                 185                 190
   Phe Leu Ala Gln Pro Pro Ser Gly Gln Arg Gly Gly Ala Arg Arg Pro
           195                 200                 205
   Asp Ser Arg Leu Leu Glu Leu His Ile Thr Met Pro Phe Ser Ser Pro
       210                 215                 220
   Met Glu Ala Glu Leu Val Arg Arg Ile Leu Ser Arg Asp Ala Ala Pro
   225                 230                 235                 240
   Leu Pro Arg Pro Gly Ala Val Leu Lys Asp Phe Thr Val Ser Gly Asn
                   245                 250                 255
   Leu Leu Phe Ile Arg Leu Thr Ala Ala Asp His Arg Gln Leu Gln Leu
               260                 265                 270
   Ser Ile Ser Ser Cys Leu Gln Gln Leu Ser Leu Leu Met Trp Ile Thr
           275                 280                 285
   Gln Cys Phe Leu Pro Val Phe Leu Ala Gln Ala Pro Ser Gly Gln Arg
       290                 295                 300
   Arg
   305
<210> 35
   <211> 867
   <212> DNA
   <213> Artificial Sequence
<220>
   <223> NY-ESO-1/LAGE construct
<400> 35
   atgcatcatc atcatcatca ccaggcggaa ggccgtggca ccggtggtag caccggcgat 60
   gcggatggtc cgggcggtcc gggtattccg gacgggcctg gtggtaatgc gggtgggcca 120
   ggtgaagcgg gtgcgaccgg tggtcgtggt ccgcgggggg caggcgcagc acgtgcatct 180
   ggtccgggtg gtggtgcacc gcgcggtccg catggtggtg cggcgagcgg cctgaatggt 240
   tgctgccgtt gcggtgcgcg tggtccggaa agccgtctgc tggaatttta tctggccatg 300
   ccgtttgcga ccccgatgga agcggaactg gcccgtcgta gcctggctca agatgcaccg 360
   ccgctgccgg ttccgggcgt gctgctgaaa gaatttaccg tgagcggcaa cattctgacc 420
   attcgtctga cggcggcaga ccatcgtcag ctgcaactga gcattagcag ctgcctgcaa 480
   cagctgtctc tgctgatgtg gattacccag tgctttctgc cggtgtttct ggcccagccg 540
   ccgtctggtc aacgtggtgg cgcgcgtcgt ccggattctc gcctgctgga actgcatatt 600
   accatgccgt tcagctctcc aatggaggcc gaattagtgc gtcgcattct gagccgtgat 660
   gcggcaccgc tgccgcgtcc aggtgcggtt ctgaaagact tcaccgtatc tggcaacctg 720
   ctgtttatcc gtctgaccgc agcggaccac cgccaattac aattatctat cagctcttgt 780
   ttacaacaac tgtcgctgtt aatgtggatc actcaatgtt tcctgccagt attcctggct 840
   caggccccga gcggtcagcg tcgttaa                                     867
<210> 36
   <211> 288
   <212> PRT
   <213> Artificial Sequence
<220>
   <223> NY-ESO-1/LAGE construct
<400> 36
   Met His His His His His His Gln Ala Glu Gly Arg Gly Thr Gly Gly
    1               5                  10                  15
   Ser Thr Gly Asp Ala Asp Gly Pro Gly Gly Pro Gly Ile Pro Asp Gly
               20                  25                  30
   Pro Gly Gly Asn Ala Gly Gly Pro Gly Glu Ala Gly Ala Thr Gly Gly
           35                  40                  45
   Arg Gly Pro Arg Gly Ala Gly Ala Ala Arg Ala Ser Gly Pro Gly Gly
       50                  55                  60
   Gly Ala Pro Arg Gly Pro His Gly Gly Ala Ala Ser Gly Leu Asn Gly
   65                  70                  75                  80
   Cys Cys Arg Cys Gly Ala Arg Gly Pro Glu Ser Arg Leu Leu Glu Phe
                       85                  90                  95
   Tyr Leu Ala Met Pro Phe Ala Thr Pro Met Glu Ala Glu Leu Ala Arg
               100                 105                 110
   Arg Ser Leu Ala Gln Asp Ala Pro Pro Leu Pro Val Pro Gly Val Leu
           115                 120                 125
   Leu Lys Glu Phe Thr Val Ser Gly Asn Ile Leu Thr Ile Arg Leu Thr
       130                 135                 140
   Ala Ala Asp His Arg Gln Leu Gln Leu Ser Ile Ser Ser Cys Leu Gln
   145                 150                 155                 160
   Gln Leu Ser Leu Leu Met Trp Ile Thr Gln Cys Phe Leu Pro Val Phe
                   165                 170                 175
   Leu Ala Gln Pro Pro Ser Gly Gln Arg Gly Gly Ala Arg Arg Pro Asp
               180                 185                 190
   Ser Arg Leu Leu Glu Leu His Ile Thr Met Pro Phe Ser Ser Pro Met
           195                 200                 205
   Glu Ala Glu Leu Val Arg Arg Ile Leu Ser Arg Asp Ala Ala Pro Leu
       210                 215                 220
   Pro Arg Pro Gly Ala Val Leu Lys Asp Phe Thr Val Ser Gly Asn Leu
   225                 230                 235                 240
   Leu Phe Ile Arg Leu Thr Ala Ala Asp His Arg Gln Leu Gln Leu Ser
                   245                 250                 255
   Ile Ser Ser Cys Leu Gln Gln Leu Ser Leu Leu Met Trp Ile Thr Gln
               260                 265                 270
   Cys Phe Leu Pro Val Phe Leu Ala Gln Ala Pro Ser Gly Gln Arg Arg
           275                 280                 285
<210> 37
   <211> 690
   <212> DNA
   <213> Artificial Sequence
<220>
   <223> LAGE/NY-ESO-1 construct
<400> 37
   atgggccatc atcatcatca tcatcatcat catcacagca gcggccatat cgacgacgac 60
   gacaagcata tgggtgcgcg tcgtccggat agccgtctgc tggagctgca tattaccatg 120
   ccgtttagca gcccaatgga agctgagctg gtgcgtcgta ttctgtctcg tgacgcagca 180
   ccgctgccac gtccgggtgc ggttctgaaa gattttaccg tgagcggcaa cctgctgttt 240
   attcgtctga ccgcggcaga tcatcgtcag ctgcaactga gcattagcag ctgcctgcaa 300
   cagctgtctc tgctgatgtg gattacccag tgctttctgc cggtgtttct ggctcaggcg 360
   ccgtctggtc agcgtcgtgg tggtgcccgt ggcccggaat ctcgtctgct ggaattttat 420
   ctggccatgc cgttcgcgac gccgatggaa gcagagctgg cccgtcgcag cctggctcag 480
   gatgcaccgc cgctgccggt tccgggcgtg ctgctgaaag aatttacggt tagcggtaac 540
   attctgacca tccgtctgac cgcagcggac caccgccaac tgcaactgtc tatcagctct 600
   tgcctgcaac aactgtcgtt attaatgtgg atcactcaat gttttttacc agtattcctg 660
   gcccaaccgc cgagcggcca acgtcgttaa                                 690
<210> 38
   <211> 229
   <212> PRT
   <213> Artificial Sequence
<220>
   <223> LAGE/NY-ESO-1 construct
<400> 38
   Met Gly His His His His His His His His His His Ser Ser Gly His
    1               5                  10                  15
   Ile Asp Asp Asp Asp Lys His Met Gly Ala Arg Arg Pro Asp Ser Arg
               20                  25                  30
   Leu Leu Glu Leu His Ile Thr Met Pro Phe Ser Ser Pro Met Glu Ala
           35                  40                  45
   Glu Leu Val Arg Arg Ile Leu Ser Arg Asp Ala Ala Pro Leu Pro Arg
       50                  55                  60
   Pro Gly Ala Val Leu Lys Asp Phe Thr Val Ser Gly Asn Leu Leu Phe
   65                  70                  75                  80
   Ile Arg Leu Thr Ala Ala Asp His Arg Gln Leu Gln Leu Ser Ile Ser
                   85                  90                  95
   Ser Cys Leu Gln Gln Leu Ser Leu Leu Met Trp Ile Thr Gln Cys Phe
               100                 105                 110
   Leu Pro Val Phe Leu Ala Gln Ala Pro Ser Gly Gln Arg Arg Gly Gly
           115                 120                 125
   Ala Arg Gly Pro Glu Ser Arg Leu Leu Glu Phe Tyr Leu Ala Met Pro
       130                 135                 140
   Phe Ala Thr Pro Met Glu Ala Glu Leu Ala Arg Arg Ser Leu Ala Gln
   145                 150                 155                 160
   Asp Ala Pro Pro Leu Pro Val Pro Gly Val Leu Leu Lys Glu Phe Thr
                   165                 170                 175
   Val Ser Gly Asn Ile Leu Thr Ile Arg Leu Thr Ala Ala Asp His Arg
               180                 185                 190
   Gln Leu Gln Leu Ser Ile Ser Ser Cys Leu Gln Gln Leu Ser Leu Leu
           195                 200                 205
   Met Trp Ile Thr Gln Cys Phe Leu Pro Val Phe Leu Ala Gln Pro Pro
       210                 215                 220
   Ser Gly Gln Arg Arg
   225
<210> 39
   <211> 639
   <212> DNA
   <213> Artificial Sequence
<220>
   <223> LAGE/NY-ESO-1 construct
<400> 39
   atgcatcatc atcatcatca cggtgcgcgt cgtccggata gccgtctgct ggagctgcat 60
   attaccatgc cgtttagcag cccaatggaa gctgagctgg tgcgtcgtat tctgtctcgt 120
   gacgcagcac cgctgccacg tccgggtgcg gttctgaaag attttaccgt gagcggcaac 180
   ctgctgttta ttcgtctgac cgcggcagat catcgtcagc tgcaactgag cattagcagc 240
   tgcctgcaac agctgtctct gctgatgtgg attacccagt gctttctgcc ggtgtttctg 300
   gctcaggcgc cgtctggtca gcgtcgtggt ggtgcccgtg gcccggaatc tcgtctgctg 360
   gaattttatc tggccatgcc gttcgcgacg ccgatggaag cagagctggc ccgtcgcagc 420
   ctggctcagg atgcaccgcc gctgccggtt ccgggcgtgc tgctgaaaga atttacggtt 480
   agcggtaaca ttctgaccat ccgtctgacc gcagcggacc accgccaact gcaactgtct 540
   atcagctctt gcctgcaaca actgtcgtta ttaatgtgga tcactcaatg ctttttacca 600
   gtattcctgg cccaaccgcc gagcggccaa cgtcgttaa                        639
<210> 40
   <211> 212
   <212> PRT
   <213> Artificial Sequence
<220>
   <223> LAGE/NY-ESO-1 construct
<400> 40
   Met His His His His His His Gly Ala Arg Arg Pro Asp Ser Arg Leu
    1               5                   10                  15
   Leu Glu Leu His Ile Thr Met Pro Phe Ser Ser Pro Met Glu Ala Glu
               20                  25                  30
   Leu Val Arg Arg Ile Leu Ser Arg Asp Ala Ala Pro Leu Pro Arg Pro
           35                  40                  45
   Gly Ala Val Leu Lys Asp Phe Thr Val Ser Gly Asn Leu Leu Phe Ile
       50                  55                  60
   Arg Leu Thr Ala Ala Asp His Arg Gln Leu Gln Leu Ser Ile Ser Ser
   65                  70                  75                  80
   Cys Leu Gln Gln Leu Ser Leu Leu Met Trp Ile Thr Gln Cys Phe Leu
                   85                  90                  95
   Pro Val Phe Leu Ala Gln Ala Pro Ser Gly Gln Arg Arg Gly Gly Ala
               100                 105                 110
   Arg Gly Pro Glu Ser Arg Leu Leu Glu Phe Tyr Leu Ala Met Pro Phe
           115                 120                 125
   Ala Thr Pro Met Glu Ala Glu Leu Ala Arg Arg Ser Leu Ala Gln Asp
       130                 135                 140
   Ala Pro Pro Leu Pro Val Pro Gly Val Leu Leu Lys Glu Phe Thr Val
   145                 150                 155                 160
   Ser Gly Asn Ile Leu Thr Ile Arg Leu Thr Ala Ala Asp His Arg Gln
                   165                 170                 175
   Leu Gln Leu Ser Ile Ser Ser Cys Leu Gln Gln Leu Ser Leu Leu Met
               180                 185                 190
   Trp Ile Thr Gln Cys Phe Leu Pro Val Phe Leu Ala Gln Pro Pro Ser
           195                 200                 205
   Gly Gln Arg Arg
       210
<210> 41
   <211> 783
   <212> DNA
   <213> Artificial Sequence
<220>
   <223> LAGE/NY-ESO-1 construct
<400> 41
   atgggccatc atcatcatca tcatcatcat catcacagca gcggccatat cgacgacgac 60
   gacaagcata tggcaggtgc agcgcgtgca tctggtccag gtggcggtgc gccgcgtggc 120
   ccgcatggtg gtgcagctag tgcgcaagat ggtcgttgcc cgtgtggtgc gcgtcgtccg 180
   gatagccgtc tgctggagct gcatattacc atgccgttta gcagcccaat ggaagctgag 240
   ctggtgcgtc gtattctgtc tcgtgacgca gcaccgctgc cacgtccggg tgcggttctg 300
   aaagatttta ccgtgagcgg caacctgctg tttattcgtc tgaccgcggc agatcatcgt 360
   cagctgcaac tgagcattag cagctgcctg caacagctgt ctctgctgat gtggattacc 420
   cagtgctttc tgccggtgtt tctggctcag gcgccgtctg gtcagcgtcg tggtggtgcc 480
   cgtggcccgg aatctcgtct gctggaattt tatctggcca tgccgttcgc gacgccgatg 540
   gaagcagagc tggcccgtcg cagcctggct caggatgcac cgccgctgcc ggttccgggc 600
   gtgctgctga aagaatttac ggttagcggt aacattctga ccatccgtct gaccgcagcg 660
   gaccaccgcc aactgcaact gtctatcagc tcttgcctgc aacaactgtc gttattaatg 720
   tggatcactc aatgtttttt accagtattc ctggcccaac cgccgagcgg ccaacgtcgt 780
   taa                                                               783
<210> 42
   <211> 260
   <212> PRT
   <213> Artificial Sequence
<220>
   <223> LAGE/NY-ESO-1 construct
<400> 42
   Met Gly His His His His His His His His His His Ser Ser Gly His
    1               5                  10                  15
   Ile Asp Asp Asp Asp Lys His Met Ala Gly Ala Ala Arg Ala Ser Gly
               20                  25                  30
   Pro Gly Gly Gly Ala Pro Arg Gly Pro His Gly Gly Ala Ala Ser Ala
           35                  40                  45
   Gln Asp Gly Arg Cys Pro Cys Gly Ala Arg Arg Pro Asp Ser Arg Leu
       50                  55                  60
   Leu Glu Leu His Ile Thr Met Pro Phe Ser Ser Pro Met Glu Ala Glu
   65                  70                  75                  80
   Leu Val Arg Arg Ile Leu Ser Arg Asp Ala Ala Pro Leu Pro Arg Pro
                   85                  90                  95
   Gly Ala Val Leu Lys Asp Phe Thr Val Ser Gly Asn Leu Leu Phe Ile
               100                 105                 110
   Arg Leu Thr Ala Ala Asp His Arg Gln Leu Gln Leu Ser Ile Ser Ser
           115                 120                 125
   Cys Leu Gln Gln Leu Ser Leu Leu Met Trp Ile Thr Gln Cys Phe Leu
       130                 135                 140
   Pro Val Phe Leu Ala Gln Ala Pro Ser Gly Gln Arg Arg Gly Gly Ala
   145                 150                 155                 160
   Arg Gly Pro Glu Ser Arg Leu Leu Glu Phe Tyr Leu Ala Met Pro Phe
                   165                 170                 175
   Ala Thr Pro Met Glu Ala Glu Leu Ala Arg Arg Ser Leu Ala Gln Asp
               180                 185                 190
   Ala Pro Pro Leu Pro Val Pro Gly Val Leu Leu Lys Glu Phe Thr Val
           195                 200                 205
   Ser Gly Asn Ile Leu Thr Ile Arg Leu Thr Ala Ala Asp His Arg Gln
       210                 215                 220
   Leu Gln Leu Ser Ile Ser Ser Cys Leu Gln Gln Leu Ser Leu Leu Met
   225                 230                 235                 240
   Trp Ile Thr Gln Cys Phe Leu Pro Val Phe Leu Ala Gln Pro Pro Ser
                   245                 250                 255
   Gly Gln Arg Arg
               260
<210> 43
   <211> 732
   <212> DNA
   <213> Artificial Sequence
<220>
   <223> LAGE/NY-ESO-1 construct
<400> 43
   atgcatcatc atcatcatca cgcaggtgca gcgcgtgcat ctggtccagg tggcggtgcg 60
   ccgcgtggcc cgcatggtgg tgcagctagt gcgcaagatg gtcgttgccc gtgtggtgcg 120
   cgtcgtccgg atagccgtct gctggagctg catattacca tgccgtttag cagcccaatg 180
   gaagctgagc tggtgcgtcg tattctgtct cgtgacgcag caccgctgcc acgtccgggt 240
   gcggttctga aagattttac cgtgagcggc aacctgctgt ttattcgtct gaccgcggca 300
   gatcatcgtc agctgcaact gagcattagc agctgcctgc aacagctgtc tctgctgatg 360
   tggattaccc agtgctttct gccggtgttt ctggctcagg cgccgtctgg tcagcgtcgt 420
   ggtggtgccc gtggcccgga atctcgtctg ctggaatttt atctggccat gccgttcgcg 480
   acgccgatgg aagcagagct ggcccgtcgc agcctggctc aggatgcacc gccgctgccg 540
   gttccgggcg tgctgctgaa agaatttacg gttagcggta acattctgac catccgtctg 600
   accgcagcgg accaccgcca actgcaactg tctatcagct cttgcctgca acaactgtcg 660
   ttattaatgt ggatcactca atgtttttta ccagtattcc tggcccaacc gccgagcggc 720
   caacgtcgtt aa                                                     732
<210> 44
   <211> 243
   <212> PRT
   <213> Artificial Sequence
<220>
   <223> LAGE/NY-ESO-1 construct
<400> 44
   Met His His His His His His Ala Gly Ala Ala Arg Ala Ser Gly Pro
    1               5                  10                  15
   Gly Gly Gly Ala Pro Arg Gly Pro His Gly Gly Ala Ala Ser Ala Gln
               20                  25                  30
   Asp Gly Arg Cys Pro Cys Gly Ala Arg Arg Pro Asp Ser Arg Leu Leu
           35                  40                  45
   Glu Leu His Ile Thr Met Pro Phe Ser Ser Pro Met Glu Ala Glu Leu
       50                  55                  60
   Val Arg Arg Ile Leu Ser Arg Asp Ala Ala Pro Leu Pro Arg Pro Gly
   65                  70                  75                  80
   Ala Val Leu Lys Asp Phe Thr Val Ser Gly Asn Leu Leu Phe Ile Arg
                   85                  90                  95
   Leu Thr Ala Ala Asp His Arg Gln Leu Gln Leu Ser Ile Ser Ser Cys
               100                 105                 110
   Leu Gln Gln Leu Ser Leu Leu Met Trp Ile Thr Gln Cys Phe Leu Pro
           115                 120                 125
   Val Phe Leu Ala Gln Ala Pro Ser Gly Gln Arg Arg Gly Gly Ala Arg
       130                 135                 140
   Gly Pro Glu Ser Arg Leu Leu Glu Phe Tyr Leu Ala Met Pro Phe Ala
   145                 150                 155                 160
   Thr Pro Met Glu Ala Glu Leu Ala Arg Arg Ser Leu Ala Gln Asp Ala
                   165                 170                 175
   Pro Pro Leu Pro Val Pro Gly Val Leu Leu Lys Glu Phe Thr Val Ser
               180                 185                 190
   Gly Asn Ile Leu Thr Ile Arg Leu Thr Ala Ala Asp His Arg Gln Leu
           195                 200                 205
   Gln Leu Ser Ile Ser Ser Cys Leu Gln Gln Leu Ser Leu Leu Met Trp
       210                 215                 220
   Ile Thr Gln Cys Phe Leu Pro Val Phe Leu Ala Gln Pro Pro Ser Gly
   225                 230                 235                 240
   Gln Arg Arg
<210> 45
   <211> 921
   <212> DNA
   <213> Artificial Sequence
<220>
   <223> LAGE/NY-ESO-1 construct
<400> 45
   atgggccatc atcatcatca tcatcatcat catcacagca gcggccatat cgacgacgac 60
   gacaagcata tgcaggcgga aggccgtggt actggcggta gcaccggcga tgcagatggt 120
   ccgggcggtc cgggtattcc ggatggtccg ggtggtaatg caggtggtcc aggtgaagca 180
   ggtgcgactg gcggtcgtgg tccacgcggt gcaggtgcag cgcgtgcatc tggtccaggt 240
   ggcggtgcgc cgcgtggccc gcatggtggt gcagctagtg cgcaagatgg tcgttgcccg 300
   tgtggtgcgc gtcgtccgga tagccgtctg ctggagctgc atattaccat gccgtttagc 360
   agcccaatgg aagctgagct ggtgcgtcgt attctgtctc gtgacgcagc accgctgcca 420
   cgtccgggtg cggttctgaa agattttacc gtgagcggca acctgctgtt tattcgtctg 480
   accgcggcag atcatcgtca gctgcaactg agcattagca gctgcctgca acagctgtct 540
   ctgctgatgt ggattaccca gtgctttctg ccggtgtttc tggctcaggc gccgtctggt 600
   cagcgtcgtg gtggtgcccg tggcccggaa tctcgtctgc tggaatttta tctggccatg 660
   ccgttcgcga cgccgatgga agcagagctg gcccgtcgca gcctggctca ggatgcaccg 720
   ccgctgccgg ttccgggcgt gctgctgaaa gaatttacgg ttagcggtaa cattctgacc 780
   atccgtctga ccgcagcgga ccaccgccaa ctgcaactgt ctatcagctc ttgcctgcaa 840
   caactgtcgt tattaatgtg gatcactcaa tgttttttac cagtattcct ggcccaaccg 900
   ccgagcggcc aacgtcgtta a                                           921
<210> 46
   <211> 306
   <212> PRT
   <213> Artificial Sequence
<220>
   <223> LAGE/NY-ESO-1 construct
<400> 46
<210> 47
   <211> 870
   <212> DNA
   <213> Artificial Sequence
<220>
   <223> LAGE/NY-ESO-1 construct
<400> 47
   atgcatcatc atcatcatca ccaggcggaa ggccgtggta ctggcggtag caccggcgat 60
   gcagatggtc cgggcggtcc gggtattccg gatggtccgg gtggtaatgc aggtggtcca 120
   ggtgaagcag gtgcgactgg cggtcgtggt ccacgcggtg caggtgcagc gcgtgcatct 180
   ggtccaggtg gcggtgcgcc gcgtggcccg catggtggtg cagctagtgc gcaagatggt 240
   cgttgcccgt gtggtgcgcg tcgtccggat agccgtctgc tggagctgca tattaccatg 300
   ccgtttagca gcccaatgga agctgagctg gtgcgtcgta ttctgtctcg tgacgcagca 360
   ccgctgccac gtccgggtgc ggttctgaaa gattttaccg tgagcggcaa cctgctgttt 420
   attcgtctga ccgcggcaga tcatcgtcag ctgcaactga gcattagcag ctgcctgcaa 480
   cagctgtctc tgctgatgtg gattacccag tgctttctgc cggtgtttct ggctcaggcg 540
   ccgtctggtc agcgtcgtgg tggtgcccgt ggcccggaat ctcgtctgct ggaattttat 600
   ctggccatgc cgttcgcgac gccgatggaa gcagagctgg cccgtcgcag cctggctcag 660
   gatgcaccgc cgctgccggt tccgggcgtg ctgctgaaag aatttacggt tagcggtaac 720
   attctgacca tccgtctgac cgcagcggac caccgccaac tgcaactgtc tatcagctct 780
   tgcctgcaac aactgtcgtt attaatgtgg atcactcaat gttttttacc agtattcctg 840
   gcccaaccgc cgagcggcca acgtcgttaa                                  870
<210> 48
   <211> 289
   <212> PRT
   <213> Artificial Sequence
<220>
   <223> LAGE/NY-ESO-1 construct
<400> 48
   Met His His His His His His Gln Ala Glu Gly Arg Gly Thr Gly Gly
    1               5                  10                  15
   Ser Thr Gly Asp Ala Asp Gly Pro Gly Gly Pro Gly Ile Pro Asp Gly
               20                  25                  30
   Pro Gly Gly Asn Ala Gly Gly Pro Gly Glu Ala Gly Ala Thr Gly Gly
           35                  40                  45
   Arg Gly Pro Arg Gly Ala Gly Ala Ala Arg Ala Ser Gly Pro Gly Gly
       50                  55                  60
   Gly Ala Pro Arg Gly Pro His Gly Gly Ala Ala Ser Ala Gln Asp Gly
   65                  70                  75                  80
   Arg Cys Pro Cys Gly Ala Arg Arg Pro Asp Ser Arg Leu Leu Glu Leu
                   85                  90                  95
   His Ile Thr Met Pro Phe Ser Ser Pro Met Glu Ala Glu Leu Val Arg
               100                 105                 110
   Arg Ile Leu Ser Arg Asp Ala Ala Pro Leu Pro Arg Pro Gly Ala Val
           115                 120                 125
   Leu Lys Asp Phe Thr Val Ser Gly Asn Leu Leu Phe Ile Arg Leu Thr
       130                 135                 140
   Ala Ala Asp His Arg Gln Leu Gln Leu Ser Ile Ser Ser Cys Leu Gln
   145                 150                 155                 160
   Gln Leu Ser Leu Leu Met Trp Ile Thr Gln Cys Phe Leu Pro Val Phe
                   165                 170                 175
   Leu Ala Gln Ala Pro Ser Gly Gln Arg Arg Gly Gly Ala Arg Gly Pro
               180                 185                 190
   Glu Ser Arg Leu Leu Glu Phe Tyr Leu Ala Met Pro Phe Ala Thr Pro
           195                 200                 205
   Met Glu Ala Glu Leu Ala Arg Arg Ser Leu Ala Gln Asp Ala Pro Pro
       210                 215                 220
   Leu Pro Val Pro Gly Val Leu Leu Lys Glu Phe Thr Val Ser Gly Asn
   225                 230                 235                 240
   Ile Leu Thr Ile Arg Leu Thr Ala Ala Asp His Arg Gln Leu Gln Leu
                   245                 250                 255
   Ser Ile Ser Ser Cys Leu Gln Gln Leu Ser Leu Leu Met Trp Ile Thr
               260                 265                 270
   Gln Cys Phe Leu Pro Val Phe Leu Ala Gln Pro Pro Ser Gly Gln Arg
           275                 280                 285
   Arg
<210> 49
   <211> 180
   <212> PRT
   <213> Homo Sapien
<400> 49
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Arg
               180
<210> 50
   <211> 420
   <212> DNA
   <213> Homo Sapien
<400> 50
   atggcaggcg cagcacgtgc atctggtccg ggtggtggtg caccgcgcgg tccgcatggt 60
   ggtgcggcga gcggcctgaa tggttgctgc cgttgcggtg cgcgtggtcc ggaaagccgt 120
   ctgctggaat tttatctggc catgccgttt gcgaccccga tggaagcgga actggcccgt 180
   cgtagcctgg ctcaagatgc accgccgctg ccggttccgg gcgtgctgct gaaagaattt 240
   accgtgagcg gcaacattct gaccattcgt ctgacggcgg cagaccatcg tcagctgcaa 300
   ctgagcatta gcagctgcct gcaacagctg tctctgctga tgtggattac ccagtgcttt 360
   ctgccggtgt ttctggccca gccgccgtct ggtcaacgtc accaccacca ccaccactaa 420
<210> 51
   <211> 139
   <212> PRT
   <213> Homo Sapien
<400> 51
   Met Ala Gly Ala Ala Arg Ala Ser Gly Pro Gly Gly Gly Ala Pro Arg
    1               5                  10                  15
   Gly Pro His Gly Gly Ala Ala Ser Gly Leu Asn Gly Cys Cys Arg Cys
               20                  25                  30
   Gly Ala Arg Gly Pro Glu Ser Arg Leu Leu Glu Phe Tyr Leu Ala Met
           35                  40                  45
   Pro Phe Ala Thr Pro Met Glu Ala Glu Leu Ala Arg Arg Ser Leu Ala
       50                  55                  60
   Gln Asp Ala Pro Pro Leu Pro Val Pro Gly Val Leu Leu Lys Glu Phe
   65                  70                  75                  80
   Thr Val Ser Gly Asn Ile Leu Thr Ile Arg Leu Thr Ala Ala Asp His
                   85                  90                  95
   Arg Gln Leu Gln Leu Ser Ile Ser Ser Cys Leu Gln Gln Leu Ser Leu
               100                 105                 110
   Leu Met Trp Ile Thr Gln Cys Phe Leu Pro Val Phe Leu Ala Gln Pro
           115                 120                 125
   Pro Ser Gly Gln Arg His His His His His His
       130                 135
<210> 52
   <211> 750
   <212> DNA
   <213> Homo Sapien
<400> 52
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcaggcg cagcacgtgc atctggtccg 360
   ggtggtggtg caccgcgcgg tccgcatggt ggtgcggcga gcggcctgaa tggttgctgc 420
   cgttgcggtg cgcgtggtcc ggaaagccgt ctgctggaat tttatctggc catgccgttt 480
   gcgaccccga tggaagcgga actggcccgt cgtagcctgg ctcaagatgc accgccgctg 540
   ccggttccgg gcgtgctgct gaaagaattt accgtgagcg gcaacattct gaccattcgt 600
   ctgacggcgg cagaccatcg tcagctgcaa ctgagcatta gcagctgcct gcaacagctg 660
   tctctgctga tgtggattac ccagtgcttt ctgccggtgt ttctggccca gccgccgtct 720
   ggtcaacgtc accaccacca ccaccactaa                                  750
<210> 53
   <211> 249
   <212> PRT
   <213> Homo Sapien
<400> 53
   Met Asp Pro Ser Ser His Ser Ser Asn Met Ala Asn Thr Gln Met Lys
   1               5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Gly Ala Ala Arg Ala Ser Gly Pro Gly Gly Gly Ala Pro Arg Gly Pro
           115                 120                 125
   His Gly Gly Ala Ala Ser Gly Leu Asn Gly Cys Cys Arg Cys Gly Ala
       130                 135                 140
   Arg Gly Pro Glu Ser Arg Leu Leu Glu Phe Tyr Leu Ala Met Pro Phe
   145                 150                 155                 160
   Ala Thr Pro Met Glu Ala Glu Leu Ala Arg Arg Ser Leu Ala Gln Asp
                   165                 170                 175
   Ala Pro Pro Leu Pro Val Pro Gly Val Leu Leu Lys Glu Phe Thr Val
               180                 185                 190
   Ser Gly Asn Ile Leu Thr Ile Arg Leu Thr Ala Ala Asp His Arg Gln
           195                 200                 205
   Leu Gln Leu Ser Ile Ser Ser Cys Leu Gln Gln Leu Ser Leu Leu Met
       210                 215                 220
   Trp Ile Thr Gln Cys Phe Leu Pro Val Phe Leu Ala Gln Pro Pro Ser
   225                 230                 235                 240
   Gly Gln Arg His His His His His His
                   245
<210> 54
   <211> 327
   <212> DNA
   <213> Homo Sapien
<400> 54
   atgggtgcgc gtggtccgga aagccgtctg ctggaatttt atctggccat gccgtttgcg 60
   accccgatgg aagcggaact ggcccgtcgt agcctggctc aagatgcacc gccgctgccg 120
   gttccgggcg tgctgctgaa agaatttacc gtgagcggca acattctgac cattcgtctg 180
   acggcggcag accatcgtca gctgcaactg agcattagca gctgcctgca acagctgtct 240
   ctgctgatgt ggattaccca gtgctttctg ccggtgtttc tggcccagcc gccgtctggt 300
   caacgtcacc accaccacca ccactaa                                     327
<210> 55
   <211> 108
   <212> PRT
   <213> Homo Sapien
<400> 55
   Met Gly Ala Arg Gly Pro Glu Ser Arg Leu Leu Glu Phe Tyr Leu Ala
    1               5                  10                  15
   Met Pro Phe Ala Thr Pro Met Glu Ala Glu Leu Ala Arg Arg Ser Leu
               20                  25                  30
   Ala Gln Asp Ala Pro Pro Leu Pro Val Pro Gly Val Leu Leu Lys Glu
           35                  40                  45
   Phe Thr Val Ser Gly Asn Ile Leu Thr Ile Arg Leu Thr Ala Ala Asp
       50                  55                  60
   His Arg Gln Leu Gln Leu Ser Ile Ser Ser Cys Leu Gln Gln Leu Ser
   65                  70                  75                  80
   Leu Leu Met Trp Ile Thr Gln Cys Phe Leu Pro Val Phe Leu Ala Gln
                   85                  90                  95
   Pro Pro Ser Gly Gln Arg His His His His His His
               100                 105
<210> 56
   <211> 657
   <212> DNA
   <213> Homo Sapien
<400> 56
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accggtgcgc gtggtccgga aagccgtctg 360
   ctggaatttt atctggccat gccgtttgcg accccgatgg aagcggaact ggcccgtcgt 420
   agcctggctc aagatgcacc gccgctgccg gttccgggcg tgctgctgaa agaatttacc 480
   gtgagcggca acattctgac cattcgtctg acggcggcag accatcgtca gctgcaactg 540
   agcattagca gctgcctgca acagctgtct ctgctgatgt ggattaccca gtgctttctg 600
   ccggtgtttc tggcccagcc gccgtctggt caacgtcacc accaccacca ccactaa    657
<210> 57
   <211> 218
   <212> PRT
   <213> Homo Sapien
<400> 57
   Met Asp Pro Ser Ser His Ser Ser Asn Met Ala Asn Thr Gln Met Lys
    1              5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gly
               100                 105                 110
   Ala Arg Gly Pro Glu Ser Arg Leu Leu Glu Phe Tyr Leu Ala Met Pro
           115                 120                 125
   Phe Ala Thr Pro Met Glu Ala Glu Leu Ala Arg Arg Ser Leu Ala Gln
       130                 135                 140
   Asp Ala Pro Pro Leu Pro Val Pro Gly Val Leu Leu Lys Glu Phe Thr
   145                 150                 155                 160
   Val Ser Gly Asn Ile Leu Thr Ile Arg Leu Thr Ala Ala Asp His Arg
                   165                 170                 175
   Gln Leu Gln Leu Ser Ile Ser Ser Cys Leu Gln Gln Leu Ser Leu Leu
               180                 185                 190
   Met Trp Ile Thr Gln Cys Phe Leu Pro Val Phe Leu Ala Gln Pro Pro
           195                 200                 205
   Ser Gly Gln Arg His His His His His His
       210                 215
<210> 58
   <211> 180
   <212> PRT
   <213> Homo Sapien
<220>
   <221> PEPTIDE
   <222> (0)...(0)
   <223> Lage 1a
<400> 58
   Met Gln Ala Glu Gly Gln Gly Thr Gly Gly Ser Thr Gly Asp Ala Asp
    1              5                   10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Arg Gly Gly Ala Pro Arg Gly Pro
       50                  55                  60
   His Gly Gly Ala Ala Ser Ala Gln Asp Gly Arg Cys Pro Cys Gly Ala
   65                  70                  75                  80
   Arg Arg Pro Asp Ser Arg Leu Leu Gln Leu His Ile Thr Met Pro Phe
                   85                  90                  95
   Ser Ser Pro Met Glu Ala Glu Leu Val Arg Arg Ile Leu Ser Arg Asp
                   100                 105                 110
   Ala Ala Pro Leu Pro Arg Pro Gly Ala Val Leu Lys Asp Phe Thr Val
           115                 120                 125
   Ser Gly Asn Leu Leu Phe Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Ala Pro Ser
                   165                 170                 175
   Gly Gln Arg Arg
               180
<210> 59
   <211> 561
   <212> DNA
   <213> Homo Sapien
<400> 59
   atgcaggcgg aaggccgtgg tactggcggt agcaccggcg atgcagatgg tccgggcggt 60
   ccgggtattc cggatggtcc gggtggtaat gcaggtggtc caggtgaagc aggtgcgact 120
   ggcggtcgtg gtccacgcgg tgcaggtgca gcgcgtgcat ctggtccagg tggcggtgcg 180
   ccgcgtggcc cgcatggtgg tgcagctagt gcgcaagatg gtcgttgccc gtgtggtgcg 240
   cgtcgtccgg atagccgtct gctggagctg catattacca tgccgtttag cagcccaatg 300
   gaagctgagc tggtgcgtcg tattctgtct cgtgacgcag caccgctgcc acgtccgggt 360
   gcggttctga aagattttac cgtgagcggc aacctgctgt ttattcgtct gaccgcggca 420
   gatcatcgtc agctgcaact gagcattagc agctgcctgc aacagctgtc tctgctgatg 480
   tggattaccc agtgctttct gccggtgttt ctggctcagg cgccgtctgg tcagcgtcgt 540
   caccaccacc accaccacta a                                          561
<210> 60
   <211> 186
   <212> PRT
   <213> Homo Sapien
<400> 60
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Ala Gln Asp Gly Arg Cys Pro Cys Gly Ala
   65                  70                  75                  80
   Arg Arg Pro Asp Ser Arg Leu Leu Glu Leu His Ile Thr Met Pro Phe
                   85                  90                  95
   Ser Ser Pro Met Glu Ala Glu Leu Val Arg Arg Ile Leu Ser Arg Asp
               100                 105                 110
   Ala Ala Pro Leu Pro Arg Pro Gly Ala Val Leu Lys Asp Phe Thr Val
           115                 120                 125
   Ser Gly Asn Leu Leu Phe Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Ala Pro Ser
                   165                 170                 175
   Gly Gln Arg Arg His His His His His His
               180                 185
<210> 61
   <211> 891
   <212> DNA
   <213> Homo Sapien
<400> 61
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa acccaggcgg aaggccgtgg tactggcggt 360
   agcaccggcg atgcagatgg tccgggcggt ccgggtattc cggatggtcc gggtggtaat 420
   gcaggtggtc caggtgaagc aggtgcgact ggcggtcgtg gtccacgcgg tgcaggtgca 480
   gcgcgtgcat ctggtccagg tggcggtgcg ccgcgtggcc cgcatggtgg tgcagctagt 540
   gcgcaagatg gtcgttgccc gtgtggtgcg cgtcgtccgg atagccgtct gctggagctg 600
   catattacca tgccgtttag cagcccaatg gaagctgagc tggtgcgtcg tattctgtct 660
   cgtgacgcag caccgctgcc acgtccgggt gcggttctga aagattttac cgtgagcggc 720
   aacctgctgt ttattcgtct gaccgcggca gatcatcgtc agctgcaact gagcattagc 780
   agctgcctgc aacagctgtc tctgctgatg tggattaccc agtgctttct gccggtgttt 840
   ctggctcagg cgccgtctgg tcagcgtcgt caccaccacc accaccacta a          891
<210> 62
   <211> 296
   <212> PRT
   <213> Homo Sapien
<400> 62
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gln
               100                 105                 110
   Ala Glu Gly Arg Gly Thr Gly Gly Ser Thr Gly Asp Ala Asp Gly Pro
           115                 120                 125
   Gly Gly Pro Gly Ile Pro Asp Gly Pro Gly Gly Asn Ala Gly Gly Pro
       130                 135                 140
   Gly Glu Ala Gly Ala Thr Gly Gly Arg Gly Pro Arg Gly Ala Gly Ala
   145                 150                 155                 160
   Ala Arg Ala Ser Gly Pro Gly Gly Gly Ala Pro Arg Gly Pro His Gly
                   165                 170                 175
   Gly Ala Ala Ser Ala Gln Asp Gly Arg Cys Pro Cys Gly Ala Arg Arg
                   180                 185                 190
   Pro Asp Ser Arg Leu Leu Glu Leu His Ile Thr Met Pro Phe Ser Ser
               195                 200                 205
   Pro Met Glu Ala Glu Leu Val Arg Arg Ile Leu Ser Arg Asp Ala Ala
       210                 215                 220
   Pro Leu Pro Arg Pro Gly Ala Val Leu Lys Asp Phe Thr Val Ser Gly
   225                 230                 235                 240
   Asn Leu Leu Phe Ile Arg Leu Thr Ala Ala Asp His Arg Gln Leu Gln
                   245                 250                 255
   Leu Ser Ile Ser Ser Cys Leu Gln Gln Leu Ser Leu Leu Met Trp Ile
               260                 265                 270
   Thr Gln Cys Phe Leu Pro Val Phe Leu Ala Gln Ala Pro Ser Gly Gln
           275                 280                 285
   Arg Arg His His His His His His
       290                 295
<210> 63
   <211> 423
   <212> DNA
   <213> Homo Sapien
<400> 63
   atggcaggtg cagcgcgtgc atctggtcca ggtggcggtg cgccgcgtgg cccgcatggt 60
   ggtgcagcta gtgcgcaaga tggtcgttgc ccgtgtggtg cgcgtcgtcc ggatagccgt 120
   ctgctggagc tgcatattac catgccgttt agcagcccaa tggaagctga gctggtgcgt 180
   cgtattctgt ctcgtgacgc agcaccgctg ccacgtccgg gtgcggttct gaaagatttt 240
   accgtgagcg gcaacctgct gtttattcgt ctgaccgcgg cagatcatcg tcagctgcaa 300
   ctgagcatta gcagctgcct gcaacagctg tctctgctga tgtggattac ccagtgcttt 360
   ctgccggtgt ttctggctca ggcgccgtct ggtcagcgtc gtcaccacca ccaccaccac 420
   taa                                                               423
<210> 64
   <211> 140
   <212> PRT
   <213> Homo Sapien
<400> 64
   Met Ala Gly Ala Ala Arg Ala Ser Gly Pro Gly Gly Gly Ala Pro Arg
    1               5                  10                  15
   Gly Pro His Gly Gly Ala Ala Ser Ala Gln Asp Gly Arg Cys Pro Cys
               20                  25                  30
   Gly Ala Arg Arg Pro Asp Ser Arg Leu Leu Glu Leu His Ile Thr Met
           35                  40                  45
   Pro Phe Ser Ser Pro Met Glu Ala Glu Leu Val Arg Arg Ile Leu Ser
       50                  55                  60
   Arg Asp Ala Ala Pro Leu Pro Arg Pro Gly Ala Val Leu Lys Asp Phe
   65                  70                  75                  80
   Thr Val Ser Gly Asn Leu Leu Phe Ile Arg Leu Thr Ala Ala Asp His
                   85                  90                  95
   Arg Gln Leu Gln Leu Ser Ile Ser Ser Cys Leu Gln Gln Leu Ser Leu
               100                 105                 110
   Leu Met Trp Ile Thr Gln Cys Phe Leu Pro Val Phe Leu Ala Gln Ala
           115                 120                 125
   Pro Ser Gly Gln Arg Arg His His His His His His
       130                 135                 140
<210> 65
   <211> 753
   <212> DNA
   <213> Homo Sapien
<400> 65
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcaggtg cagcgcgtgc atctggtcca 360
   ggtggcggtg cgccgcgtgg cccgcatggt ggtgcagcta gtgcgcaaga tggtcgttgc 420
   ccgtgtggtg cgcgtcgtcc ggatagccgt ctgctggagc tgcatattac catgccgttt 480
   agcagcccaa tggaagctga gctggtgcgt cgtattctgt ctcgtgacgc agcaccgctg 540
   ccacgtccgg gtgcggttct gaaagatttt accgtgagcg gcaacctgct gtttattcgt 600
   ctgaccgcgg cagatcatcg tcagctgcaa ctgagcatta gcagctgcct gcaacagctg 660
   tctctgctga tgtggattac ccagtgcttt ctgccggtgt ttctggctca ggcgccgtct 720
   ggtcagcgtc gtcaccacca ccaccaccac taa                              753
<210> 66
   <211> 250
   <212> PRT
   <213> Homo Sapien
<400> 66
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Gly Ala Ala Arg Ala Ser Gly Pro Gly Gly Gly Ala Pro Arg Gly Pro
           115                 120                 125
   His Gly Gly Ala Ala Ser Ala Gln Asp Gly Arg Cys Pro Cys Gly Ala
       130                 135                 140
   Arg Arg Pro Asp Ser Arg Leu Leu Glu Leu His Ile Thr Met Pro Phe
   145                 150                 155                 160
   Ser Ser Pro Met Glu Ala Glu Leu Val Arg Arg Ile Leu Ser Arg Asp
                   165                 170                 175
   Ala Ala Pro Leu Pro Arg Pro Gly Ala Val Leu Lys Asp Phe Thr Val
               180                 185                 190
   Ser Gly Asn Leu Leu Phe Ile Arg Leu Thr Ala Ala Asp His Arg Gln
           195                 200                 205
   Leu Gln Leu Ser Ile Ser Ser Cys Leu Gln Gln Leu Ser Leu Leu Met
       210                 215                 220
   Trp Ile Thr Gln Cys Phe Leu Pro Val Phe Leu Ala Gln Ala Pro Ser
   225                 230                 235                 240
   Gly Gln Arg Arg His His His His His His
                   245                 250
<210> 67
   <211> 330
   <212> DNA
   <213> Homo Sapien
<400> 67
   atgggtgcgc gtcgtccgga tagccgtctg ctggagctgc atattaccat gccgtttagc 60
   agcccaatgg aagctgagct ggtgcgtcgt attctgtctc gtgacgcagc accgctgcca 120
   cgtccgggtg cggttctgaa agattttacc gtgagcggca acctgctgtt tattcgtctg 180
   accgcggcag atcatcgtca gctgcaactg agcattagca gctgcctgca acagctgtct 240
   ctgctgatgt ggattaccca gtgctttctg ccggtgtttc tggctcaggc gccgtctggt 300
   cagcgtcgtc accaccacca ccaccactaa                                  330
<210> 68
   <211> 109
   <212> PRT
   <213> Homo Sapien
<400> 68
   Met Gly Ala Arg Arg Pro Asp Ser Arg Leu Leu Glu Leu His Ile Thr
    1               5                  10                  15
   Met Pro Phe Ser Ser Pro Met Glu Ala Glu Leu Val Arg Arg Ile Leu
               20                  25                  30
   Ser Arg Asp Ala Ala Pro Leu Pro Arg Pro Gly Ala Val Leu Lys Asp
           35                  40                  45
   Phe Thr Val Ser Gly Asn Leu Leu Phe Ile Arg Leu Thr Ala Ala Asp
       50                  55                  60
   His Arg Gln Leu Gln Leu Ser Ile Ser Ser Cys Leu Gln Gln Leu Ser
   65                  70                  75                  80
   Leu Leu Met Trp Ile Thr Gln Cys Phe Leu Pro Val Phe Leu Ala Gln
                   85                  90                  95
   Ala Pro Ser Gly Gln Arg Arg His His His His His His
               100                 105
<210> 69
   <211> 660
   <212> DNA
   <213> Homo Sapien
<400> 69
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accggtgcgc gtcgtccgga tagccgtctg 360
   ctggagctgc atattaccat gccgtttagc agcccaatgg aagctgagct ggtgcgtcgt 420
   attctgtctc gtgacgcagc accgctgcca cgtccgggtg cggttctgaa agattttacc 480
   gtgagcggca acctgctgtt tattcgtctg accgcggcag atcatcgtca gctgcaactg 540
   agcattagca gctgcctgca acagctgtct ctgctgatgt ggattaccca gtgctttctg 600
   ccggtgtttc tggctcaggc gccgtctggt cagcgtcgtc accaccacca ccaccactaa 660
<210> 70
   <211> 219
   <212> PRT
   <213> homo sapien
<400> 70
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gly
               100                 105                 110
   Ala Arg Arg Pro Asp Ser Arg Leu Leu Glu Leu His Ile Thr Met Pro
           115                 120                 125
   Phe Ser Ser Pro Met Glu Ala Glu Leu Val Arg Arg Ile Leu Ser Arg
       130                 135                 140
   Asp Ala Ala Pro Leu Pro Arg Pro Gly Ala Val Leu Lys Asp Phe Thr
   145                 150                 155                 160
   Val Ser Gly Asn Leu Leu Phe Ile Arg Leu Thr Ala Ala Asp His Arg
                   165                 170                 175
   Gln Leu Gln Leu Ser Ile Ser Ser Cys Leu Gln Gln Leu Ser Leu Leu
               180                 185                 190
   Met Trp Ile Thr Gln Cys Phe Leu Pro Val Phe Leu Ala Gln Ala Pro
           195                 200                 205
   Ser Gly Gln Arg Arg His His His His His His
   210 215
<210> 71
   <211> 210
   <212> PRT
   <213> Homo Sapien
<220>
   <221> PEPTIDE
   <222> (0) ... (0)
   <223> Lage 1b
<400> 71
   Met Gln Ala Glu Gly Gln Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Arg Gly Gly Ala Pro Arg Gly Pro
           50                  55                  60
   His Gly Gly Ala Ala Ser Ala Gln Asp Gly Arg Cys Pro Cys Gly Ala
   65                  70                  75                  80
   Arg Arg Pro Asp Ser Arg Leu Leu Gln Leu His Ile Thr Met Pro Phe
                   85                  90                  95
   Ser Ser Pro Met Glu Ala Glu Leu Val Arg Arg Ile Leu Ser Arg Asp
               100                 105                 110
   Ala Ala Pro Leu Pro Arg Pro Gly Ala Val Leu Lys Asp Phe Thr Val
           115                 120                 125
   Ser Gly Asn Leu Leu Phe Met Ser Val Arg Asp Gln Asp Arg Glu Gly
       130                 135                 140
   Ala Gly Arg Met Arg Val Val Gly Trp Gly Leu Gly Ser Ala Ser Pro
   145                 150                 155                 160
   Glu Gly Gln Lys Ala Arg Asp Leu Arg Thr Pro Lys His Lys Val Ser
                   165                 170                 175
   Glu Gln Arg Pro Gly Thr Pro Gly Pro Pro Pro Pro Glu Gly Ala Gln
               180                 185                 190
   Gly Asp Gly Cys Arg Gly Val Ala Phe Asn Val Met Phe Ser Ala Pro
           195                 200                 205
   His Ile
       210
<210> 72
   <211> 978
   <212> DNA
   <213> Homo Sapien
<400> 72
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcggccg cacatatggg tgcgcgtggt 360
   ccggaaagcc gtctgctgga attttatctg gccatgccgt ttgcgacccc gatggaagcg 420
   gaactggccc gtcgtagcct ggctcaagat gcaccgccgc tgccggttcc gggcgtgctg 480
   ctgaaagaat ttaccgtgag cggcaacatt ctgaccattc gtctgacggc ggcagaccat 540
   cgtcagctgc aactgagcat tagcagctgc ctgcaacagc tgtctctgct gatgtggatt 600
   acccagtgct ttctgccggt gtttctggcc cagccgccgt ctggtcaacg tggtggcgcg 660
   cgtcgtccgg attctcgcct gctggaactg catattacca tgccgttcag ctctccaatg 720
   gaggccgaat tagtgcgtcg cattctgagc cgtgatgcgg caccgctgcc gcgtccaggt 780
   gcggttctga aagacttcac cgtatctggc aacctgctgt ttatccgtct gaccgcagcg 840
   gaccaccgcc aattacaatt atctatcagc tcttgtttac aacaactgtc gctgttaatg 900
   tggatcactc aatgtttcct gccagtattc ctggctcagg ccccgagcgg tcagcgtcgt 960
   caccaccacc accaccac                                                978
<210> 73
   <211> 326
   <212> PRT
   <213> Homo Sapien
<400> 73
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Ala Ala His Met Gly Ala Arg Gly Pro Glu Ser Arg Leu Leu Glu Phe
           115                 120                 125
   Tyr Leu Ala Met Pro Phe Ala Thr Pro Met Glu Ala Glu Leu Ala Arg
       130                 135                 140
   Arg Ser Leu Ala Gln Asp Ala Pro Pro Leu Pro Val Pro Gly Val Leu
   145                 150                 155                 160
   Leu Lys Glu Phe Thr Val Ser Gly Asn Ile Leu Thr Ile Arg Leu Thr
                   165                 170                 175
   Ala Ala Asp His Arg Gln Leu Gln Leu Ser Ile Ser Ser Cys Leu Gln
               180                 185                 190
   Gln Leu Ser Leu Leu Met Trp Ile Thr Gln Cys Phe Leu Pro Val Phe
           195                 200                 205
   Leu Ala Gln Pro Pro Ser Gly Gln Arg Gly Gly Ala Arg Arg Pro Asp
       210                 215                 220
   Ser Arg Leu Leu Glu Leu His Ile Thr Met Pro Phe Ser Ser Pro Met
   225                 230                 235                 240
   Glu Ala Glu Leu Val Arg Arg Ile Leu Ser Arg Asp Ala Ala Pro Leu
                   245                 250                 255
   Pro Arg Pro Gly Ala Val Leu Lys Asp Phe Thr Val Ser Gly Asn Leu
               260                 265                 270
   Leu Phe Ile Arg Leu Thr Ala Ala Asp His Arg Gln Leu Gln Leu Ser
           275                 280                 285
   Ile Ser Ser Cys Leu Gln Gln Leu Ser Leu Leu Met Trp Ile Thr Gln
       290                 295                 300
   Cys Phe Leu Pro Val Phe Leu Ala Gln Ala Pro Ser Gly Gln Arg Arg
   305                 310                 315                 320
   His His His His His His
                   325
<210> 74
   <211> 1209
   <212> DNA
   <213> Homo Sapien
<400> 74
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcggccg cacatatgca ggcggaaggc 360
   cgtggcaccg gtggtagcac cggcgatgcg gatggtccgg gcggtccggg tattccggac 420
   gggcctggtg gtaatgcggg tgggccaggt gaagcgggtg cgaccggtgg tcgtggtccg 480
   cggggggcag gcgcagcacg tgcatctggt ccgggtggtg gtgcaccgcg cggtccgcat 540
   ggtggtgcgg cgagcggcct gaatggttgc tgccgttgcg gtgcgcgtgg tccggaaagc 600
   cgtctgctgg aattttatct ggccatgccg tttgcgaccc cgatggaagc ggaactggcc 660
   cgtcgtagcc tggctcaaga tgcaccgccg ctgccggttc cgggcgtgct gctgaaagaa 720
   tttaccgtga gcggcaacat tctgaccatt cgtctgacgg cggcagacca tcgtcagctg 780
   caactgagca ttagcagctg cctgcaacag ctgtctctgc tgatgtggat tacccagtgc 840
   tttctgccgg tgtttctggc ccagccgccg tctggtcaac gtggtggcgc gcgtcgtccg 900
   gattctcgcc tgctggaact gcatattacc atgccgttca gctctccaat ggaggccgaa 960
   ttagtgcgtc gcattctgag ccgtgatgcg gcaccgctgc cgcgtccagg tgcggttctg 1020
   aaagacttca ccgtatctgg caacctgctg tttatccgtc tgaccgcagc ggaccaccgc 1080
   caattacaat tatctatcag ctcttgttta caacaactgt cgctgttaat gtggatcact 1140
   caatgtttcc tgccagtatt cctggctcag gccccgagcg gtcagcgtcg tcaccaccac 1200
   caccaccac                                                         1209
<210> 75
   <211> 403
   <212> PRT
   <213> Homo Sapien
<400> 75
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
               100                 105                 110
   Ala Ala His Met Gln Ala Glu Gly Arg Gly Thr Gly Gly Ser Thr Gly
           115                 120                 125
   Asp Ala Asp Gly Pro Gly Gly Pro Gly Ile Pro Asp Gly Pro Gly Gly
       130                 135                 140
   Asn Ala Gly Gly Pro Gly Glu Ala Gly Ala Thr Gly Gly Arg Gly Pro
   145                 150                 155                 160
   Arg Gly Ala Gly Ala Ala Arg Ala Ser Gly Pro Gly Gly Gly Ala Pro
                   165                 170                 175
   Arg Gly Pro His Gly Gly Ala Ala Ser Gly Leu Asn Gly Cys Cys Arg
               180                 185                 190
   Cys Gly Ala Arg Gly Pro Glu Ser Arg Leu Leu Glu Phe Tyr Leu Ala
           195                 200                 205
   Met Pro Phe Ala Thr Pro Met Glu Ala Glu Leu Ala Arg Arg Ser Leu
       210                 215                 220
   Ala Gln Asp Ala Pro Pro Leu Pro Val Pro Gly Val Leu Leu Lys Glu
   225                 230                 235                 240
   Phe Thr Val Ser Gly Asn Ile Leu Thr Ile Arg Leu Thr Ala Ala Asp
                   245                 250                 255
   His Arg Gln Leu Gln Leu Ser Ile Ser Ser Cys Leu Gln Gln Leu Ser
               260                 265                 270
   Leu Leu Met Trp Ile Thr Gln Cys Phe Leu Pro Val Phe Leu Ala Gln
           275                 280                 285
   Pro Pro Ser Gly Gln Arg Gly Gly Ala Arg Arg Pro Asp Ser Arg Leu
       290                 295                 300
   Leu Glu Leu His Ile Thr Met Pro Phe Ser Ser Pro Met Glu Ala Glu
   305                 310                 315                 320
   Leu Val Arg Arg Ile Leu Ser Arg Asp Ala Ala Pro Leu Pro Arg Pro
                   325                 330                 335
   Gly Ala Val Leu Lys Asp Phe Thr Val Ser Gly Asn Leu Leu Phe Ile
               340                 345                 350
   Arg Leu Thr Ala Ala Asp His Arg Gln Leu Gln Leu Ser Ile Ser Ser
           355                 360                 365
   Cys Leu Gln Gln Leu Ser Leu Leu Met Trp Ile Thr Gln Cys Phe Leu
       370                 375                 380
   Pro Val Phe Leu Ala Gln Ala Pro Ser Gly Gln Arg Arg His His His
   385                 390                 395                 400
   His His His
<210> 76
   <211> 981
   <212> DNA
   <213> Homo Sapien
<400> 76
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcggccg cacatatggg tgcgcgtcgt 360
   ccggatagcc gtctgctgga gctgcatatt accatgccgt ttagcagccc aatggaagct 420
   gagctggtgc gtcgtattct gtctcgtgac gcagcaccgc tgccacgtcc gggtgcggtt 480
   ctgaaagatt ttaccgtgag cggcaacctg ctgtttattc gtctgaccgc ggcagatcat 540
   cgtcagctgc aactgagcat tagcagctgc ctgcaacagc tgtctctgct gatgtggatt 600
   acccagtgct ttctgccggt gtttctggct caggcgccgt ctggtcagcg tcgtggtggt 660
   gcccgtggcc cggaatctcg tctgctggaa ttttatctgg ccatgccgtt cgcgacgccg 720
   atggaagcag agctggcccg tcgcagcctg gctcaggatg caccgccgct gccggttccg 780
   ggcgtgctgc tgaaagaatt tacggttagc ggtaacattc tgaccatccg tctgaccgca 840
   gcggaccacc gccaactgca actgtctatc agctcttgcc tgcaacaact gtcgttatta 900
   atgtggatca ctcaatgttt tttaccagta ttcctggccc aaccgccgag cggccaacgt 960
   cgtcaccacc accaccacca c                                           981
<210> 77
   <211> 327
   <212> PRT
   <213> Homo Sapien
<400> 77
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Ala
                       100                     105                     110
   Ala Ala His Met Gly Ala Arg Arg Pro Asp Ser Arg Leu Leu Glu Leu
               115                     120                     125
   His Ile Thr Met Pro Phe Ser Ser Pro Met Glu Ala Glu Leu Val Arg
       130                     135                     140
   Arg Ile Leu Ser Arg Asp Ala Ala Pro Leu Pro Arg Pro Gly Ala Val
   145                     150                     155                     160
   Leu Lys Asp Phe Thr Val Ser Gly Asn Leu Leu Phe Ile Arg Leu Thr
                   165                     170                     175
   Ala Ala Asp His Arg Gln Leu Gln Leu Ser Ile Ser Ser Cys Leu Gln
               180                     185                     190
   Gln Leu Ser Leu Leu Met Trp Ile Thr Gln Cys Phe Leu Pro Val Phe
           195                     200                     205
   Leu Ala Gln Ala Pro Ser Gly Gln Arg Arg Gly Gly Ala Arg Gly Pro
       210                     215                     220
   Glu Ser Arg Leu Leu Glu Phe Tyr Leu Ala Met Pro Phe Ala Thr Pro
   225                     230                     235                     240
   Met Glu Ala Glu Leu Ala Arg Arg Ser Leu Ala Gln Asp Ala Pro Pro
                   245                     250                     255
   Leu Pro Val Pro Gly Val Leu Leu Lys Glu Phe Thr Val Ser Gly Asn
               260                     265                     270
   Ile Leu Thr Ile Arg Leu Thr Ala Ala Asp His Arg Gln Leu Gln Leu
           275                     280                     285
   Ser Ile Ser Ser Cys Leu Gln Gln Leu Ser Leu Leu Met Trp Ile Thr
       290                     295                     300
   Gln Cys Phe Leu Pro Val Phe Leu Ala Gln Pro Pro Ser Gly Gln Arg
   305                     310                     315                     320
   Arg His His His His His His
                   325
<210> 78
   <211> 1212
   <212> DNA
   <213> Homo Sapien
<400> 78
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accgcggccg cacatatgca ggcggaaggc 360
   cgtggtactg gcggtagcac cggcgatgca gatggtccgg gcggtccggg tattccggat 420
   ggtccgggtg gtaatgcagg tggtccaggt gaagcaggtg cgactggcgg tcgtggtcca 480
   cgcggtgcag gtgcagcgcg tgcatctggt ccaggtggcg gtgcgccgcg tggcccgcat 540
   ggtggtgcag ctagtgcgca agatggtcgt tgcccgtgtg gtgcgcgtcg tccggatagc 600
   cgtctgctgg agctgcatat taccatgccg tttagcagcc caatggaagc tgagctggtg 660
   cgtcgtattc tgtctcgtga cgcagcaccg ctgccacgtc cgggtgcggt tctgaaagat 720
   tttaccgtga gcggcaacct gctgtttatt cgtctgaccg cggcagatca tcgtcagctg 780
   caactgagca ttagcagctg cctgcaacag ctgtctctgc tgatgtggat tacccagtgc 840
   tttctgccgg tgtttctggc tcaggcgccg tctggtcagc gtcgtggtgg tgcccgtggc 900
   ccggaatctc gtctgctgga attttatctg gccatgccgt tcgcgacgcc gatggaagca 960
   gagctggccc gtcgcagcct ggctcaggat gcaccgccgc tgccggttcc gggcgtgctg 1020
   ctgaaagaat ttacggttag cggtaacatt ctgaccatcc gtctgaccgc agcggaccac 1080
   cgccaactgc aactgtctat cagctcttgc ctgcaacaac tgtcgttatt aatgtggatc 1140
   actcaatgtt ttttaccagt attcctggcc caaccgccga gcggccaacg tcgtcaccac 1200
   caccaccacc ac 1212
<210> 79
   <211> 404
   <212> PRT
   <213> Homo Sapien
<400> 79
<210> 80
   <211> 918
   <212> DNA
   <213> Homo Sapien
<400> 80
   atgggccatc atcatcatca tcatcatcat catcacagca gcggccatat cgacgacgac 60
   gacaagcata tgcaggcgga aggccgtggt actggcggta gcaccggcga tgcagatggt 120
   ccgggcggtc cgggtattcc ggatggtccg ggtggtaatg caggtggtcc aggtgaagca 180
   ggtgcgactg gcggtcgtgg tccacgcggt gcaggtgcag cgcgtgcatc tggtccaggt 240
   ggcggtgcgc cgcgtggccc gcatggtggt gcagctagtg cgcaagatgg tcgttgcccg 300
   tgtggtgcgc gtcgtccgga tagccgtctg ctggagctgc atattaccat gccgtttagc 360
   agcccaatgg aagctgagct ggtgcgtcgt attctgtctc gtgacgcagc accgctgcca 420
   cgtccgggtg cggttctgaa agattttacc gtgagcggca acctgctgtt tattcgtctg 480
   accgcggcag atcatcgtca gctgcaactg agcattagca gctgcctgca acagctgtct 540
   ctgctgatgt ggattaccca gtgctttctg ccggtgtttc tggctcaggc gccgtctggt 600
   cagcgtcgtg gtggtgcccg tggcccggaa tctcgtctgc tggaatttta tctggccatg 660
   ccgttcgcga cgccgatgga agcagagctg gcccgtcgca gcctggctca ggatgcaccg 720
   ccgctgccgg ttccgggcgt gctgctgaaa gaatttacgg ttagcggtaa cattctgacc 780
   atccgtctga ccgcagcgga ccaccgccaa ctgcaactgt ctatcagctc ttgcctgcaa 840
   caactgtcgt tattaatgtg gatcactcaa tgttttttac cagtattcct ggcccaaccg 900
   ccgagcggcc aacgtcgt                                                918
<210> 81
   <211> 306
   <212> PRT
   <213> Homo Sapien
<400> 81
<210> 82
   <211> 684
   <212> DNA
   <213> Homo Sapien
<400> 82
   atgggccatc atcatcatca tcatcatcat catcacagca gcggccatat cgacgacgac 60
   gacaagcata tgggtgcgcg tggtccggaa agccgtctgc tggaatttta tctggccatg 120
   ccgtttgcga ccccgatgga agcggaactg gcccgtcgta gcctggctca agatgcaccg 180
   ccgctgccgg ttccgggcgt gctgctgaaa gaatttaccg tgagcggcaa cattctgacc 240
   attcgtctga cggcggcaga ccatcgtcag ctgcaactga gcattagcag ctgcctgcaa 300
   cagctgtctc tgctgatgtg gattacccag tgctttctgc cggtgtttct ggcccagccg 360
   ccgtctggtc aacgtggtgg cgcgcgtcgt ccggattctc gcctgctgga actgcatatt 420
   accatgccgt tcagctctcc aatggaggcc gaattagtgc gtcgcattct gagccgtgat 480
   gcggcaccgc tgccgcgtcc aggtgcggtt ctgaaagact tcaccgtatc tggcaacctg 540
   ctgtttatcc gtctgaccgc agcggaccac cgccaattac aattatctat cagctcttgt 600
   ttacaacaac tgtcgctgtt aatgtggatc actcaatgtt tcctgccagt attcctggct 660
   caggccccga gcggtcagcg tcgt                                        684
<210> 83
   <211> 228
   <212> PRT
   <213> Homo Sapien
<400> 83
   Met Gly His His His His His His His His His His Ser Ser Gly His
    1               5                  10                  15
   Ile Asp Asp Asp Asp Lys His Met Gly Ala Arg Gly Pro Glu Ser Arg
               20                  25                  30
   Leu Leu Glu Phe Tyr Leu Ala Met Pro Phe Ala Thr Pro Met Glu Ala
           35                  40                  45
   Glu Leu Ala Arg Arg Ser Leu Ala Gln Asp Ala Pro Pro Leu Pro Val
       50                  55                  60
   Pro Gly Val Leu Leu Lys Glu Phe Thr Val Ser Gly Asn Ile Leu Thr
   65                  70                  75                  80
   Ile Arg Leu Thr Ala Ala Asp His Arg Gln Leu Gln Leu Ser Ile Ser
                   85                  90                  95
   Ser Cys Leu Gln Gln Leu Ser Leu Leu Met Trp Ile Thr Gln Cys Phe
               100                 105                 110
   Leu Pro Val Phe Leu Ala Gln Pro Pro Ser Gly Gln Arg Gly Gly Ala
           115                 120                 125
   Arg Arg Pro Asp Ser Arg Leu Leu Glu Leu His Ile Thr Met Pro Phe
       130                 135                 140
   Ser Ser Pro Met Glu Ala Glu Leu Val Arg Arg Ile Leu Ser Arg Asp
   145                 150                 155                 160
   Ala Ala Pro Leu Pro Arg Pro Gly Ala Val Leu Lys Asp Phe Thr Val
                   165                 170                 175
   Ser Gly Asn Leu Leu Phe Ile Arg Leu Thr Ala Ala Asp His Arg Gln
               180                 185                 190
   Leu Gln Leu Ser Ile Ser Ser Cys Leu Gln Gln Leu Ser Leu Leu Met
           195                 200                 205
   Trp Ile Thr Gln Cys Phe Leu Pro Val Phe Leu Ala Gln Ala Pro Ser
       210                 215                 220
   Gly Gln Arg Arg
   225
<210> 84
   <211> 915
   <212> DNA
   <213> Homo Sapien
<400> 84
   atgggccatc atcatcatca tcatcatcat catcacagca gcggccatat cgacgacgac 60
   gacaagcata tgcaggcgga aggccgtggc accggtggta gcaccggcga tgcggatggt 120
   ccgggcggtc cgggtattcc ggacggccct ggtggtaatg cgggtgggcc aggtgaagcg 180
   ggtgcgaccg gtggtcgtgg tccgcggggg gcaggcgcag cacgtgcatc tggtccgggt 240
   ggtggtgcac cgcgcggtcc gcatggtggt gcggcgagcg gcctgaatgg ttgctgccgt 300
   tgcggtgcgc gtggtccgga aagccgtctg ctggaatttt atctggccat gccgtttgcg 360
   accccgatgg aagcggaact ggcccgtcgt agcctggctc aagatgcacc gccgctgccg 420
   gttccgggcg tgctgctgaa agaatttacc gtgagcggca acattctgac cattcgtctg 480
   acggcggcag accatcgtca gctgcaactg agcattagca gctgcctgca acagctgtct 540
   ctgctgatgt ggattaccca gtgctttctg ccggtgtttc tggcccagcc gccgtctggt 600
   caacgtggtg gcgcgcgtcg tccggattct cgcctgctgg aactgcatat taccatgccg 660
   ttcagctctc caatggaggc cgaattagtg cgtcgcattc tgagccgtga tgcggcaccg 720
   ctgccgcgtc caggtgcggt tctgaaagac ttcaccgtat ctggcaacct gctgtttatc 780
   cgtctgaccg cagcggacca ccgccaatta caattatcta tcagctcttg tttacaacaa 840
   ctgtcgctgt taatgtggat cactcaatgt ttcctgccag tattcctggc tcaggccccg 900
   agcggtcagc gtcgt                                                  915
<210> 85
   <211> 305
   <212> PRT
   <213> Homo Sapien
<400> 85
   Met Gly His His His His His His His His His His Ser Ser Gly His
    1               5                  10                  15
   Ile Asp Asp Asp Asp Lys His Met Gln Ala Glu Gly Arg Gly Thr Gly
               20                  25                  30
   Gly Ser Thr Gly Asp Ala Asp Gly Pro Gly Gly Pro Gly Ile Pro Asp
           35                  40                  45
   Gly Pro Gly Gly Asn Ala Gly Gly Pro Gly Glu Ala Gly Ala Thr Gly
       50                  55                  60
   Gly Arg Gly Pro Arg Gly Ala Gly Ala Ala Arg Ala Ser Gly Pro Gly
   65                  70                  75                  80
   Gly Gly Ala Pro Arg Gly Pro His Gly Gly Ala Ala Ser Gly Leu Asn
                   85                  90                  95
   Gly Cys Cys Arg Cys Gly Ala Arg Gly Pro Glu Ser Arg Leu Leu Glu
               100                 105                 110
   Phe Tyr Leu Ala Met Pro Phe Ala Thr Pro Met Glu Ala Glu Leu Ala
           115                 120                 125
   Arg Arg Ser Leu Ala Gln Asp Ala Pro Pro Leu Pro Val Pro Gly Val
       130                 135                 140
   Leu Leu Lys Glu Phe Thr Val Ser Gly Asn Ile Leu Thr Ile Arg Leu
   145                 150                 155                 160
   Thr Ala Ala Asp His Arg Gln Leu Gln Leu Ser Ile Ser Ser Cys Leu
                   165                 170                 175
   Gln Gln Leu Ser Leu Leu Met Trp Ile Thr Gln Cys Phe Leu Pro Val
               180                 185                 190
   Phe Leu Ala Gln Pro Pro Ser Gly Gln Arg Gly Gly Ala Arg Arg Pro
           195                 200                 205
   Asp Ser Arg Leu Leu Glu Leu His Ile Thr Met Pro Phe Ser Ser Pro
       210                 215                 220
   Met Glu Ala Glu Leu Val Arg Arg Ile Leu Ser Arg Asp Ala Ala Pro
   225                 230                 235                 240
   Leu Pro Arg Pro Gly Ala Val Leu Lys Asp Phe Thr Val Ser Gly Asn
                   245                 250                 255
   Leu Leu Phe Ile Arg Leu Thr Ala Ala Asp His Arg Gln Leu Gln Leu
               260                 265                 270
   Ser Ile Ser Ser Cys Leu Gln Gln Leu Ser Leu Leu Met Trp Ile Thr
           275                 280                 285
   Gln Cys Phe Leu Pro Val Phe Leu Ala Gln Ala Pro Ser Gly Gln Arg
       290                 295                 300
   Arg
   305
<210> 86
   <211> 687
   <212> DNA
   <213> Homo Sapien
<400> 86
   atgggccatc atcatcatca tcatcatcat catcacagca gcggccatat cgacgacgac 60
   gacaagcata tgggtgcgcg tcgtccggat agccgtctgc tggagctgca tattaccatg 120
   ccgtttagca gcccaatgga agctgagctg gtgcgtcgta ttctgtctcg tgacgcagca 180
   ccgctgccac gtccgggtgc ggttctgaaa gattttaccg tgagcggcaa cctgctgttt 240
   attcgtctga ccgcggcaga tcatcgtcag ctgcaactga gcattagcag ctgcctgcaa 300
   cagctgtctc tgctgatgtg gattacccag tgctttctgc cggtgtttct ggctcaggcg 360
   ccgtctggtc agcgtcgtgg tggtgcccgt ggcccggaat ctcgtctgct ggaattttat 420
   ctggccatgc cgttcgcgac gccgatggaa gcagagctgg cccgtcgcag cctggctcag 480
   gatgcaccgc cgctgccggt tccgggcgtg ctgctgaaag aatttacggt tagcggtaac 540
   attctgacca tccgtctgac cgcagcggac caccgccaac tgcaactgtc tatcagctct 600
   tgcctgcaac aactgtcgtt attaatgtgg atcactcaat gttttttacc agtattcctg 660
   gcccaaccgc cgagcggcca acgtcgt                                    687
<210> 87
   <211> 229
   <212> PRT
   <213> Homo Sapien
<400> 87
   Met Gly His His His His His His His His His His Ser Ser Gly His
    1               5                  10                  15
   Ile Asp Asp Asp Asp Lys His Met Gly Ala Arg Arg Pro Asp Ser Arg
               20                  25                  30
   Leu Leu Glu Leu His Ile Thr Met Pro Phe Ser Ser Pro Met Glu Ala
           35                  40                  45
   Glu Leu Val Arg Arg Ile Leu Ser Arg Asp Ala Ala Pro Leu Pro Arg
       50                  55                  60
   Pro Gly Ala Val Leu Lys Asp Phe Thr Val Ser Gly Asn Leu Leu Phe
   65                  70                  75                  80
   Ile Arg Leu Thr Ala Ala Asp His Arg Gln Leu Gln Leu Ser Ile Ser
                   85                  90                  95
   Ser Cys Leu Gln Gln Leu Ser Leu Leu Met Trp Ile Thr Gln Cys Phe
               100                 105                 110
   Leu Pro Val Phe Leu Ala Gln Ala Pro Ser Gly Gln Arg Arg Gly Gly
           115                 120                 125
   Ala Arg Gly Pro Glu Ser Arg Leu Leu Glu Phe Tyr Leu Ala Met Pro
       130                 135                 140
   Phe Ala Thr Pro Met Glu Ala Glu Leu Ala Arg Arg Ser Leu Ala Gln
   145                 150                 155                 160
   Asp Ala Pro Pro Leu Pro Val Pro Gly Val Leu Leu Lys Glu Phe Thr
                   165                 170                 175
   Val Ser Gly Asn Ile Leu Thr Ile Arg Leu Thr Ala Ala Asp His Arg
               180                 185                 190
   Gln Leu Gln Leu Ser Ile Ser Ser Cys Leu Gln Gln Leu Ser Leu Leu
           195                 200                 205
   Met Trp Ile Thr Gln Cys Phe Leu Pro Val Phe Leu Ala Gln Pro Pro
       210                 215                 220
   Ser Gly Gln Arg Arg
   225
<210> 88
   <211> 1197
   <212> DNA
   <213> Homo Sapien
<400> 88
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa acccaggcgg aaggccgtgg tactggcggt 360
   agcaccggcg atgcagatgg tccgggcggt ccgggtattc cggatggtcc gggtggtaat 420
   gcaggtggtc caggtgaagc aggtgcgact ggcggtcgtg gtccacgcgg tgcaggtgca 480
   gcgcgtgcat ctggtccagg tggcggtgcg ccgcgtggcc cgcatggtgg tgcagctagt 540
   gcgcaagatg gtcgttgccc gtgtggtgcg cgtcgtccgg atagccgtct gctggagctg 600
   catattacca tgccgtttag cagcccaatg gaagctgagc tggtgcgtcg tattctgtct 660
   cgtgacgcag caccgctgcc acgtccgggt gcggttctga aagattttac cgtgagcggc 720
   aacctgctgt ttattcgtct gaccgcggca gatcatcgtc agctgcaact gagcattagc 780
   agctgcctgc aacagctgtc tctgctgatg tggattaccc agtgctttct gccggtgttt 840
   ctggctcagg cgccgtctgg tcagcgtcgt ggtggtgccc gtggcccgga atctcgtctg 900
   ctggaatttt atctggccat gccgttcgcg acgccgatgg aagcagagct ggcccgtcgc 960
   agcctggctc aggatgcacc gccgctgccg gttccgggcg tgctgctgaa agaatttacg 1020
   gttagcggta acattctgac catccgtctg accgcagcgg accaccgcca actgcaactg 1080
   tctatcagct cttgcctgca acaactgtcg ttattaatgt ggatcactca atgtttttta 1140
   ccagtattcc tggcccaacc gccgagcggc caacgtcgtc accaccacca ccaccac    1197
<210> 89
   <211> 399
   <212> PRT
   <213> Homo Sapien
<400> 89
   Met Asp Pro Ser Ser His Ser Ser Asn Met Ala Asn Thr Gln Met Lys
   1               5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gln
               100                 105                 110
   Ala Glu Gly Arg Gly Thr Gly Gly Ser Thr Gly Asp Ala Asp Gly Pro
           115                 120                 125
   Gly Gly Pro Gly Ile Pro Asp Gly Pro Gly Gly Asn Ala Gly Gly Pro
       130                 135                 140
   Gly Glu Ala Gly Ala Thr Gly Gly Arg Gly Pro Arg Gly Ala Gly Ala
   145                 150                 155                 160
   Ala Arg Ala Ser Gly Pro Gly Gly Gly Ala Pro Arg Gly Pro His Gly
                   165                 170                 175
   Gly Ala Ala Ser Ala Gln Asp Gly Arg Cys Pro Cys Gly Ala Arg Arg
               180                 185                 190
   Pro Asp Ser Arg Leu Leu Glu Leu His Ile Thr Met Pro Phe Ser Ser
           195                 200                 205
   Pro Met Glu Ala Glu Leu Val Arg Arg Ile Leu Ser Arg Asp Ala Ala
       210                 215                 220
   Pro Leu Pro Arg Pro Gly Ala Val Leu Lys Asp Phe Thr Val Ser Gly
   225                 230                 235                 240
   Asn Leu Leu Phe Ile Arg Leu Thr Ala Ala Asp His Arg Gln Leu Gln
                   245                 250                 255
   Leu Ser Ile Ser Ser Cys Leu Gln Gln Leu Ser Leu Leu Met Trp Ile
               260                 265                 270
   Thr Gln Cys Phe Leu Pro Val Phe Leu Ala Gln Ala Pro Ser Gly Gln
           275                 280                 285
   Arg Arg Gly Gly Ala Arg Gly Pro Glu Ser Arg Leu Leu Glu Phe Tyr
       290                 295                 300
   Leu Ala Met Pro Phe Ala Thr Pro Met Glu Ala Glu Leu Ala Arg Arg
   305                 310                 315                 320
   Ser Leu Ala Gln Asp Ala Pro Pro Leu Pro Val Pro Gly Val Leu Leu
                   325                 330                 335
   Lys Glu Phe Thr Val Ser Gly Asn Ile Leu Thr Ile Arg Leu Thr Ala
               340                 345                 350
   Ala Asp His Arg Gln Leu Gln Leu Ser Ile Ser Ser Cys Leu Gln Gln
           355                 360                 365
   Leu Ser Leu Leu Met Trp Ile Thr Gln Cys Phe Leu Pro Val Phe Leu
       370                 375                 380
   Ala Gln Pro Pro Ser Gly Gln Arg Arg His His His His His His
   385                 390                 395
<210> 90
   <211> 1194
   <212> DNA
   <213> Homo Sapien
<400> 90
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa acccaggcgg aaggccgtgg caccggtggt 360
   agcaccggcg atgcggatgg tccgggcggt ccgggtattc cggacgggcc tggtggtaat 420
   gcgggtgggc caggtgaagc gggtgcgacc ggtggtcgtg gtccgcgggg ggcaggcgca 480
   gcacgtgcat ctggtccggg tggtggtgca ccgcgcggtc cgcatggtgg tgcggcgagc 540
   ggcctgaatg gttgctgccg ttgcggtgcg cgtggtccgg aaagccgtct gctggaattt 600
   tatctggcca tgccgtttgc gaccccgatg gaagcggaac tggcccgtcg tagcctggct 660
   caagatgcac cgccgctgcc ggttccgggc gtgctgctga aagaatttac cgtgagcggc 720
   aacattctga ccattcgtct gacggcggca gaccatcgtc agctgcaact gagcattagc 780
   agctgcctgc aacagctgtc tctgctgatg tggattaccc agtgctttct gccggtgttt 840
   ctggcccagc cgccgtctgg tcaacgtggt ggcgcgcgtc gtccggattc tcgcctgctg 900
   gaactgcata ttaccatgcc gttcagctct ccaatggagg ccgaattagt gcgtcgcatt 960
   ctgagccgtg atgcggcacc gctgccgcgt ccaggtgcgg ttctgaaaga cttcaccgta 1020
   tctggcaacc tgctgtttat ccgtctgacc gcagcggacc accgccaatt acaattatct 1080
   atcagctctt gtttacaaca actgtcgctg ttaatgtgga tcactcaatg tttcctgcca 1140
   gtattcctgg ctcaggcccc gagcggtcag cgtcgtcacc accaccacca ccac       1194
<210> 91
   <211> 398
   <212> PRT
   <213> Homo Sapien
<400> 91
   Met Asp Pro Ser Ser His Ser Ser Asn Met Ala Asn Thr Gln Met Lys
    1              5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Gln
               100                 105                 110
   Ala Glu Gly Arg Gly Thr Gly Gly Ser Thr Gly Asp Ala Asp Gly Pro
           115                 120                 125
   Gly Gly Pro Gly Ile Pro Asp Gly Pro Gly Gly Asn Ala Gly Gly Pro
       130                 135                 140
   Gly Glu Ala Gly Ala Thr Gly Gly Arg Gly Pro Arg Gly Ala Gly Ala
   145                 150                 155                 160
   Ala Arg Ala Ser Gly Pro Gly Gly Gly Ala Pro Arg Gly Pro His Gly
                   165                 170                 175
   Gly Ala Ala Ser Gly Leu Asn Gly Cys Cys Arg Cys Gly Ala Arg Gly
               180                 185                 190
   Pro Glu Ser Arg Leu Leu Glu Phe Tyr Leu Ala Met Pro Phe Ala Thr
           195                 200                 205
   Pro Met Glu Ala Glu Leu Ala Arg Arg Ser Leu Ala Gln Asp Ala Pro
       210                 215                 220
   Pro Leu Pro Val Pro Gly Val Leu Leu Lys Glu Phe Thr Val Ser Gly
   225                 230                 235                 240
   Asn Ile Leu Thr Ile Arg Leu Thr Ala Ala Asp His Arg Gln Leu Gln
                   245                 250                 255
   Leu Ser Ile Ser Ser Cys Leu Gln Gln Leu Ser Leu Leu Met Trp Ile
               260                 265                 270
   Thr Gln Cys Phe Leu Pro Val Phe Leu Ala Gln Pro Pro Ser Gly Gln
           275                 280                 285
   Arg Gly Gly Ala Arg Arg Pro Asp Ser Arg Leu Leu Glu Leu His Ile
       290                 295                 300
   Thr Met Pro Phe Ser Ser Pro Met Glu Ala Glu Leu Val Arg Arg Ile
   305                 310                 315                 320
   Leu Ser Arg Asp Ala Ala Pro Leu Pro Arg Pro Gly Ala Val Leu Lys
                   325                 330                 335
   Asp Phe Thr Val Ser Gly Asn Leu Leu Phe Ile Arg Leu Thr Ala Ala
               340                 345                 350
   Asp His Arg Gln Leu Gln Leu Ser Ile Ser Ser Cys Leu Gln Gln Leu
           355                 360                 365
   Ser Leu Leu Met Trp Ile Thr Gln Cys Phe Leu Pro Val Phe Leu Ala
       370                 375                 380
   Gln Ala Pro Ser Gly Gln Arg Arg His His His His His His
   385                 390                 395
<210> 92
   <211> 867
   <212> DNA
   <213> Homo Sapien
<400> 92
   atgcatcatc atcatcatca ccaggcggaa ggccgtggta ctggcggtag caccggcgat 60
   gcagatggtc cgggcggtcc gggtattccg gatggtccgg gtggtaatgc aggtggtcca 120
   ggtgaagcag gtgcgactgg cggtcgtggt ccacgcggtg caggtgcagc gcgtgcatct 180
   ggtccaggtg gcggtgcgcc gcgtggcccg catggtggtg cagctagtgc gcaagatggt 240
   cgttgcccgt gtggtgcgcg tcgtccggat agccgtctgc tggagctgca tattaccatg 300
   ccgtttagca gcccaatgga agctgagctg gtgcgtcgta ttctgtctcg tgacgcagca 360
   ccgctgccac gtccgggtgc ggttctgaaa gattttaccg tgagcggcaa cctgctgttt 420
   attcgtctga ccgcggcaga tcatcgtcag ctgcaactga gcattagcag ctgcctgcaa 480
   cagctgtctc tgctgatgtg gattacccag tgctttctgc cggtgtttct ggctcaggcg 540
   ccgtctggtc agcgtcgtgg tggtgcccgt ggcccggaat ctcgtctgct ggaattttat 600
   ctggccatgc cgttcgcgac gccgatggaa gcagagctgg cccgtcgcag cctggctcag 660
   gatgcaccgc cgctgccggt tccgggcgtg ctgctgaaag aatttacggt tagcggtaac 720
   attctgacca tccgtctgac cgcagcggac caccgccaac tgcaactgtc tatcagctct 780
   tgcctgcaac aactgtcgtt attaatgtgg atcactcaat gttttttacc agtattcctg 840
   gcccaaccgc cgagcggcca acgtcgt                                    867
<210> 93
   <211> 289
   <212> PRT
   <213> Homo Sapien
<400> 93
   Met His His His His His His Gln Ala Glu Gly Arg Gly Thr Gly Gly
    1               5                  10                  15
   Ser Thr Gly Asp Ala Asp Gly Pro Gly Gly Pro Gly Ile Pro Asp Gly
               20                  25                  30
   Pro Gly Gly Asn Ala Gly Gly Pro Gly Glu Ala Gly Ala Thr Gly Gly
           35                  40                  45
   Arg Gly Pro Arg Gly Ala Gly Ala Ala Arg Ala Ser Gly Pro Gly Gly
       50                  55                  60
   Gly Ala Pro Arg Gly Pro His Gly Gly Ala Ala Ser Ala Gln Asp Gly
   65                  70                  75                  80
   Arg Cys Pro Cys Gly Ala Arg Arg Pro Asp Ser Arg Leu Leu Glu Leu
                   85                  90                  95
   His Ile Thr Met Pro Phe Ser Ser Pro Met Glu Ala Glu Leu Val Arg
               100                 105                 110
   Arg Ile Leu Ser Arg Asp Ala Ala Pro Leu Pro Arg Pro Gly Ala Val
           115                 120                 125
   Leu Lys Asp Phe Thr Val Ser Gly Asn Leu Leu Phe Ile Arg Leu Thr
       130                 135                 140
   Ala Ala Asp His Arg Gln Leu Gln Leu Ser Ile Ser Ser Cys Leu Gln
   145                 150                 155                 160
   Gln Leu Ser Leu Leu Met Trp Ile Thr Gln Cys Phe Leu Pro Val Phe
                   165                 170                 175
   Leu Ala Gln Ala Pro Ser Gly Gln Arg Arg Gly Gly Ala Arg Gly Pro
               180                 185                 190
   Glu Ser Arg Leu Leu Glu Phe Tyr Leu Ala Met Pro Phe Ala Thr Pro
           195                 200                 205
   Met Glu Ala Glu Leu Ala Arg Arg Ser Leu Ala Gln Asp Ala Pro Pro
       210                 215                 220
   Leu Pro Val Pro Gly Val Leu Leu Lys Glu Phe Thr Val Ser Gly Asn
   225                 230                 235                 240
   Ile Leu Thr Ile Arg Leu Thr Ala Ala Asp His Arg Gln Leu Gln Leu
                   245                 250                 255
   Ser Ile Ser Ser Cys Leu Gln Gln Leu Ser Leu Leu Met Trp Ile Thr
               260                 265                 270
   Gln Cys Phe Leu Pro Val Phe Leu Ala Gln Pro Pro Ser Gly Gln Arg
           275                 280                 285
   Arg
<210> 94
   <211> 633
   <212> DNA
   <213> Homo Sapien
<400> 94
   atgcatcatc atcatcatca cggtgcgcgt ggtccggaaa gccgtctgct ggaattttat 60
   ctggccatgc cgtttgcgac cccgatggaa gcggaactgg cccgtcgtag cctggctcaa 120
   gatgcaccgc cgctgccggt tccgggcgtg ctgctgaaag aatttaccgt gagcggcaac 180
   attctgacca ttcgtctgac ggcggcagac catcgtcagc tgcaactgag cattagcagc 240
   tgcctgcaac agctgtctct gctgatgtgg attacccagt gctttctgcc ggtgtttctg 300
   gcccagccgc cgtctggtca acgtggtggc gcgcgtcgtc cggattctcg cctgctggaa 360
   ctgcatatta ccatgccgtt cagctctcca atggaggccg aattagtgcg tcgcattctg 420
   agccgtgatg cggcaccgct gccgcgtcca ggtgcggttc tgaaagactt caccgtatct 480
   ggcaacctgc tgtttatccg tctgaccgca gcggaccacc gccaattaca attatctatc 540
   agctcttgtt tacaacaact gtcgctgtta atgtggatca ctcaatgttt cctgccagta 600
   ttcctggctc aggccccgag cggtcagcgt cgt                               633
<210> 95
   <211> 211
   <212> PRT
   <213> Homo Sapien
<400> 95
   Met His His His His His His Gly Ala Arg Gly Pro Glu Ser Arg Leu
    1               5                  10                  15
   Leu Glu Phe Tyr Leu Ala Met Pro Phe Ala Thr Pro Met Glu Ala Glu
               20                  25                  30
   Leu Ala Arg Arg Ser Leu Ala Gln Asp Ala Pro Pro Leu Pro Val Pro
           35                  40                  45
   Gly Val Leu Leu Lys Glu Phe Thr Val Ser Gly Asn Ile Leu Thr Ile
       50                  55                  60
   Arg Leu Thr Ala Ala Asp His Arg Gln Leu Gln Leu Ser Ile Ser Ser
   65                  70                  75                  80
   Cys Leu Gln Gln Leu Ser Leu Leu Met Trp Ile Thr Gln Cys Phe Leu
                   85                  90                  95
   Pro Val Phe Leu Ala Gln Pro Pro Ser Gly Gln Arg Gly Gly Ala Arg
               100                 105                 110
   Arg Pro Asp Ser Arg Leu Leu Glu Leu His Ile Thr Met Pro Phe Ser
           115                 120                 125
   Ser Pro Met Glu Ala Glu Leu Val Arg Arg Ile Leu Ser Arg Asp Ala
       130                 135                 140
   Ala Pro Leu Pro Arg Pro Gly Ala Val Leu Lys Asp Phe Thr Val Ser
   145                 150                 155                 160
   Gly Asn Leu Leu Phe Ile Arg Leu Thr Ala Ala Asp His Arg Gln Leu
                   165                 170                 175
   Gln Leu Ser Ile Ser Ser Cys Leu Gln Gln Leu Ser Leu Leu Met Trp
               180                 185                 190
   Ile Thr Gln Cys Phe Leu Pro Val Phe Leu Ala Gln Ala Pro Ser Gly
           195                 200                 205
   Gln Arg Arg
       210
<210> 96
   <211> 864
   <212> DNA
   <213> Homo Sapien
<400> 96
   atgcatcatc atcatcatca ccaggcggaa ggccgtggca ccggtggtag caccggcgat 60
   gcggatggtc cgggcggtcc gggtattccg gacgggcctg gtggtaatgc gggtgggcca 120
   ggtgaagcgg gtgcgaccgg tggtcgtggt ccgcgggggg caggcgcagc acgtgcatct 180
   ggtccgggtg gtggtgcacc gcgcggtccg catggtggtg cggcgagcgg cctgaatggt 240
   tgctgccgtt gcggtgcgcg tggtccggaa agccgtctgc tggaatttta tctggccatg 300
   ccgtttgcga ccccgatgga agcggaactg gcccgtcgta gcctggctca agatgcaccg 360
   ccgctgccgg ttccgggcgt gctgctgaaa gaatttaccg tgagcggcaa cattctgacc 420
   attcgtctga cggcggcaga ccatcgtcag ctgcaactga gcattagcag ctgcctgcaa 480
   cagctgtctc tgctgatgtg gattacccag tgctttctgc cggtgtttct ggcccagccg 540
   ccgtctggtc aacgtggtgg cgcgcgtcgt ccggattctc gcctgctgga actgcatatt 600
   accatgccgt tcagctctcc aatggaggcc gaattagtgc gtcgcattct gagccgtgat 660
   gcggcaccgc tgccgcgtcc aggtgcggtt ctgaaagact tcaccgtatc tggcaacctg 720
   ctgtttatcc gtctgaccgc agcggaccac cgccaattac aattatctat cagctcttgt 780
   ttacaacaac tgtcgctgtt aatgtggatc actcaatgtt tcctgccagt attcctggct 840
   caggccccga gcggtcagcg tcgt                                        864
<210> 97
   <211> 288
   <212> PRT
   <213> Homo Sapien
<400> 97
   Met His His His His His His Gln Ala Glu Gly Arg Gly Thr Gly Gly
    1               5                  10                  15
   Ser Thr Gly Asp Ala Asp Gly Pro Gly Gly Pro Gly Ile Pro Asp Gly
               20                  25                  30
   Pro Gly Gly Asn Ala Gly Gly Pro Gly Glu Ala Gly Ala Thr Gly Gly
           35                  40                  45
   Arg Gly Pro Arg Gly Ala Gly Ala Ala Arg Ala Ser Gly Pro Gly Gly
       50                  55                  60
   Gly Ala Pro Arg Gly Pro His Gly Gly Ala Ala Ser Gly Leu Asn Gly
   65                  70                  75                  80
   Cys Cys Arg Cys Gly Ala Arg Gly Pro Glu Ser Arg Leu Leu Glu Phe
                   85                  90                  95
   Tyr Leu Ala Met Pro Phe Ala Thr Pro Met Glu Ala Glu Leu Ala Arg
               100                 105                 110
   Arg Ser Leu Ala Gln Asp Ala Pro Pro Leu Pro Val Pro Gly Val Leu
           115                 120                 125
   Leu Lys Glu Phe Thr Val Ser Gly Asn Ile Leu Thr Ile Arg Leu Thr
       130                 135                 140
   Ala Ala Asp His Arg Gln Leu Gln Leu Ser Ile Ser Ser Cys Leu Gln
   145                 150                 155                 160
   Gln Leu Ser Leu Leu Met Trp Ile Thr Gln Cys Phe Leu Pro Val Phe
                   165                 170                 175
   Leu Ala Gln Pro Pro Ser Gly Gln Arg Gly Gly Ala Arg Arg Pro Asp
               180                 185                 190
   Ser Arg Leu Leu Glu Leu His Ile Thr Met Pro Phe Ser Ser Pro Met
           195                 200                 205
   Glu Ala Glu Leu Val Arg Arg Ile Leu Ser Arg Asp Ala Ala Pro Leu
       210                 215                 220
   Pro Arg Pro Gly Ala Val Leu Lys Asp Phe Thr Val Ser Gly Asn Leu
   225                 230                 235                 240
   Leu Phe Ile Arg Leu Thr Ala Ala Asp His Arg Gln Leu Gln Leu Ser
                   245                 250                 255
   Ile Ser Ser Cys Leu Gln Gln Leu Ser Leu Leu Met Trp Ile Thr Gln
               260                 265                 270
   Cys Phe Leu Pro Val Phe Leu Ala Gln Ala Pro Ser Gly Gln Arg Arg
           275                 280                 285
<210> 98
   <211> 450
   <212> PRT
   <213> Artificial Sequence
   <223> Protein D-MAGE-A3-His
<400> 98
   Met Asp Pro Lys Thr Leu Ala Leu Ser Leu Leu Ala Ala Gly Val Leu
    1               5                  10                  15
   Ala Gly Cys Ser Ser His Ser Ser Asn Met Ala Asn Thr Gln Met Lys
               20                  25                  30
   Ser Asp Lys Ile Ile Ile Ala His Arg Gly Ala Ser Gly Tyr Leu Pro
           35                  40                  45
   Glu His Thr Leu Glu Ser Lys Ala Leu Ala Phe Ala Gln Gln Ala Asp
       50                  55                  60
   Tyr Leu Glu Gln Asp Leu Ala Met Thr Lys Asp Gly Arg Leu Val Val
   65                  70                  75                  80
   Ile His Asp His Phe Leu Asp Gly Leu Thr Asp Val Ala Lys Lys Phe
                   85                  90                  95
   Pro His Arg His Arg Lys Asp Gly Arg Tyr Tyr Val Ile Asp Phe Thr
               100                 105                 110
   Leu Lys Glu Ile Gln Ser Leu Glu Met Thr Glu Asn Phe Glu Thr Met
           115                 120                 125
   Asp Leu Glu Gln Arg Ser Gln His Cys Lys Pro Glu Glu Gly Leu Glu
       130                 135                 140
   Ala Arg Gly Glu Ala Leu Gly Leu Val Gly Ala Gln Ala Pro Ala Thr
   145                 150                 155                 160
   Glu Glu Gln Glu Ala Ala Ser Ser Ser Ser Thr Leu Val Glu Val Thr
                   165                 170                 175
   Leu Gly Glu Val Pro Ala Ala Glu Ser Pro Asp Pro Pro Gln Ser Pro
               180                 185                 190
   Gln Gly Ala Ser Ser Leu Pro Thr Thr Met Asn Tyr Pro Leu Trp Ser
           195                 200                 205
   Gln Ser Tyr Glu Asp Ser Ser Asn Gln Glu Glu Glu Gly Pro Ser Thr
       210                 215                 220
   Phe Pro Asp Leu Glu Ser Glu Phe Gln Ala Ala Leu Ser Arg Lys Val
   225                 230                 235                 240
   Ala Glu Leu Val His Phe Leu Leu Leu Lys Tyr Arg Ala Arg Glu Pro
                   245                 250                 255
   Val Thr Lys Ala Glu Met Leu Gly Ser Val Val Gly Asn Trp Gln Tyr
               260                 265                 270
   Phe Phe Pro Val Ile Phe Ser Lys Ala Ser Ser Ser Leu Gln Leu Val
           275                 280                 285
   Phe Gly Ile Glu Leu Met Glu Val Asp Pro Ile Gly His Leu Tyr Ile
       290                 295                 300
   Phe Ala Thr Cys Leu Gly Leu Ser Tyr Asp Gly Leu Leu Gly Asp Asn
   305                 310                 315                 320
   Gln Ile Met Pro Lys Ala Gly Leu Leu Ile Ile Val Leu Ala Ile Ile
                   325                 330                 335
   Ala Arg Glu Gly Asp Cys Ala Pro Glu Glu Lys Ile Trp Glu Glu Leu
               340                 345                 350
   Ser Val Leu Glu Val Phe Glu Gly Arg Glu Asp Ser Ile Leu Gly Asp
           355                 360                 365
   Pro Lys Lys Leu Leu Thr Gln His Phe Val Gln Glu Asn Tyr Leu Glu
       370                 375                 380
   Tyr Arg Gln Val Pro Gly Ser Asp Pro Ala Cys Tyr Glu Phe Leu Trp
   385                 390                 395                 400
   Gly Pro Arg Ala Leu Val Glu Thr Ser Tyr Val Lys Val Leu His His
                   405                 410                 415
   Met Val Lys Ile Ser Gly Gly Pro His Ile Ser Tyr Pro Pro Leu His
               420                 425                 430
   Glu Trp Val Leu Arg Glu Gly Glu Glu Gly Gly His His His His His
           435                 440                 445
   His His
       450